(19) Europäisches Patentamt
European Patent Office
Office européen des brevets

(11) EP 3 456 820 B1

(12) EUROPÄISCHE PATENTSCHRIFT

(45) Veröffentlichungstag und Bekanntmachung des Hinweises auf die Patenterteilung:
**19.06.2024 Patentblatt 2024/25**

(21) Anmeldenummer: **18203708.5**

(22) Anmeldetag: **16.12.2011**

(51) Internationale Patentklassifikation (IPC):
***C12N 9/04*** *(2006.01)* ***C12P 33/06*** *(2006.01)*
***C12P 33/02*** *(2006.01)*

(52) Gemeinsame Patentklassifikation (CPC):
**C12N 9/0006; C12N 9/0008; C12P 33/00; C12Y 101/01201**

# (54) NEUARTIGE 7BETA-HYDROXYSTEROID DEHYDROGENASE-MUTANTEN UND VERFAHREN ZUR HERSTELLUNG VON URSODESOXYCHOLSÄURE

New 7beta-hydroxysteriod dehydrogenase mutants and method for producing ursodeoxycholic acid

7 ß-hydroxystéroïdes déshydrogénases mutants d'un nouveau type et procédé de fabrication d'acide ursodésoxycholique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.12.2010 EP 10015726**

(43) Veröffentlichungstag der Anmeldung:
**20.03.2019 Patentblatt 2019/12**

(62) Dokumentnummer(n) der früheren Anmeldung(en) nach Art. 76 EPÜ:
**11799094.5 / 2 652 129**

(73) Patentinhaber: **PharmaZell GmbH**
**83064 Raubling (DE)**

(72) Erfinder:
- **WEUSTER-BOTZ, Dirk**
  **80634 München (DE)**
- **BRAUN, Michael**
  **97980 Bad Mergentheim-Edelfingen (DE)**
- **AIGNER, Arno**
  **83104 Tuntenhausen (DE)**
- **SUN, Boqiao**
  **80336 München (DE)**
- **KANTZOW, Christina**
  **81545 München (DE)**
- **BRESCH, Sven**
  **4053 Basel (CH)**
- **BAKONYI, Daniel**
  **50969 Köln (DE)**
- **HUMMEL, Werner**
  **52445 Titz (DE)**

(74) Vertreter: **Reitstötter Kinzebach**
**Patentanwälte**
**Sternwartstraße 4**
**81679 München (DE)**

(56) Entgegenhaltungen:
- **CARREA GIACOMO ET AL: "Enzymatic synthesis of 12-ketoursodeoxycholic acid from dehydrocholic acid in a membrane reactor", BIOTECHNOLOGY LETTERS, KEW, SURREY, GB, Bd. 14, Nr. 12, 1. Dezember 1992 (1992-12-01), Seiten 1131-1135, XP002579160, ISSN: 0141-5492**
- **PEDRINI P ET AL: "Xanthomonas maltophilia CBS 897.97 as a source of new 7beta- and 7alpha-hydroxysteroid dehydrogenases and cholylglycine hydrolase: Improved biotransformations of bile acids", STEROIDS, ELSEVIER SCIENCE PUBLISHERS, NEW YORK, NY, US, Bd. 71, Nr. 3, 1. März 2006 (2006-03-01), Seiten 189-198, XP025127769, ISSN: 0039-128X, DOI: DOI:10.1016/J.STEROIDS.2005.10.002 [gefunden am 2006-03-01]**
- **MACDONALD I A ET AL: "Epimerization versus dehydroxylation of the 7alpha-hydroxyl- group of primary bile acids: Competitive studies with Clostridium absonum and 7alpha-dehydroxylating bacteria (Eubacterium SP.)", JOURNAL OF STEROID BIOCHEMISTRY, PERGAMON PRESS PLC, GB, Bd. 17, Nr. 3, 1. September 1982 (1982-09-01), Seiten 287-293, XP025877762, ISSN: 0022-4731, DOI: 10.1016/0022-4731(82)90203-5 [gefunden am 1982-09-01]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

• **PEDRO VICO ET AL: "Dehydroepiandrosterone (DHEA) metabolism in Saccharomyces cerevisiae expressing mammalian steroid hydroxylase CYP7B: Ayr1p and Fox2p display 17?-hydroxysteroid dehydrogenase activity", YEAST, vol. 19, no. 10, 1 July 2002 (2002-07-01), pages 873-886, XP055663845, ISSN: 0749-503X, DOI: 10.1002/yea.882**

Bemerkungen:

Das gesamte Dokument mit Referenztabelle(n) und Sequenzliste(n) kann von der Webseite des EPA heruntergeladen werden

**Beschreibung**

Die Erfindung betrifft neuartige Verfahren zur Herstellung von Ursodesoxycholsäure (UDCS) sowie dazu verwendbare rekombinante, mehrfach modifizierte Mikroorganismen.

## Hintergrund der Erfindung:

Zur medikamentösen Behandlung von Gallensteinleiden werden seit vielen Jahren u. a. die Wirkstoffe Ursodesoxycholsäure (UDCS oder UDCA) und das zugehörige Diastereomer Chenodesoxycholsäure (CDCS oder CDCA) eingesetzt. Beide Verbindungen unterscheiden sich lediglich durch die Konfiguration der Hydroxygruppe am C-Atom 7 (UDCS: β - Konfiguration, CDCS: α-Konfiguration). Zur Herstellung von UDCS sind im Stand der Technik verschieden Verfahren beschreiben, welche rein chemisch durchgeführt werden oder aus einer Kombination chemischer und enzymatischer Verfahrensschritte bestehen. Ausgangspunkt ist jeweils Cholsäure (CS oder CA) oder die aus Cholsäure hergestellte CDCS.

So kann die klassisch chemische Methode zur UDCS Herstellung wie folgt schematisch dargestellt werden:

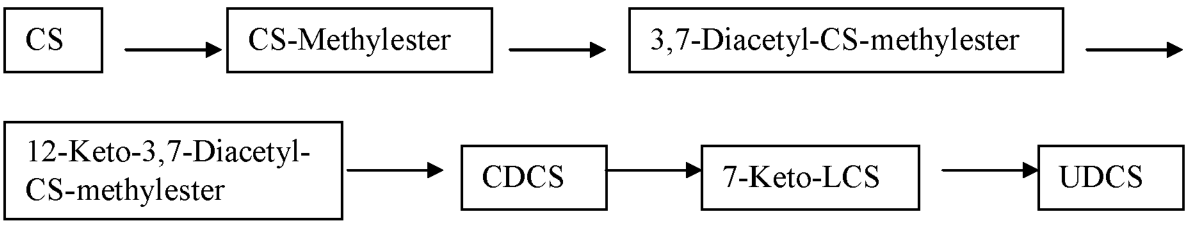


Ein gravierender Nachteil ist unter anderem folgender: da die chemische Oxidation nicht selektiv ist, müssen die Carboxy-Gruppe und die 3α und 7α-Hydroxy-Gruppe durch Veresterung geschützt werden.

Ein alternatives chemisch/enzymatische Verfahren basierend auf der Verwendung des Enzyms 12α-Hydroxysteroid Dehydrogenase (12α-HSDH) kann wie folgt dargestellt werden und ist z.B. beschrieben in der PCT/EP2009/002190 der vorliegenden Anmelderin.

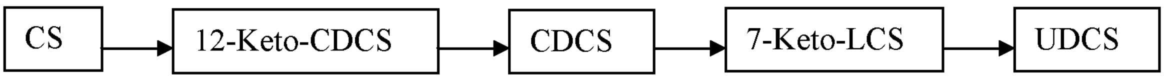


Die 12α-HSDH oxidiert dabei CS selektiv zu 12-Keto-CDCS. Die zwei nach der klassisch chemischen Methode erforderlichen Schützschritte entfallen dabei.

Weiterhin wird von Monti, D., et al., (One-Pot Multienzymatic Synthesis of 12-Ketoursodeoxycholic Acid: Subtle Cofactor Specificities Rule the Reaction Equilibria of Five Biocatalysts Working in a Row. Advanced Synthesis & Catalysis, 2009) ein alternatives enzymatisch-chemisches Verfahren beschrieben, das wie folgt schematisch darstellbar ist:

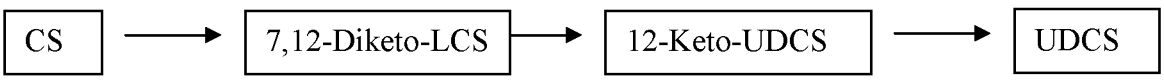


Die CS wird zuerst von 7α-HSDH aus *Bacteroides fragilis* ATCC 25285 (Zhu, D., et al., Enzymatic enantioselective reduction of -ketoesters by a thermostable 7 -hydroxysteroid dehydrogenase from Bacteroides fragilis. Tetrahedron, 2006. 62(18): p. 4535-4539) und 12α-HSDH zu 7,12-Diketo-LCS oxidiert. Diese beiden Enzyme sind jeweils NADH abhängig. Nach der Reduktion durch 7β-HSDH (NADPH abhängig) aus *Clostridium absonum* ATCC 27555 (DSM 599) (MacDonald, I.A. and P.D. Roach, Bile induction of 7 alpha- and 7 beta-hydroxysteroid dehydrogenases in Clostridium absonum. Biochim Biophys Acta, 1981. 665(2): p. 262-9) entsteht 12-Keto-UDCS. Durch Wolff-Kishner-Reduktion wird das Endprodukt erhalten. Nachteilig bei diesem Verfahren ist, dass wegen der Gleichgewichtslage der katalysieren Reaktion eine komplette Umsetzung nicht möglich ist, und dass für die erste Stufe der Umsetzung zwei unterschiedliche Enzyme eingesetzt werden müssen, was das Verfahren verteuert. Zur Kofaktor-Regenerierung werden Lactat Dehydrogenase (LDH; zur Regenerierung von $NAD^+$) und Glucose Dehydrogenase (GlcDH oder GDH, zur Regenerierung von NADPH) eingesetzt. Nachteilig bei der dort verwendeten Kofaktor-Regenerierung ist, dass das entstehende Ko-Produkt nur sehr schwer aus dem Reaktionsgemisch zu entfernen ist, so dass das Reaktionsgleichgewicht nicht positiv beeinflusst werden kann, was eine unvollständige Umsetzung des Edukts bedingt.

Eine 7β-HSDH aus Stamm *Collinsella aerofaciens* ATCC 25986 (DSM 3979; ehemalige *Eubacterium aerofaciens*) wurde im Jahr 1982 von Hirano und Masuda beschrieben (Hirano, S. and N. Masuda, Characterization of NADP-dependent 7 beta-hydroxysteroid dehydrogenases from Peptostreptococcus productus and Eubacterium aerofaciens. Appl Environ Microbiol, 1982. 43(5): p. 1057-63). Sequenzinformationen wurden zu diesem Enzym nicht offenbart. Das durch Gelfiltration bestimmte Molekulargewicht betrug 45,000 Da (vgl. Hirano, Seite 1059, linke Spalte). Weiterhin konnte für das dortige Enzym die Reduktion der 7-Oxo-Gruppe zur 7ß-Hydroxygruppe nicht beobachtet werden (vgl. Hirano, Seite 1061, Diskussion 1. Absatz), der Fachmann erkennt somit, dass das von Hirano et al beschriebene Enzym nicht zur Katalyse der Reduktion von Dehydrocholsäure (DHCS oder DHCA) in 7-Position zu 3,12-Diketo-7ß-CS geeignet ist.

In der älteren internationalen Patentanmeldung PCT/EP2010/068576 der Anmelderin wird eine neuartige 7β-HSDH aus *Collinsella aerofaciens* ATCC 25986 beschrieben, welche unter anderem ein Molekulargewicht (bei SDS-Gelelektrophorese) von etwa 28-32 kDa, ein Molekulargewicht (bei Gelfiltration, unter nicht denaturierenden Bedingungen, wie insbesondere ohne SDS): von etwa 53 bis 60 kDa, und die Befähigung zur stereoselektiven Reduktion der 7-Carbonyl-Gruppe von 7-Keto-LCS in eine 7β-Hydroxy-Gruppe aufweist.

Außerdem wird in der PCT/EP2010/068576 ein Verfahren zur UDCS Herstellung bereitgestellt, welches schematisch wie folgt darstellbar ist:

CS → DHCS → 12-Keto-UDCS → UDCS

Dabei erfolgt die Oxidation von CS auf klassisch chemischem Weg in einfacher Weise. Die DHCS wird von Enzympaar 7β-HSDH und 3α-HSDH einzeln nacheinander oder in einem Topf zu 12-Keto-UDCS reduziert. Kombiniert mit der Wolff-Kishner-Reduktion kann UDCS somit in nur drei Schritte aus CS synthetisiert werden. Während die 7β-HSDH vom Kofaktor NADPH abhängig ist, benötigt die 3α-HSDH den Kofaktor NADH. Die Verfügbarkeit von Enzympaaren mit Abhängigkeit vom gleichen Kofaktor oder erweiterter Anhängigkeit, (z.B. von den Kofaktoren NADH und NADPH) wäre von Vorteil, weil damit die Kofaktor-Regenerierung vereinfacht werden könnte.

Eine Aufgabe der vorliegenden Erfindung besteht in der Bereitstellung neuartiger mikrobieller Synthesewege, welche sich insbesondere auch durch eine vereinfachte Kofaktor-Regenerierung bei der reduktiven Herstellung von UDCS über DHCS auszeichnen.

## Kurzfassung der Erfindung:

Obige Aufgaben konnten überraschenderweise gelöst werden durch Bereitstellung der rekombinanten Mikroorganismen und Verfahren gemäß den beiliegenden Ansprüchen.

## Figurenbeschreibung:

Figur 1a zeigt die Aminosäuresequenz der 7ß-HSDH aus *Collinsella aerofaciens* und Figur 1b die kodierende Nukleinsäuresequenz zur Aminosäuresequenz von Figur 1a; Figur 1c zeigt die Aminosäuresequenz der 3α-HSDH aus *Comanomonas testosteroni* und Figur 1d die kodierende Nukleinsäuresequenz zur Aminosäuresequenz von Figur 1c; Figur 1e zeigt die Aminosäuresequenz der 3α-HSDH aus *Rattus norvegicus* und Figur 1f die kodierende Nukleinsäuresequenz zur Aminosäuresequenz von Figur 1e; Figur 1g zeigt die kodierende Nukleinsäuresequenz der FDH-Mutante D221G und Figur 1h die Aminosäuresequenz zur Nukleinsäuresequenz von Figur 1g.

Figur 2 zeigt das SDS-Gel einer erfindungsgemäß hergestellten, gereinigten 7β-HSDH und zwar auf Bahn 1: Zellrohextrakt; Bahn 2: gereinigtes Protein; Bahn M: Page Rouler™, Molekulargewichtsmarker (Fermentas, Deutschland).

Figur 3 zeigt die Konstruktionsschema von (A) pET21a(+), (B) pET22b(+), (C) pCOLA (Mod) und (D) pET28a(+).

Figur 4 zeigt die Konstruktionsschemata von pET21a(+) FDH D221G (Fig. 4a) und pET21a(+) FDH 7β-HSDH (Fig. 4b).

Figur 5 zeigt das Konstruktionsschema von pCOLA(mod) 3α-HSDH.

Figur 6 zeigt einen Aktivitätsvergleich für /β-HSDH Wildtyp und die 7ß-HSDH Mutanten G39A und G39S, und zwar in Reihe A: die Auftragung der spezifischen Enzymaktivität gegen unterschiedliche eingesetzte Substratkonzentrationen bei konstanter Kofaktorkonzentration von 100 μM; und in Reihe B die Auftragung der Enzymaktivität gegen unterschiedliche eingesetzt Kofaktorkonzentrationen bei konstanter Substratkonzentration von 0,3 mM.

Figur 7 zeigt das HPLC-Chromatogramm der Biotransformation con DHCS mit 7β-HSDH und 3α-HSDH im Ganzzellprozess. Gezeigt ist das HPLC-Chromatogramm der Ganzzellumsetzung des Stammes *E. coli* BL21 (DE3) hdhA⁻

KanR⁺ pET21a(+) FDH 7β-HSDH pCOLA(mod) 3α-HSDH. Als Startbedingungen wurden 50 mM Substrat, 400 mM Cosubstrat, pH 6,0 gewählt. Die Probenahme erfolgte nach 48 h. Zu sehen sind die Peaks von 12-Keto-UDCS (1), eines unbekannten Nebenprodukts (2), 3,12-Diketo-UDCS(3), 7,12-Diketo-UDCS (4) und DHCS (5).

Figur 8 zeigt das Konstruktionsschema des Triple-Vektors pET21a(+) FDH 7beta (G39A) der die kodierenden Sequenzen für FDH D221G, 7β-HSDH G39A und 3α-HSDH trägt.

Figur 9 zeigt den Verlauf der Ganzzell-Biotransformation von DHCS. Es sind die Peakflächenanteile (nach HPLC Analyse) von 12-keto-UDCS, 3,12-diketo-UDCS, 7,12-diketo-UDCS und DHCS im zeitlichen Verlauf angegeben.

Figur 10 zeigt einen Sequenzvergleich zwischen dem 7β-HSDH-Wildtyp und ausgewählten Mutanten. Die als "7beta-HSDH Wildtyp", "7beta-HSDH G39D", "7beta-HSDH G39D R40L", "7beta-HSDH G39D R40I" und "7beta-HSDH G39D R40V" bezeichneten Sequenzen entsprechen den SEQ ID NO:2, SEQ ID NO:37, SEQ ID NO:38 und SEQ ID NO:39.

Figur 11 zeigt eine enzymkinetische Untersuchung der 7β-HSDH und deren Mutanten. Aufgetragen ist die spezifische Enzymaktivität gegen unterschiedliche eingesetzte Substratkonzentrationen (DHCA-Konzentration) bei einer konstanten Kofaktorkonzentration von 0,1 mM NADPH bzw. 0,5 mM NADH.

Figur 12 zeigt eine schematische Darstellung einer erfindungsgemäßen zweistufigen enzymatischen Reduktion von Dehydrocholsäure zu 12-keto-Ursodesoxycholsäure, wobei eine NADH-abhängige 7ß-HSDH eingesetzt wird und eine Formiatdehydrogenase (FDH) zur Kofaktorregenerierung verwendet wird.

Figur 13 zeigt eine schematische Darstellung des Konstruktionsprinzips der Vektoren pFr7(D), pFr7(DI), pFr7(DL) und pFr7(DV) die zur Expression verschiedener NADH-abhängigen Mutanten von 7ß-HSDH eingesetzt wurden.

Figur 14 zeigt Gallensalzanteile von Biotransformationsansätzen unter Verwendung von NADH-abhängigen 7β-HSDH-Mutanten. Gezeigt sind Ergebnisse nach 24 h Prozesszeit und Einsatz von 100 mM Substrat (DHCA).

Figur 15 zeigt eine schematische Darstellung des Vektors pFr3T7(D).

Figur 16 zeigt das Ergebnis einer Ganzzellbiotransformation mit dem Stamm *E. coli* BL49 pFr3T7(D), welcher die NADH-abhängige 7β-HSDH (G39D), eine NADH-abhängige 3α-HSDH und eine NADH-abhängige FDH beinhaltet. Die Biotransformationen wurden bei folgenden Bedingungen durchgeführt: 20 mL Reaktionsvolumen, 17,7 g/lBTM Zellen, 100 mM DHCA, 500 mM Ammoniumformiat, 26 % Glycerin, 50 mM MgCl2, 50 mM KPi-Puffer (pH 6,5). Der pH wurde während der ersten 5 Stunden stündlich manuell mit Ameisensäure auf den Ausgangswert eingestellt.

Figur 17 zeigt schematische Darstellungen verschiedener verwendeter Vektoren: a) pF(G)r7(A)r3 (wobei dieser dem in Figur 8 gezeigten Vektor entspricht) und b) pF(G)r7(S)r3.

Figur 18 zeigt eine schematische Darstellung der zweistufigen enzymatischen Reduktion von Dehydrocholsäure zu 12-keto-Ursodesoxycholsäure, wobei eine NADPH-abhängige 7ß-HSDH eingesetzt wird und eine Formiatdehydrogenase (FDH)-Mutante, die sowohl NADPH- als auch NADH regeneriert, zur Kofaktorregenerierung verwendet wird.

Figur 19 zeigt einen zeitlich aufgelösten Verlauf der Biotransformation mit dem Stamm *E. coli* BL49 pF(G)r7(A)r3 im Litermaßstab. Der Ansatz enthielt 70 mM DHCA, 17,7 g/l BTM des gelagerten Biokatalysators, 500 mM Natriumformiat, 26 % (v/v) Glycerin, 50 mM $MgCl_2$, in 50 mM KPi-Puffer (pH 6,5). Durch pH-Regelung wurde der pH während der gesamten Biotransformation auf pH 6,5 gehalten.

Figur 20 zeigt eine schematische Darstellung des Vektors p3T7(A)rG.

Figur 21 zeigt eine schematische Darstellung des Vektors p7(A)T3rG.

Figur 22 zeigt einen zeitlich aufgelösten Verlauf der Biotransformation mit dem Stamm *E. coli* BL49 p7(A)T3rG, welcher eine GDH zur Kofaktorregenerierung beinhaltet. Der Ansatz enthielt 100 mM DHCA, 17,7 g /l BTM des gelagerten Biokatalysators, 500 mM Glucose, 10 mM $MgCl_2$, in 50 mM KPi-Puffer (pH 7) ohne (obere Teilfigur) und mit 0,1 mM NAD (untere Teilfigur). Der pH wurde manuell mit Kalilauge auf den Ausgangswert eingestellt.

Figur 23 zeigt schematische Darstellungen verschiedener Vektoren: a) Vektor pF(G)r7(A) und b) Vektor pFr3.

Figur 24 zeigt eine schematische Darstellung der zweistufigen enzymatischen Reduktion von Dehydrocholsäure zu 12-keto-Ursodesoxycholsäure unter Verwendung von zwei unterschiedlichen Ganzzellbiokatalysatoren. Die Reaktionen A und D werden hierbei durch einen 7β-HSDH enthaltenden Ganzzellbiokatalysator katalysiert, die Reaktionen B und C werden von einen 3α-HSDH enthaltenden Ganzzellbiokatalysator katalysiert. Im Verlauf der zweistufigen Umsetzung muss das Intermediat 3,12-diketo-Ursodesoxycholsäure aus dem 7β-HSDH enthaltenden Ganzzellbiokatalysator heraus und in den 3α-HSDH enthaltenden Ganzzellbiokatalysator hinein gelangen, das Intermediat 7,12-diketo-Ursodesoxycholsäure muss aus dem 3α-HSDH enthaltenden Ganzzellbiokatalysatoren heraus und in den 7β-HSDH enthaltenden Ganzzellbiokatalysator hinein gelangen.

Figur 25 zeigt die Gallensalzanteile von Biotransformationsansätzen nach 24 h bei Einsatz von 70 mM Substrat (DHCA). Gezeigt sind die Ansätze bei Einsatz unterschiedlicher Anteile an den beiden Biokatalysatorstämmen *E. coli* BL49 pF(G)r7(A) und *E. coli* BL49 pFr3.

Figur 26 zeigt einen zeitlich aufgelösten Verlauf der Biotransformation mit dem Biokatalysatoren *E. coli* BL49 pF(G)r7(A) und *E. coli* BL49 pFr3 Litermaßstab. Der Ansatz enthielt 90 mM DHCA, 8,85 g /l $_{BTM}$ *E. coli* BL49 pF(G)r7(A) und 8,85 g/l $_{BTM}$ *E. coli* BL49 pFr3, 500 mM Ammoniumformiat, 26 % (v/v) Glycerin, 50 mM $MgCl_2$, in

50 mM KPi-Puffer (pH 6,5). Durch pH-Regelung wurde der pH während der gesamten Biotransformation mit Ameisensäure auf pH 6,5 gehalten.

## Spezielle Ausführungsformen der Erfindung

Die Erfindung betrifft insbesondere die speziellen Ausführungsformen gemäß den beiliegenden Ansprüchen.
Weiterhin werden hierin folgende Gegenstände beschrieben:
Eine 7ß-Hydroxysteroiddehydrogenase (7ß-HSDH)-Mutante, welche wenigstens die stereospezifische enzymatische Reduktion eines 7-Ketosteroids zum korrespondierenden 7-Hydroxysteroid katalysiert, wobei die Mutante im Vergleich zum nicht-mutierten Enzym, wie insbesondere ein Enzym umfassend SEQ ID NO:2 und/oder wenigstens das Sequenzmotiv VMVGRRE gemäß Position 36 bis 42 davon, eine verringerte Substratinhibition (insbesondere für das 7-Ketosteroid-Substrat) aufweist; und/oder eine veränderte Kofaktor-Nutzung (z.B. erhöhte, veränderte Spezifität bezüglich eines Kofaktors (insbesondere NADH bzw. NADPH) oder eine erweiterte Abhängigkeit, das heißt Nutzung eines zusätzlichen, bisher nicht genutzten Kofaktors) aufweist.
Insbesondere beschrieben wird eine solche Mutante, die keine Substratinhibition zeigt oder die einen $K_i$-Wert für das 7-Ketosteroid-Substrat, insbesondere für Dehydrocholsäure (DHCS), im Bereich von >10 mM, wie z.B. bei 11 bis 200 mM, 12 bis 150 mM, 15 bis 100 mM, aufweist.
Weiterhin wird beschrieben eine solche Mutante, wobei die spezifische Aktivität (U/mg) in Gegenwart des Kofaktors NADPH im Vergleich zum nichtmutierten Enzym um wenigsten 1, 5 oder 10 % , insbesondere aber wenigstens um das 1-fache, insbesondere um das 2- bis 10-fache erhöht oder erniedrigt ist; oder im Wesentlichen fehlt und durch die Nutzung von NADH ersetzt ist, oder eine erweiterte Nutzung von NADPH und HADH aufweist).
Weiterhin wird beschrieben eine solche Mutante, welche wenigstens die stereospezifische enzymatische Reduktion eines 7-Ketosteroids zum korrespondierenden 7-Hydroxysteroid katalysiert, gegebenenfalls nach einer der vorhergehenden Ausführungsformen, wobei die Mutante wenigstens eine Mutation in der Aminosäure-Sequenz gemäß SEQ ID NO: 2 oder einer davon abgeleiteten Aminosäuresequenz mit wenigstens 60% Sequenzidentität, wie z.B. wenigstens 65, 70, 75, 80, 85, oder 90, wie z.B. wenigstens 91, 92, 93, 94, 95, 96, 97, 98, 99 oder 99,5% zu dieser Sequenz.
Weiterhin wird beschrieben eine solche 7ß-Hydroxysteroiddehydrogenase (7ß-HSDH)-Mutante, welche wenigstens die stereospezifische enzymatische Reduktion eines 7-Ketosteroids zum korrespondierenden 7-Hydroxysteroid katalysiert, gegebenenfalls nach einer der vorhergehenden Ausführungsformen, wobei die Mutante wenigstens eine Mutation im Sequenzmotiv VMVGRRE gemäß Position 36 bis 42 von SEQ ID NO:2 oder in dem korrespondierenden Sequenzmotiv einer davon abgeleiteten Aminosäuresequenz mit wenigstens 60% Sequenzidentität aufweist, wie z.B. wenigstens 65, 70, 75, 80, 85, oder 90, wie z.B. wenigstens 91, 92, 93, 94, 95, 96, 97, 98, 99 oder 99,5% zu dieser Sequenz.
Weiterhin wird beschrieben eine solche Mutante, ausgewählt unter

a) den Einfachmutanten G39$X_1$ und R40$X_2$.,
b) den Zweifach-Mutanten (G39$X_1$, R40$X_2$), (R40$X_2$, R41$X_3$) und (G39$X_1$, R41$X_3$) oder
c) der Dreifach-Mutante (G39$X_1$, R40$X_2$, R41$X_3$),

(jeweils bezogen auf SEQ ID NO:2)
wobei $X_1$, $X_2$ und $X_3$ jeweils unabhängig voneinander für eine beliebige, insbesondere eine beliebige, die Substratinhibition verringernde und/oder die Kofaktornutzung oder Kofaktor-Abhängigkeit modifizierende, von G bzw. R verschiedene, insbesondere natürliche Aminosäure stehen;

oder
d) den korrespondierenden Einfach-, Zweifach- oder Dreifach-Mutanten einer von SEQ ID NO:2 abgeleiteten Aminosäuresequenz mit wenigstens 60% Sequenzidentität, wie z.B. wenigstens 65, 70, 75, 80, 85, oder 90, wie z.B. wenigstens 91, 92, 93, 94, 95, 96, 97, 98, 99 oder 99,5% zu dieser Sequenz.

Beispiele solcher Einfachmutanten umfassen: G39A, G39S, G39D, G39V, G39T, G39P, G39N, G39E, G39Q, G39H, G39R, G39K und G39W, und R40D, R40E, R40I, R40V, R40L, R40G, R40A.

Beispiele solcher Doppelmutanten umfassen:

Zweierkombinationen obiger G39$X_1$ und R40$X_2$-Mutanten, wobei $X_1$ insbesondere für D oder E steht und/oder $X_2$ eine beliebige Aminosäure, insbesondere proteinogene Aminosäure, wie insbesondere eine Aminosäure mit aliphatischer Seitenkette sein kann, wie z.B. (G39D,R40I), (G39D,R40L), (G39D,R40V); sowie die analogen Doppelmutanten mit G39E anstelle von G39D; sowie
Zweierkombinationen obiger G39$X_1$-Mutanten oder R40$X_2$-Mutanten mit R41$X_3$-Mutanten, worin $X_3$ eine be-

liebige, insbesondere eine die Substratinhibition verringernde und/oder die Kofaktornutzung oder Kofaktorabhängigkeit modifizierende, von R verschiedene insbesondere natürliche Aminosäure ist, wie z.B. des Typs (G39$X_1$,R41$X_3$) oder (R40$X_2$,R41$X_3$), z.B. mit $X_1$=D oder E; $X_2$=I, L oder V; $X_3$= N, I, L oder V)
wie z.B. (R40D,R41I); (R40D,R41L); (R40D,R41V); (R40I,R41I); (R40V,R41I), (R40L,R41I).

Beispiele solcher Dreifachmutanten umfassen beliebige Dreier-Kombinationen obiger Einfachmutanten G39$X_1$, R40$X_2$ und R41$X_3$; wobei $X_1$, $X_2$ und $X_3$ wie oben definiert sind; insbesondere aber wobei $X_1$ für D, oder E steht und/oder $X_2$ und $X_3$ unabhängig voneinander für eine beliebige Aminosäure, insbesondere eine proteinogene Aminosäure stehen, wie insbesondere Dreichfachmutanten des Typs (G39$X_1$=D oder E; R40$X_2$=I, L oder V; R41$X_3$= N, I, L oder V), wie z.B. (G39D,R40I,R41N).

Gegebenenfalls können obige Mutanten zusätzlich oder alternativ, insbesondere zusätzlich, wenigstens eine weitere Substitution , wie z.B. 1, 2, 3 oder 4 Substitutionen in den Positionen K44, R53, K61 und R64. Hierbei können diese Reste unabhängig voneinander durch eine beliebige Aminosäure, insbesondere eine proteinogene Aminosäure ersetzt sein, insbesondere eine Substitution derart, dass die so erzeugte Mutente eine verringerte Substratinhibition (insbesondere für das 7-Ketosteroid-Substrat) aufweist; und/oder eine veränderte Kofaktornutzung oder Kofaktorabhängigkeit (z.B. erhöhte, veränderte Spezifität bezüglich eines Kofaktors oder eine erweiterte Abhängigkeit, das heißt Nutzung eines zusätzlichen, bisher nicht genutzten Kofaktors) wie hierin definiert aufweist.

Weiterhin ist hierin beschrieben eine Nukleinsäuresequenz kodierend für eine der obigen 7ß-HSDH Mutanten.

Weiterhin ist hierin beschrieben eine Expressionskassette, umfassend eine solche Nukleinsäuresequenz unter der genetischen Kontrolle wenigstens einer regulativen Nukleinsäuresequenz, sowie ggf. kodierende Sequenzen für wenigstens ein (wie z.B. 1 ,2 oder 3) weiteres Enzym, ausgewählt unter Hydroxysteroiddehydrogenasen, insbesondere 3α-HSDH, und zur Kofaktorregenerierung geeignete Dehydrogenasen, wie z.B. FDH, GDH, ADH, G-6-PDH, PDH. Insbesondere können die in einer Expressions-Kassette enthaltenen Enzyme verschiedene, bevorzugt aber gleiche Kofaktorenpaare nutzen, wie z.B. das Kofaktorenpaar $NAD^+$/NADH oder $NADP^+$/NADPH.

Weiterhin ist hierin beschrieben ein Vektor, umfassend wenigstens eine solche Expressionskassette.

Weiterhin ist hierin beschrieben ein rekombinanter Mikroorganismus, der wenigstens eine wie oben beschriebene Nukleinsäuresequenz oder wenigstens eine wie oben beschriebene Expressionskassette trägt oder wenigstens einen wie oben beschriebenen Vektor trägt und zusätzlich gegebenenfalls die kodierende Sequenz für eine 3α-Hydroxysteroiddehydrogenase (3α-HSDH trägt).

Außerdem ist hierin beschrieben ein Verfahren zur enzymatischen oder mikrobiellen Synthese von 7ß-Hydroxysteroiden, wobei man das korrespondierende 7-Ketosteroid in Gegenwart einer oben genannten 7ß-HSDH Mutante oder in Gegenwart eines diese Mutante exprimierenden rekombinanten Mikroorganismus umsetzt, und wenigstens ein gebildetes Reduktionsprodukt aus dem Reaktionsansatz gegebenenfalls isoliert.

Insbesondere ist dabei das zu reduzierende Ketosteroid ausgewählt unter

a) Dehydrocholsäure (DHCS),
b) 7-Keto-lithocholsäure (7-Keto-LCS),
c) 7,12-Diketo-lithocholsäure (7,12-Diketo-LCS) und
d) den Derivaten davon, wie insbesondere einem Salz, Amid oder Alkylester der Säure.

Insbesondere erfolgt dabei die Reduktion in Gegenwart (und unter Verbrauch) von NADPH und/oder NADH.

Außerdem wird hierin beschrieben ein Verfahren zur enzymatischen oder mikrobiellen Oxidation von 7ß-Hydroxysteroiden, wobei man das Hydroxysteroid in Gegenwart einer oben genannten 7β-HSDH-Mutante oder in Gegenwart eines diese Mutante exprimierenden Mikroorganismus umsetzt, und ein gebildetes Oxidationsprodukt aus dem Reaktionsansatz gegebenenfalls isoliert.

Insbesondere ist dabei das 7ß-Hydroxysteroid die 3,12-Diketo-7ß-CS oder ein Derivat davon, wie insbesondere ein Salz, Amid oder Alkylester, ist.

Insbesondere erfolgt dabei die Oxidation in Gegenwart (und unter Verbrauch) von $NADP^+$ und/oder $NAD^+$.

Insbesondere werden die verbrauchten Redoxäquivalente chemisch, elektrochemisch oder enzymatisch, insbesondere in situ, regeneriert.

Insbesondere wird dabei verbrauchtes NADPH durch Kopplung mit einem NADPH-regenerierenden Enzym regeneriert, wobei dieses insbesondere ausgewählt ist unter NADPH Dehydrogenasen, Alkoholdehydrogenasen (ADH), und NADPH regenerierenden Formiatdehydrogenasen (FDH), sowie Glucosedehydrogenase (GDH)-, Glucose-6-Phosphat-Dehydrogenase (G-6-PDH), oder Phosphit-Dehydrogenasen (PtDH), wobei das NADPH-regenerierenden Enzym gegebenenfalls von einem rekombinanten Mikroorganismus exprimiert wird; oder
verbrauchtes NADH durch Kopplung mit einem NADH-regenerierenden Enzym regeneriert, wobei dieses insbesondere ausgewählt ist unter NADH Dehydrogenasen, NADH regenerierenden Formiatdehydrogenasen (FDH), NADH regenerierenden Alkoholdehydrogenasen (ADH), NADH regenerierenden Glucose-6-Phosphat-Dehydrogenasen (G6PDH), NADH regenerierenden Phosphitdehydrogenasen (PtDH) sowie NADH regenerierenden Glucosedehydrogenasen

(GDH), wobei das NADH-regenerierende Enzym ggf. in einem rekombinanten Mikroorganismus exprimiert wird.

Die Expression des Kofaktor-regenerierenden Enzyms in einem rekombinanten Mikroorganismus ist dabei bevorzugt.

Das NADPH-regenerierende Enzym ist dabei ausgewählt unter natürlichen oder rekombinanten, isolierten oder angereicherten

e) Alkoholdehydrogenasen (EC 1.1.1.2) und
f) davon abgeleiteten funktionalen Äquivalenten

Das NADPH-regenerierende Enzym ist dabei ausgewählt unter Mutanten einer $NAD^+$-abhängigen Formiatdehydrogenase (FDH), welche insbesondere wenigstens die enzymatische Oxidation von Ameisensäure zu $CO_2$ katalysiert, wobei die Mutante im Vergleich zum nicht-mutierten Enzym ausschließlich oder zusätzlich, insbesondere zusätzlich $NADP^+$ als Kofaktor akzeptiert; oder
das NADH-regenerierende Enzym ist ausgewählt unter einer $NAD^+$-abhängigen FDH oder einer $NAD^+$-abhängigen GDH, wie insbesondere einer FDH aus *Mycobacterium vaccae* N10, gemäß SEQ ID NO:36 und einer GDH aus *Bacillus subtilis* gemäß SEQ ID NO:48 (welche NADPH und/oder NADH regeneriert) oder einem zu den beiden genannten Enzymen jeweils funktional äquivalenten (bezüglich Kofaktor-Regenerierung), abgewandelten Form davon.

Die FDH-Mutante reduziert $NADP^+$ mit einer spezifischen Aktivität zu NADPH, die etwa 0,1 bis 1.000 %, wie etwa 1 bis 100 %, 5 bis 80 %oder 10 bis 50 %, der spezifischen Aktivität des nichtmutierten (Wildtyp-) Enzyms für die Reduktion von $NAD^+$ zu NADH entspricht.

In diesem Verfahren weist die $NADP^+$-akzeptierende FDH-Mutante wenigstens eine Mutation in der Aminosäure-Sequenz einer FDH aus *Mycobacterium vaccae* N10 gemäß SEQ ID NO: 36 oder einer davon abgeleiteten Aminosäuresequenz mit wenigstens 60% Sequenzidentität, wie z.B. wenigstens 65, 70, 75, 80, 85, oder 90, wie z.B. wenigstens 91, 92, 93, 94, 95, 96, 97, 98, 99 oder 99,5% zu dieser Sequenz, auf.

Insbesondere weist die $NADP^+$-akzeptierende Mutante wenigstens eine Mutation im Sequenzmotiv TDRHRL gemäß Position 221 bis 226 von SEQ ID NO:36 oder in dem korrespondierenden Sequenzmotiv einer davon abgeleiteten Aminosäuresequenz mit wenigstens 60% Sequenzidentität, wie z.B. wenigstens 65, 70, 75, 80, 85, oder 90, wie z.B. wenigstens 91, 92, 93, 94, 95, 96, 97, 98, 99 oder 99,5% zu dieser Sequenz, auf.

Nichtlimitierende Beispiele möglicherweise geeigneter FDH-Mutanten umfassen Mutationen in den Positionen D222 und /oder R223 von SEQ ID NO:36 Als Beispiele können genannt werden D222X mit X = G, A, K oder N; und R223X mit X = H oder Y, sowie Kombinationen von Mutationen in Position 222 und 223.

Insbesondere ist die $NADP^+$-akzeptierende Mutante ausgewählt unter der Einfachmutante D222G gemäß SEQ ID NO:36 (hierin häufiger auch als "D221G" Mutante bezeichnet (bei Zählung, ausgehend von Ala in Position 2 von SEQ ID NO:36 als erste Aminosäure) oder den korrespondierenden Einfachmutanten einer davon abgeleiteten Aminosäuresequenz mit wenigstens 60% Sequenzidentität, wie z.B. wenigstens 65, 70, 75, 80, 85, oder 90, wie z.B. wenigstens 91, 92, 93, 94, 95, 96, 97, 98, 99 oder 99,5% zu dieser Sequenz. Als konkrete Beispiele können genannt werde FDH Mutanten nach SEQ ID NO: 15, 19 und 35.

Weitere geeignete FDH Enzyme sind ausgehend von den z.B. aus *Candida boidinii* oder *Pseudomonas sp,* isolierbaren Wildtyp-Enzymen und Einführung wenigstens einer zu obigen Mutationen entsprechenden funktionale Mutation zur Veränderung der Kofaktor-Spezifität zugänglich. Zudem gibt es im Stand der Technik zahlreiche Untersuchungen zur Verbesserung verschiedener FDH Eigenschaften, wie chemische oder thermische Stabilität oder katalytische Aktivität. Diese sind z.B. in Tishkov et al., Biomolecular Engineering 23 (2006), 89-110 zusammengefasst. So können dort beschriebene einfache oder mehrfache Punktmutationen, wie z.B. zur Erhöhung der Enzymstabilität, mit den erfindungsgemäß beschriebenen Mutationen zur modifizierten Kofaktor-Nutzung kombiniert werden.

Weiterhin sind hierin beschrieben Nukleinsäuresequenzen, ausgewählt unter Nukleinsäuresequenzen

a) gleichzeitig kodierend für eine FDH Mutante wie oben beschrieben und eine 7ß-HSDH Mutante wie oben beschrieben und gegebenenfalls eine 3$\alpha$-HSDH;
b) gleichzeitig kodierend für eine FDH Mutante wie oben beschrieben und einer nichtmutierten 7ß-HSDH und eine 3$\alpha$-HSDH (anwendbar mit dem Gegenstand der Erfindung);
c) kodierend für ein Fusionsprotein umfassend eine FDH Mutante wie oben beschrieben und eine 7ß-HSDH Mutante wie oben beschrieben und gegebenenfalls eine 3$\alpha$-HSDH;
d) kodierend für ein Fusionsprotein umfassend eine FDH Mutante wie oben beschrieben und einer nichtmutierten 7ß-HSDH und eine 3$\alpha$-HSDH (anwendbar mit dem Gegenstand der Erfindung),
e) gleichzeitig kodierend für FDH-Wildtyp und eine 7$\beta$-HSDH-Mutante wie oben beschrieben und ggf. eine 3$\alpha$-HSDH;
f) kodierend für ein Fusionsprotein, umfassend den FDH-Wildtyp, eine 7$\beta$-HSDH-Mutante wie oben beschrieben und ggf. eine 3$\alpha$-HSDH; oder
g) gleichzeitig kodierend für eine GDH, einen 7$\beta$-HSDH-Wildtyp und eine 3$\alpha$-HSDH (anwendbar mit dem Gegenstand

der Erfindung);
h) kodierend für ein Fusionsprotein, umfassend eine GDH, einen 7β-HSDH-Wildtyp und eine 3α-HSDH (anwendbar mit dem Gegenstand der Erfindung);
i) gleichzeitig kodierend für eine GDH, eine 7β-HSDH-Mutante wie oben beschrieben und ggf. eine 3α-HSDH; und
k) kodierend für ein Fusionsprotein, umfassend eine GDH, eine 7β-HSDH-Mutante wie oben beschrieben und ggf. eine 3α-HSDH;

wobei die kodierenden Sequenzen unabhängig voneinander ein oder mehrfach in dem Konstrukt, wie z.B. in 2, 3, 4, 5, oder 6 bis 10 Kopien, enthalten sein können. Durch Wahl der geeignete Kopienzahl können so eventuell gegebene Aktivitätsunterschiede in den einzelnen Expressionsprodukten kompensiert werden.

Weiterhin ist hierin beschrieben eine Expressionskassette, umfassend eine solche Nukleinsäuresequenz unter der genetischen Kontrolle wenigstens einer regulativen Nukleinsäuresequenz, wobei die kodierenden Sequenzen unabhängig voneinander ein oder mehrfach in dem Konstrukt, wie z.B. in 2, 3, 4, 5, oder 6 bis 10 Kopien, enthalten sein können. Durch Wahl der geeignete Kopienzahl können so eventuell gegebene Aktivitätsunterschiede in den einzelnen Expressionsprodukten kompensiert werden.

Weiterhin ist hierin beschrieben ein Vektor, umfassend wenigstens eine solche Expressions-kassette, wobei die kodierenden Sequenzen unabhängig voneinander ein oder mehrfach in dem Vektorkonstrukt, wie z.B. in 2, 3, 4, 5, oder 6 bis 10 Kopien, enthalten sein können. Durch Wahl der geeignete Kopienzahl können so eventuell gegebene Aktivitätsunterschiede in den einzelnen Expressionsprodukten kompensiert werden.

Weiterhin ist hierin beschrieben ein rekombinanter Mikroorganismus, der wenigstens eine solche Nukleinsäuresequenz oder wenigstens eine solche Expressionskassette trägt oder wenigstens einen solchen Vektor trägt.

Gegenstand der Erfindung ist insbesondere ein rekombinanter Mikroorganismus, welcher zur gleichzeitigen Expression von 7ß-HSDH (Wildtyp), einer $NADP^+$-akzeptierenden FDH Mutante und/ oder den entsprechenden FDH-Wildtyp und von 3α-HSDH befähigt ist; oder welcher zur gleichzeitigen Expression von 7β-HSDH (Wildtyp), einer hierin beschriebenen GDH und einer hierin beschriebenen 3α-HSDH befähigt ist.

Weiterhin ist hierin beschrieben ein rekombinanter Mikroorganismus, welcher zur gleichzeitigen Expression einer 7ß-HSDH Mutante, einer $NADP^+$-akzeptierenden FDH Mutante und/ oder den entsprechenden FDH-Wildtyp und gegebenenfalls von 3α-HSDH befähigt ist; oder welcher zur gleichzeitigen Expression einer 7β-HSDH-Mutante, einer hierin beschriebenen GDH und ggf. einer hierin beschriebenen 3α-HSDH befähigt ist.

Dabei ist die 7ß-HSDH-Mutante eine Mutante wie oben beschrieben.

Dabei ist die FDH-Mutante eine Mutante gemäß obiger Definition und der FDH Wildtyp ist eine FDH aus *Mycobacterium vaccae* N10 gemäß SEQ ID NO: 36 oder eine davon abgeleitete FDH mit wenigstens 60% Sequenzidentität, wie z.B. wenigstens 65, 70, 75, 80, 85, oder 90, wie z.B. wenigstens 91, 92, 93, 94, 95, 96, 97, 98, 99 oder 99,5% zu dieser Sequenz.

Dabei ist die 3α-HSDH ein Enzym, umfassend eine Aminosäuresequenz nach SEQ ID NO: 6 oder 8 oder 22 oder eine davon abgeleitete Aminosäuresequenz mit wenigstens 60% Sequenzidentität, wie z.B. wenigstens 65, 70, 75, 80, 85, oder 90, wie z.B. wenigstens 91, 92, 93, 94, 95, 96, 97, 98, 99 oder 99,5% zu dieser Sequenz.

Gegenstand der Erfindung ist insbesondere ein solcher rekombinanter Mikroorganismus welcher die kodierenden Sequenzen für 7ß-HSDH, FDH und 3α-HSDH auf einem oder mehreren (verschiedenen) Expressionskonstrukten trägt. Gegenstand der Erfindung sind somit rekombinante Mikroorganismen, welche mit einem Einplasmidsystem modifiziert (wie z.B. transformiert) sind, das die kodierenden Sequenzen für 7ß-HSDH, FDH bzw. Mutanten davon und 3α-HSDH bzw. Mutanten davon in einer oder mehreren Kopien, wie z.B. in 2, 3, 4, 5, oder 6 bis 10 Kopien, tragen. Gegenstand der Erfindung sind somit auch rekombinante Mikroorganismen, welche mit einem Einplasmidsystem modifiziert (wie z.B. transformiert) sind, das die kodierenden Sequenzen für 7β-HSDH, GDH bzw. Mutanten davon und 3α-HSDH bzw. Mutanten davon in einer oder mehreren Kopien, wie z.B. in 2, 3, 4, 5 oder 6 bis 10 Kopien tragen. Die 7ß-HSDH, sowie FDH und 3α-HSDH bzw. deren Mutanten können aber auch auf 2 oder 3 getrennten, miteinander kompatiblen Plasmiden in einer oder mehreren Kopien enthalten sein. Geeignete Basisvektoren zur Herstellung von Einplasmidsystemen und Multicopy-Plasmiden sind dem Fachmann bekannt. Als Beispiele können genannt werden für Einplasmidsystem, z.B. pET21aund für Multicopyplasmide z.B. die von der Firma Novagen vertriebenen Duet-Vektoren, wie pACYCDuet-1, pETDuet-1, pCDFDuet-1, pRSFDuet-1 und pCOLADuet-1. Derartige Vektoren, deren Kompatibilität mit anderen Vektoren und mikrobiellen Wirtsstämmen sind z.B. dem "User Protocol TB340 Rev. E0305 der Firma Novagen zu entnehmen.

Die optimale Kombination von Enzymen für die Generierung von Plasmidsystemen kann der Fachmann unter Berücksichtigung der Lehre der vorliegenden Erfindung ohne unzumutbaren Aufwand vornehmen. So kann der Fachmann beispielsweise in Abhängigkeit von der Kofaktorspezifität des jeweils verwendeten 7β-HSDH-Enzyms das zur Kofaktorregenerierung am besten geeignete Enzym, ausgewählt unter den oben genannten Dehydrogenasen, insbesondere FDH, GDH und den jeweiligen Mutanten davon, auswählen.

Weiterhin besteht die Möglichkeit, die zur Umsetzung gewählten Enzyme auf zwei oder mehrere Plasmide zu verteilen und mit den so hergestellten Plasmiden zwei oder mehrere verschiedene rekombinante Mikroorganismen

herzustellen, die dann gemeinsam für die erfindungsgemäße biokatalytische Umsetzung eingesetzt werden. Die jeweilige zur Herstellung des Plasmids verwendete Enzymkombination kann dabei insbesondere auch unter der Vorgabe einer vergleichbaren Kofaktor-Nutzung erfolgen. So kann beispielsweise ein erster Mikroorganismus mit einem Plasmid modifiziert werden, welches die kodierende Sequenz für eine NADPH-abhängige 7β-HSDH-Mutante und eine diesen Kofaktor regenerierende FDH-Mutante gemäß vorliegender Erfindung tragen. oder welches die kodierende Sequenz für eine NADPH-abhängige 7β-HSDH-Mutante und NADPH-regenerierende GDH, oder die kodierende Sequenz für eine NADH-abhängige 7β-HSDH und eine NADH-regenerierende FDH und / oder GDH trägt. Ein zweiter Mikroorganismus kann dagegen mit einem Plasmid modifiziert sein, das die kodierende Sequenz für eine NADH-abhängige 3α-HSDH und die kodierende Sequenz für einen NADH-regenerierenden FDH-Wildtyp und/ oder für eine NADH-regenerierende GDH trägt. Beide Mikroorganismen können dann gleichzeitig zur erfindungsgemäßen biokatalytischen Umsetzung eingesetzt werden.

Gegenstand der Erfindung ist auch ein Verfahren zur mikrobiellen Herstellung von Ursodesoxycholsäure (UDCS) der Formel (1)

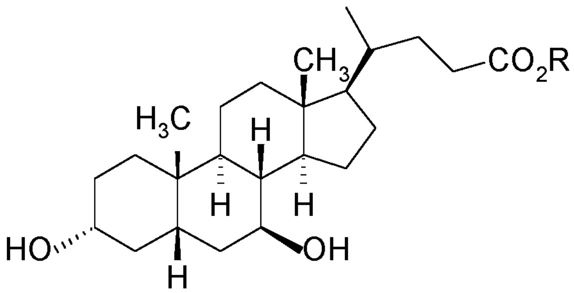

(1)

worin

R für Alkyl, $NR^1R^2$, H, ein Alkalimetallion oder $N(R^3)_4^+$steht, worin die Reste $R^3$ gleich oder verschieden sind und für H oder Alkyl stehen,

wobei man

a) gegebenenfalls eine Cholsäure (CS) der Formel (2)

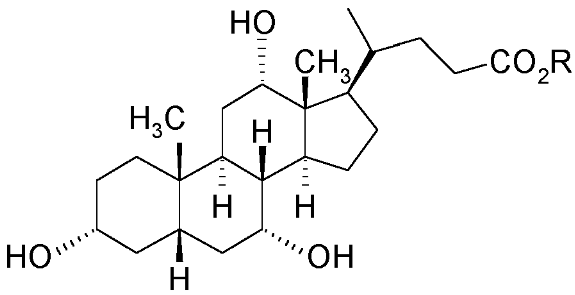

(2)

worin R die oben angegebenen Bedeutungen besitzt, zur Dehydrocholsäure (DHCS) der Formel (3)

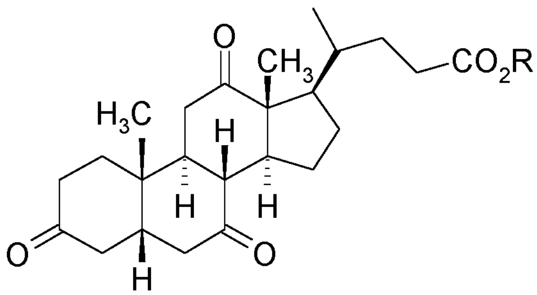

(3)

worin R die oben angegebenen Bedeutungen besitzt, chemisch oxidiert;

b) DHCS in Gegenwart wenigstens einer 7ß-HSDH und in Gegenwart wenigstens einer 3α-HSDH zur korrespondierenden 12-Keto-ursodesoxycholsäure (12-Keto UDCS) der Formel (5)

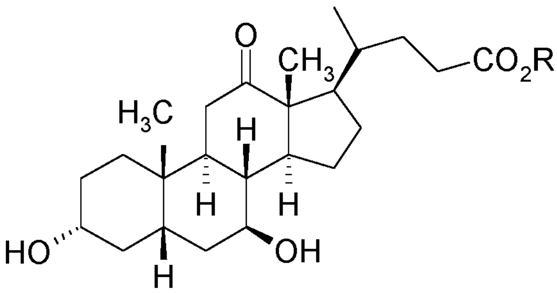

(5)

worin R die oben angegebenen Bedeutungen besitzt, (in Gegenwart und unter Verbrauch von NADH und /oder NADPH) reduziert und anschließend

c) 12-Keto-UDCS der Formel (5) chemisch zu UDCS reduziert; und

d) das Reaktionsprodukt gegebenenfalls weiter aufreinigt;

wobei die Umsetzungen der Stufe b) mikrobiell, d.h. in Gegenwart ganzer Zellen eines wie oben beschriebenen rekombinanten Mikroorganismus erfolgen, wobei der Mikroorganismus die zur Umsetzung und Kofaktorregnerierung erforderlichen Enzyme in einer hierin näher beschriebenen Weise tragen, oder aber unter Verwendung wenigstens einer hierin beschriebenen Nukleinsäuresequenz.

Beispielsweise kann dabei DHCS in Gegenwart wenigstens einer 7β-HSDH zur 3,12-Diketo-7ß-cholansäure (3,12-Diketo-7ß-CS) der Formel (4)

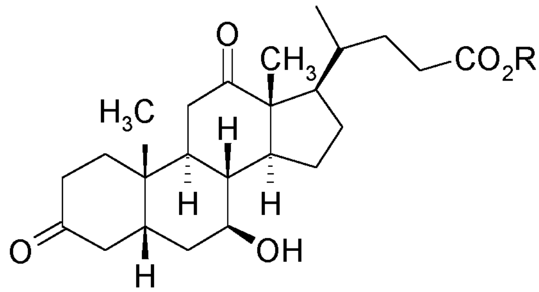

(4)

(in Gegenwart und unter Verbrauch von NADPH) reduziert werden, und
3,12-Diketo-7β-CS in Gegenwart wenigstens einer 3α-Hydroxysteroiddehydrogenase (3α-HSDH) oder Mutante davon zur korrespondierenden 12-Keto-ursodesoxycholsäure (12-Keto UDCS) der Formel (5)

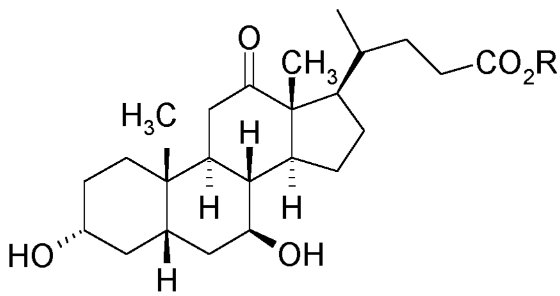

(5)

worin R die oben angegebenen Bedeutungen besitzt, (in Gegenwart und unter Verbrauch von NADH und/oder NADPH)

reduziert werden.

Weiterhin ist aber auch eine Reaktionssequenz, umfassend die Reduktion von DHCS zunächst mit 3α-HSDH und die anschließende Reduktion des dabei gebildeten Reaktionsprodukts mit 7ß-HSDH denkbar, sowie ein gleichzeitiger Ablauf beider Reaktionssequenzen, aufgrund der gleichzeitigen Anwesenheit beider HSDHs.

Gegenstand der Erfindung ist insbesondere ein Verfahren, wobei man einen rekombinanten Mikroorganismus einsetzt, der beispielsweise wenigstens eine 7β-HSDH , wenigstens eine $NADP^+$-akzeptierende FDH Mutante und wenigstens eine 3α-HSDH,wie insbesondere gemäß SEQ ID NO: 6, 8 oder 22 oder Mutanten davon im eine Sequenzidentität von mindestens etwa 60%, gleichzeitig exprimiert.; oder der wenigstens eine 7ß-HSDH, wenigstens eine GDH und wenigstens eine 3α-HSDH,wie insbesondere gemäß SEQ ID NO: 6, 8 oder 22 oder Mutanten davon im eine Sequenzidentität von mindestens etwa 60%, gleichzeitig exprimiert.

Weiterhin wird hierin ein Bioreaktor zur Durchführung eines obigen Verfahrens beschrieben, insbesondere enthaltend wenigstens eines der Enzyme (7ß-HSDH, FDH, und/oder 3α-HSDH bzw. deren Mutanten; oder 7ß-HSDH, GDH und/oder 3α-HSDH bzw. deren Mutanten) oder einen wenigstens eines dieser Enzyme rekombinant exprimierenden rekombinanten Mikroorganismus, insbesondere in immobilisierter Form.

Vorliegende Erfindung ist nicht auf die hierin beschriebenen konkreten Ausführungsformen beschränkt. Der Fachmann wird durch die Lehre der vorliegenden Erfindung vielmehr in die Lage versetzt, ohne unzumutbaren Aufwand weitere Ausgestaltungen der Erfindung bereitzustellen. So kann er beispielsweise auch weitere Enzymmutanten gezielt generieren und diese auf das gewünschte Eigenschaftsprofil (verbesserte Kofaktor-Abhängigkeit und /der Stabilität, verringerte Substratinhibition) screenen und optimieren; oder weitere geeignete Wildtypenzyme (7ß- und 3α-HSDHs, FDHs, GDHs ADHs usw) isolieren und erfindungsgemäß verwenden. Weiterhin kann er beispielsweise je nach Eigenschaftsprofil (insbesondere Kofaktor-Abhängigkeit) der verwendeten HSDHs, wie insbesondere 7ß-HSDH und 3α-HSDH oder Mutanten davon, geeignete zur Kofaktorregenerierung brauchbare Dehydrogenasen (GDH, FHD, ADH usw.) und Mutanten davon auswählen, und die ausgewählten Enzyme auf einen oder mehrere Expressionskonstrukte oder Vektoren verteilen und damit erforderlichenfalls einen oder mehrere rekombinante Mikroorganismen erzeugen, die dann ein optimiertes ganzzellbasiertes Herstellungsverfahren ermöglichen.

## Weitere Ausgestaltungen der Erfindung

1. Allgemeine Definitionen und verwendete Abkürzungen

Werden keine anderen Angaben gemacht, so bezeichnet der Begriff "7ß-HSDH" ein Dehydrogenase-Enzym, welches wenigstens die stereospezifische und/oder regiospezifische Reduktion von DHCS oder 7,12-Diketo-3α-CS (7,12-Diketo-LCS) zu 3,12-Diketo-7ß-CS oder 12-Keto-UDCS insbesondere unter stöchiometrischem Verbrauch von NADPH, und gegebenenfalls die entsprechende Umkehrreaktion katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen. Geeignete Nachweisverfahren sind z.B. im folgenden experimentellen Teil beschrieben oder aus der Literatur bekannt (z.B. *Characterization of NADP-dependent 7 beta-hydroxysteroid dehydrogenases from Peptostreptococcus productus and Eubacterium aerofaciens.* S Hirano and N Masuda. Appl Environ Microbiol. 1982). Enzyme dieser Aktivität sind unter der EC Nummer 1.1.1.201 klassifiziert.

Werden keine anderen Angaben gemacht, so bezeichnet der Begriff "3α-HSDH" ein Dehydrogenase-Enzym, welches wenigstens die stereospezifische und/oder regiospezifische Reduktion von 3,12-Diketo-7ß-CS oder DHCS zu 12-Keto-UDCS oder 7,12-Diketo-3α-CS (7,12-Diketo-LCS), insbesondere unter stöchiometrischem Verbrauch von NADH und/oder NADPH, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus Comanomonas testosteroni (z.B. ATCC11996) erhältlich. Eine NADPH abhängige 3α-HSDH ist z.B. aus der Nagern bekannt und ebenfalls einsetzbar. (Cloning and sequencing of the cDNA for rat liver 3 alphahydroxysteroid/dihydrodiol dehydrogenase, J E Pawlowski, M Huizinga and T M Penning, May 15, 1991 The Journal of Biological Chemistry, 266, 8820-8825). Enzyme dieser Aktivität sind unter der EC Nummer 1.1.1.50 klassifiziert.

Werden keine anderen Angaben gemacht, so bezeichnet der Begriff "GDH" ein Dehydrogenase-Enzym, welches wenigstens die Oxidation von β-D-Glucose zu D-Glucono-1,5-Lacton unter stöchiometrischem Verbrauch von $NAD^+$ und/oder $NADP^+$ sowie ggf. die entsprechende Umkehrreaktion katalysiert. Geeignete Enzyme sind z.B. aus *Bacillus subtili* oder *Bacillus megaterium* erhältlich. Enzyme dieser Aktivität sind unter der EC Nummer 1.1.1.47 klassifiziert. Werden keine anderen Angaben gemacht, so bezeichnet der Begriff "FDH" ein Dehydrogenase-Enzym, welches wenigstens die Oxidation von Ameisensäure (oder korrespondierenden Formiat-Salzen) zu Kohlendioxid unter stöchiometrischem Verbrauch von $NAD^+$ und/oder $NADP^+$, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus *Candida boidinii, Pseudomonas sp,* oder *Mycobacterium vaccae* erhältlich. Enzyme dieser Aktivität sind unter der EC Nummer 1.2.1.2 klassifiziert.

Unter einer "Reinform" oder einem "reinen" oder "im wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie .z.B. der Biuret-Methode oder dem Proteinnachweis nach Lowry.et al.(vgl Beschreibung in R.K. Scopes, Protein Purification, Springer Verlag, New York, Heidelberg, Berlin (1982)). Unter einem "Redoxäquivalent" versteht man eine als Elektronendonor bzw. Elektronenakzeptor brauchbare, niedermolekulare organische Verbindung, wie beispielsweise Nikotinamidderivate wie $NAD^+$ und $NADH^+$ bzw. deren reduzierte Formen NADH bzw. NADPH. "Redoxäquivalent" und "Kofaktor" werden im Kontext der vorliegenden Erfindung als Synonym verwendet. So kann ein "Kofaktor" im Sinne der Erfindung auch als "redoxfähiger Kofaktor", d.h. als Kofaktor, der in reduzierter und einer oxidierter Form vorliegen kann, umschrieben werden.

Unter einem "verbrauchten" Kofaktor versteht man diejenige reduzierte bzw. oxidierte Form des Kofaktors, die im Verlauf einer vorgegebene Reduktions- bzw. Oxidationsreaktion eines Substrats in die korrespondierende oxidierte bzw. reduzierte Form überführt wird. Durch Regenerierung wird die bei der Reaktion gebildete oxidierte bzw. reduzierte Kofaktor-Form wieder in die reduzierte bzw. oxidierte Ausgangsform zurück überführt, so dass diese für die Umsetzung des Substrats wieder zur Verfügung steht.

Unter einer "veränderten Kofaktor-Nutzung versteht man im Rahmen der vorliegenden Erfindung eine qualitative oder quantitative Veränderung im Vergleich zu einer Referenz. Insbesondere ist eine veränderte Kofaktor-Nutzung durch Vornahme von Aminosäuresequenzmutationen zu beobachten. Diese Veränderung ist dann im Vergleich zum nicht-mutierten Ausgangsenzym feststellbar. Dabei kann die Aktivität in Bezug auf einen bestimmten Kofaktor durch Vornahme einer Mutation erhöht oder erniedrigt werden oder vollständig unterbunden werden. Eine veränderte Kofaktor-Nutzung umfasst aber auch Änderungen dergestalt, dass anstelle einer Spezifität für einen einzelnen Kofaktor nunmehr wenigstens ein weiterer, vom ersten Kofaktor verschiedener, zweiter Kofaktor nutzbar ist (d.h. es liegt eine erweiterte Kofaktor-Nutzung vor) Umgekehrt kann aber auch eine ursprünglich vorhandene Befähigung zur Nutzung zweier verschiedener Kofaktoren so abgeändert werden, dass Spezifität nur für einen dieser Kofaktoren erhöht bzw. für einen dieser Kofaktoren verringert oder vollständig aufgehoben wird. So kann beispielsweise ein Enzym, das vom Kofaktor NAD (NADH) abhängig ist, aufgrund einer Veränderung der Kofaktor-Nutzung nunmehr sowohl von NAD (NADH) als auch vom Kofaktor NADP (NADPH) abhängig sein oder die ursprüngliche Abhängigkeit von NAD (NADH) kann vollständig in eine Abhängigkeit von NADP (NADPH) überführt werden und umgekehrt.

Die Begriffe "$NAD^+$/NADH-Abhängigkeit" bzw. "$NADP^+$/NADPH-Abhängigkeit" sind erfindungsgemäß, wenn nicht anders definiert, weit auszulegen. Diese Begriffe umfassen sowohl "spezifische" Abhängigkeiten, d.h. ausschließlich Abhängigkeit von $NAD^+$/NADH bzw. $NADP^+$/NADPH, als auch die Abhängigkeit der erfindungsgemäß verwendeten Enzyme von beiden Kofaktoren, d.h. Abhängigkeit von $NAD^+$/NADH und $NADP^+$/NADPH.

Entsprechendes gilt für die verwendeten Begriffe "$NAD^+$/NADH-akzeptierend" bzw. "$NADP^+$/NADPH-akzeptierend".

Die Begriffe "$NAD^+$/NADH-regenerierend" bzw. "$NADP^+$/NADPH-regenerierend" sind erfindungsgemäß, wenn nicht anders definiert, weit auszulegen. Diese Begriffe umfassen sowohl "spezifische" d.h. ausschließliche Befähigung verbrauchten Kofaktor $NAD^+$/NADH bzw. $NADP^+$/NADPH zu regenerieren, als auch die Befähigung beide Kofaktoren, d.h. $NAD^+$/NADH und $NADP^+$/NADPH, zu regenerieren.

"Proteinogene" Aminosäuren umfassen insbesondere (Einbuchstabencode): G, A, V, L, I, F, P, M, W, S, T, C, Y, N, Q, D, E, K, R und H.

Unter einer "Immobilisierung" versteht man erfindungsgemäß die kovalente oder nicht-kovalente Bindung eines erfindungsgemäß verwendeten Biokatalysators, wie z.B. einer 7ß-HSDH an einem festen, d.h. in dem umgebenden flüssigen Medium im Wesentlichen unlöslichen, Trägermaterial. Erfindungsgemäß können entsprechend auch ganze Zellen, wie die erfindungsgemäß verwendeten, rekombinanten Mikroorganismen, mit Hilfe derartiger Träger immobilisiert sein.

Eine "im Vergleich zum nicht-mutierten Enzym verringerte Substratinhibition" bedeutet, dass das die beim nicht-mutierten Enzym für ein bestimmtes Substrat beobachtete Substratinhibition nicht mehr zu beobachten ist, d.h. im Wesentlichen nicht mehr messbar ist, oder erst bei höherer Substratkonzentration einsetzt, d.h. der $K_i$-Wert erhöht ist.

Unter einer "Cholsäure-Verbindung" versteht man erfindungsgemäß Verbindungen mit dem Kohenstoffgrundgerüst, insbesondere der Steroidstruktur der Cholsäure und dem Vorhandensein von Keto- und/oder Hydroxy- oder Acyloxy-Gruppen in der Ringposition 7 und gegebenenfalls den Ringpositionen 3 und/oder 12.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das $NH_4^+$-Salz bzw. solche Ammoniumsalze umfasst, worin wenigstens ein Wasserstoffatom durch einen $C_1$-$C_6$-Alkylrest ersetzt sein kann. Typische Alkylreste sind insbesondere $C_1$-$C_4$-Alkylreste, wie Methyl, Ethyl, n- oder i-Propyl-, n-, sec- oder tert-Butyl, sowie n-Pentyl

und n-Hexyl und die ein- oder mehrfach verzweigten Analoga davon

"Alkylester" erfindungsgemäßer Verbindungen sind insbesondere Niedrigalkyl-Ester, wie z.B. $C_1$-$C_6$-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte erfindungsgemäßer Säuren mit Ammoniak oder primären oder sekundären Monoaminen. Derartige Amine sind beispielsweise Mono- oder Di-$C_1$-$C_6$-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

Erfindungsgemäße "Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und $C_1$-$C_6$-Alkylgruppen, wie z.B. Benzoyl oder Toluoyl.

Die erfindungsgemäß eingesetzten bzw. hergestellten Hydroxysteroidverbindungen, wie z.B. Cholsäure, Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

In folgender Tabelle sind die Strukturformeln, deren chemischen Namen und die verwendeten Abkürzungen wesentlicher chemischer Verbindungen tabellarisch zusammengefasst:

| Formel | Abkürzung | Chemischer Name |
|---|---|---|
| 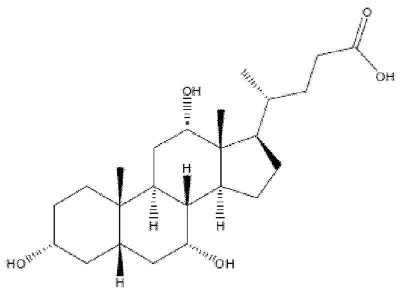 Cholsäure | CS | Cholsäure |
| 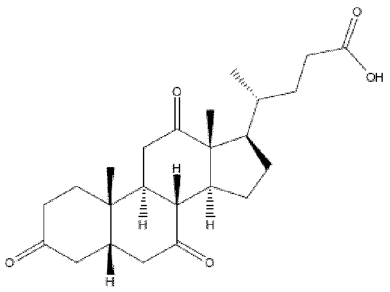 Dehydrocholsäure | DHCS | Dehydrocholsäure |
| 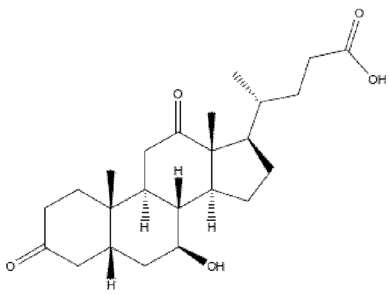 3,12-Diketo-7ß-Cholansäure | 3,12-Diketo-7β-CS | 3,12-Diketo-7β-Cholansäure |

(fortgesetzt)

| Formel | Abkürzung | Chemischer Name |
|---|---|---|
| 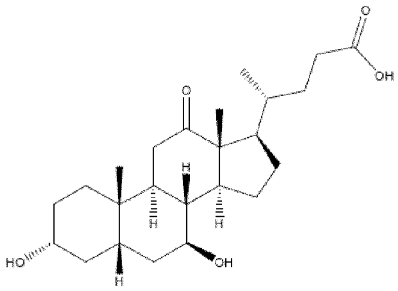<br>12keto-Ursodeoxycholsäure | 12Keto-UDCS | 12Keto-Ursodeoxycholsäure |
| 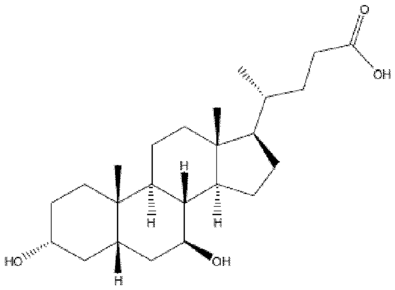<br>Ursodeoxycholsäure | UDCS | Ursodeoxycholsäure |
| 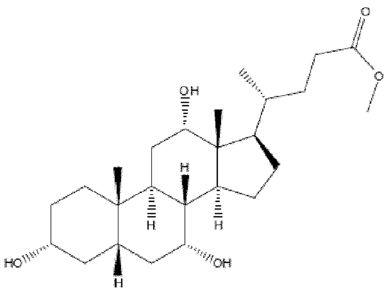<br>Cholsäure-methylester | CS-Methylester | Cholsäure-methylester |
| 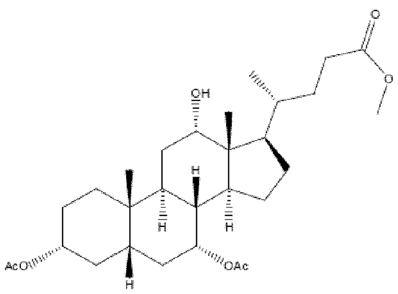<br>3,7-Diacetyl-Cholsäure-methylester | 3,7-Diacetyl-CS-methylester | 3,7-Diacetyl-Cholsäure-methylester* |

(fortgesetzt)

| Formel | Abkürzung | Chemischer Name |
|---|---|---|
| 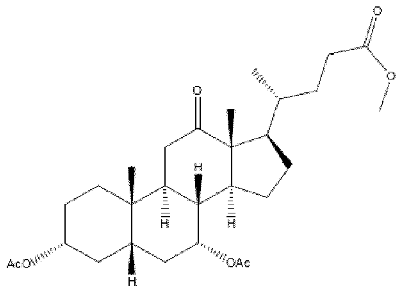 12-Keto-3,7-Diacetyl-Cholansäure-methylester | 12-Keto-3,7-Diacetyl-CS-methylester | 12-Keto-3,7-Diacetyl-Cholansäure-methylester* |
| 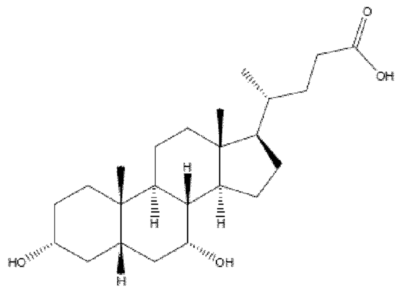 Chenodeoxycholsäure | CDCS | Chenodeoxycholsäure |
| 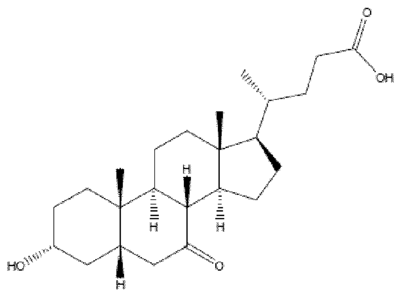 7-Keto-Lithocholsäure | 7-Keto-LCS | 7-Keto-Lithocholsäure |
| 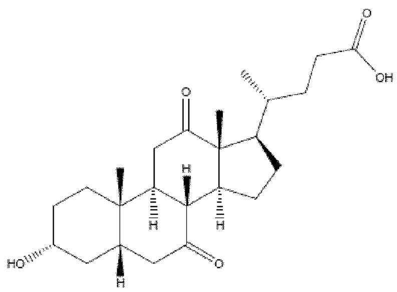 7,12-Diketo-Lithocholsäure | 7,12-Diketo-LCS | 7,12-Diketo-Lithocholsäure |

(fortgesetzt)

| Formel | Abkürzung | Chemischer Name |
|---|---|---|
| 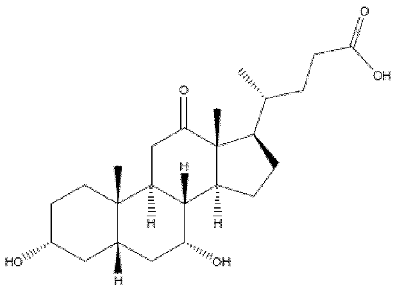 12-Keto-Chenodeoxycholsäure | 12-Keto-CDCS | 12-Keto-Chenodeoxycholsäure |

2. Proteine

Die vorliegende Erfindung ist nicht auf die konkret offenbarten Proteine bzw. Enzyme mit 7ß-HSDH-, FDH-, GDH- oder 3α-HSDH Aktivität bzw. deren Mutanten beschränkt, sondern erstreckt sich vielmehr auch auf funktionale Äquivalente davon.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. 7ß HSDH-Aktivität, besitzen.

So versteht man beispielsweise unter "funktionalen Äquivalenten" Enzyme, die im verwendeten Test auf 7ß-HSDH-, FDH-, GDH- oder 3α-HSDH -Aktivität eine um mindestens 1% , wie z.B. mindestens 10% oder 20 %, wie z.B. mindestens 50 % oder 75% oder 90 % höhere oder niedrigere Aktivität eines Ausgangs-Enzyms, umfassend eine hierin definierte Aminosäuresequenz aufweisen.

Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C.

Die 7ß-HSDH-Aktivität kann mit Hilfe verschiedener bekannter Tests nachgewiesen werden. Ohne darauf beschränkt zu sein, sei ein Test unter Verwendung eines Referenzsubstrates, wie z. B. CS oder DHCS, unter standardisierten Bedingungen wie im experimentellen Teil definiert, zu nennen.

Tests zur Bestimmung der FDH-, GDH- oder 3α-HSDH -Aktivität sind ebenfalls an sich bekannt.

Unter "funktionalen Äquivalenten" versteht man erfindungsgemäß insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere, wie z.B. 1, 2, 3, 4, 5, 6, 7, 8, 9, 10, 11, 12, 13, 14 oder 15, Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle zusammengefasst:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |

(fortgesetzt)

| Ursprünglicher Rest | Beispiele der Substitution |
|---|---|
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxylgruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Erfindungsgemäß mit umfasste "funktionale Äquivalente" sind Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren

eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Die Erfindung umfasst weiterhin die Anwendung des 7ß-HSDH-Wildtyps aus *Collinsella aerofaciens* ATCC 25986, wie er in der älteren internationalen Patentanmeldung PCT/EP2010/068576 der Anmelderin beschrieben ist, worauf hiermit ausdrücklich Bezug genommen wird.

Dieser aus *Collinsella aerofaciens* DSM 3979 erhältliche 7β-HSDH ist insbesondere durch wenigstens eine weitere der folgenden Eigenschaften, wie z.B. 2, 3, 4, 5, 6 oder 7 oder aller solcher Eigenschaften, gekennzeichnet:

a) Molekulargewicht (SDS-Gelelektrophorese): etwa 28-32 kDa, insbesondere etwa 29 bis 31 kDa oder etwa 30 kDa;

b) Molekulargewicht (Gelfiltration, unter nicht denaturierenden Bedingungen, wie insbesondere ohne SDS): etwa 53 bis 60 kDa, insbesondere etwa 55 bis 57 kDa , wie 56.1 kDa. Dies belegt die dimere Beschaffenheit der 7β-HSDH aus *Collinsella aerofaciens* DSM 3979;

c) Stereoselektiven Reduktion der 7-Carbonyl-Gruppe von 7-Keto-LCS in eine 7β-Hydroxy-Gruppe;

d) pH Optimum für die Oxidation von UDCS im Bereich von pH 8,5 bis 10,5, insbesondere 9 bis 10;

e) pH Optimum für die Reduktion von DHCS und 7-Keto-LCS im Bereich von pH 3,5 bis 6,5, insbesondere bei pH 4 bis 6;

f) wenigstens einen kinetischen Parameter aus folgender Tabelle für wenigstens eines der dort genannten Substrate/Kofaktoren; im Bereich von ±20%, insbesondere ±10%, ±5%, ±3% ±2% oder ±1% um den in der folgenden Tabelle jeweils konkret genannten Wert.

| | $K_M$ (μM) | $V_{max}$ (U/mg Protein)[b)] | $k_{cat}$ (1 μmol/(μmol×min)) |
|---|---|---|---|
| $NADP^+$ | 5.32 | 30.58 | 944.95 |
| NADPH | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| $NAD^+$ | -[a)] | - | Spuren |
| NADH | - | - | Spuren |
| [a)] konnten aufgrund der sehr geringen Aktivität nicht bestimmt werden<br>[b)] 1 U = 1 μmol/min | | | |

g) Phylogenetische Sequenzverwandtschaft der prokaryotischen 7β-HSDH aus *Collinsella aerofaciens* DSM 3979 mit der tierischen 11 β-HSDH-Untergruppe, umfassend Cavia *porcellus, Homo sapiens* und *Mus musulus,* verwandt.

Beispielsweise zeigt diese 7β-HSDH folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f).

Eine derartige 7ß-HSDH oder davon abgeleitetes funktionales Äquivalentist zudem gekennzeichnet durch

g) die stereospezifische Reduktion eines 7-Ketosteroids zum korrespondierenden 7β-Hydroxysteroid, und/oder
h) die regiospezifische Hydroxylierung eines Ketosteroids umfassend eine Ketogruppe in 7-Position und wenigstens eine weitere Ketogruppe am Steroidgerüst zum korrespondierenden 7ß-Hydroxysteroid, wie insbesondere von Dehydrocholsäure (DHCS) in 7-Position zur korrespondierenden 3,12-Diketo-7ß-Cholansäure, katalysiert, und z.B. NADPH abhängig ist.

Eine derartige 7ß-HSDH hat insbesondere eine Aminosäuresequenz gemäß SEQ ID NO:2 (Accession NO: ZP_01773061) oder eine davon abgeleiteten Sequenz mit einem Identitätsgrad von wenigstens 60%, wie z.B. wenigstens 65, 70, 75, 80, 85, oder 90, wie z.B. wenigstens 91, 92, 93, 94, 95, 96, 97, 98, 99 oder 99,5% zu dieser Sequenz; gegebenenfalls zusätzlich gekennzeichnet durch eine der folgende Eigenschaften oder Kombinationen von Eigenschaften; a); b); a) und b); a) und/oder b) und c); a) und/oder b) und c) und d); a) und/oder b) und c) und d) und e); a) und/oder b) und c) und d) und e) und f) gemäß obiger Definition.

3. Nukleinsäuren und Konstrukte

3. 1 Nukleinsäuren

Gegenstand der Erfindung sind auch Nukleinsäuresequenzen, die für ein Enzym mit 7ß-HSDH-, FDH-, GDH- und/oder 3α-HSDH Aktivität und deren Mutanten kodieren.
Die vorliegende Erfindung betrifft auch Nukleinsäuren mit einem bestimmten Identitätsgrad zu den hierin beschriebenen konkreten Sequenzen.
Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

| | |
|---|---|
| Pairwise alignment parameter: | |
| FAST algorithm | on |
| K-tuplesize | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |

(fortgesetzt)

| | |
|---|---|
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind auch Nukleinsäuresequenzen (einzel- und doppelsträngige DNA- und RNA-Sequenzen, wie z.B. cDNA und mRNA), kodierend für eines der obigen Polypeptide und deren funktionalen Äquivalente, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den erfindungsgemäßen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise

in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 × SSC (1 × SSC = 0,15 M NaCl, 15 mM Natriumcitrat, pH 7,2) oder zusätzlich in Gegenwart von 50% Formamid wie beispielsweise 42 °C in 5 × SSC, 50% Formamid zu verstehen. Vorteilhafterweise liegen die Hybridisierungsbedingungen für DNA:DNA-Hybride bei 0,1 × SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 × SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 × SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Gegenstand der Erfindung sind auch Derivate der konkret offenbarten oder ableitbaren Nukleinsäuresequenzen.

So können weitere erfindungsgemäße Nukleinsäuresequenzen z.B. von SEQ ID NO:1, 5, 7, 14, 19, 34 oder 47abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuresequenz mit der Sequenz SEQ ID NO:1, 5, 7, 14, 19, 34 oder 47sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der erfindungsgemäßen Nukleinsäuresequenzen, insbesondere der SEQ ID NO:1, 5, 7, 14, 19, 34 oder 47beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe zur der SEQ ID NO:1, 5, 7, 14, 19, 34 oder 47 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO:1, 5, 7, 14, 19, 34 oder 47 angegebenen DNA-Bereich.

Außerdem sind unter Derivaten beispielsweise Fusionen mit Promotoren zu verstehen. Die Promotoren, die

den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Protein sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcärel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique u-sing an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern wichtige Informationen in bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

3.2 Konstrukte

Gegenstand der Erfindung sind außerdem Expressionskonstrukte, enthaltend unter der genetischen Kontrolle regulativer Nukleinsäuresequenzen eine für wenigstens ein erfindungsgemäßes Polypeptid kodierende Nukleinsäuresequenz; sowie Vektoren, umfassend wenigstens eines dieser Expressionskonstrukte.

Unter einer "Expressionseinheit" wird erfindungsgemäß eine Nukleinsäure mit Expressionsaktivität verstanden, die einen Promotor, wie hierein definiert umfasst, und nach funktioneller Verknüpfung mit einer zu exprimierenden

Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäße Nukleinsäurekonstrukte umfassen insbesondere Sequenz SEQ ID NO:1, 5, 7, 14, 19, 34 oder 47oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor,

wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III$^{113}$-B1, λgt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHlac$^{+}$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

4. Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben. Einen Überblick über bakterielle Expressionssysteme für die heterologe Expression von Proteinen liefertz.B. auch Terpe, K. Appl. Microbiol. Biotechnol. (2006) 72: 211-222.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gramnegative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei vorzugsweise mindestens eine der in dieser Erfindung beschriebenen Nukleinsäuresequenzen, Nukleinsäurekonstrukte oder Vektoren, die für ein

Enzym mit 7ß-HSDH-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

5. Herstellung der UDCS

*1. Schritt: Chemische Umsetzung von* CS *zu DHCS*

Die Hydroxygruppen von CS werden mit Chromsäure bzw. Chromaten in saurer Lösung (z.B. $H_2SO_4$) zu Carbonylgruppe in an sich bekannter Weise auf klassischchemischem Weg oxidiert. Dadurch entsteht DHCS.

*2. Schritt: Enzymatische oder mikrobielle Umsetzung von DHCS zu 12-Keto-UDCS*

In wässriger Lösung wird DHCS durch 3$\alpha$-HSDH und 7$\beta$-HSDH bzw. Mutanten davon spezifisch zu 12-Keto-UDCS in Anwesenheit von NADPH bzw. NADH reduziert. Der Kofaktor NADPH bzw. NADH kann durch eine ADH bzw. FDH bzw. GDH bzw. Mutanten davon von Isopropanol bzw. Natium-formiat bzw. Glucose regeneriert werden. Die Reaktion läuft unter milder Bedingung. Beispielsweise kann die Reaktion bei pH = 6 bis 9, insbesondere etwa pH = 8 und bei etwa 10 bis 30, 15 bis 25 oder etwa 23°C durchgeführt werden. Im falle eines mikrobiellen Umsetzungsschrittes können rekombinante Mikroorganismen, welche die erforderliche(n) Enzymaktivität(en) exprimieren in Gegenwart des umzusetzenden Substrates (DHCS) anaerob oder aerob in geeigneten Flüssigmedien kultiviert werden. Geeignete Kultivierungsbedingungen sind dem Fachmann an sich bekannt. Sie umfassen Umsetzungen im pH bereich von beispielsweise 5 bis 10 oder 6 bis 9, bei Temperaturen im Bereich von 10 bis 60 oder 15 bis 45 oder 25 bis 40 oder 37 °C. Geeignete Medien umfassen z,B, die unten beschriebenen LB und TB Medien. Die Umsetzungsdauer kann dabei z,B, batchweise oder kontinuierlich oder in sonstigen üblichen Verfahrensvarianten erfolgen (wie oben beschrieben). Die Unsetzungsdauer kann dabei z.B. im Bereich von Minuten bis mehreren Stunden oder tagen Liegen, und z.B. 1h bis 48 h betragen. Gegebenenfalls kann, wenn Enzymaktivität nicht kontinuierlich exprimiert wird, diese durch Zugabe eines geeigneten Induktors, nach Erreichen einer Zielzelldichte , z,B, von etwa $OD_{600}$ = 0,5 bis 1,0, eingeleitet werden.

Weitere mögliche geeignete Abwandlungen des mikrobiellen Herstellungsverfahrens hinsichtlich Fahrweise der Fermentation, Zusätze zum Medium, Enzymimmobilisierung und Isolierung der Wertstoffe sind auch dem folgenden Abschnitt betreffend "Herstellung der Enzyme bzw. Mutanten" zu entnehmen

*3. Schritt Chemische Umsetzung von 12-Keto-UDCS zu UDCS*

Die 12-Carbonylgruppe von 12-Keto-UDCS wird mittels Wolff-Kishner-Reduktion in an sich bekannter Weise entfernt, dadurch entsteht UDCS aus 12-Keto-UDCS. In der Reaktion wird zuerst die Carbonylgruppe mit Hydrazin zum Hydrazon umgesetzt. Anschließend wird das Hydrazon in Gegenwart einer Base (z.B. KOH) auf 200 °C erhitzt, hierbei wird Stickstoff abgespaltet und UDCS entsteht.

6. Rekombinante Herstellung der Enzyme und Mutanten

Gegenstand der Erfindung sind weiterhin Verfahren zur rekombinanten Herstellung erfindungsgemäßer Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die erfindungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

7. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

**Experimenteller Teil:**

Werden keine anderen Angaben gemacht, so können die im Rahmen der vorliegenden Erfindung durchgeführten Klonierungsschritte, wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von Mikroorganismen, Anzucht von Mikroorganismen, Vermehrung von Phagen und Sequenzanalyse rekombinanter DNA wie bei Sambrook et al. (1989) a.a.O. beschrieben durchgeführt werden.

**A. Allgemeine Angaben**

**Materialien:**

Die genomische DNA von *Collinsella aerofaciens* DSM 3979 (ATCC 25986, frühere Bezeichnung *Eubacterium aerofaciens*) wurde von der Deutschen Sammlung für Mikroorganismen und Zellkulturen (DSMZ) bezogen. UDCS und 7-Keto-LCS sind an sich bekannte und in der Literatur beschriebene Ausgangsverbindungen. Alle übrigen Chemikalien wurden von Sigma-Aldrich und Fluka (Deutschland) bezogen. Sämtliche Restriktionsendonukleasen, die T4 DNA Ligase, die *Taq* DNA Polymerase, die *Phusion* DNA Polymerase und Isopropyl-β-D-1-thiogalactopyranosid (IPTG) wurden von Fermentas (Deutschland) bezogen.

**Medien:**

LB-Medium, enthaltend Trypton 10 g, Hefeextrakt 5 g, NaCl 10 g pro Liter Medium
TB-Medium, enthaltend Trypton 12 g, Hefeextrakt 24 g, 4 mL Glycerin, 10 % TB.-Puffer (11,55 g $KH_2PO_4$, 62,7 g $K_2HPO_4$, ad 500 mL $H_2O$) pro Liter Medium
Minimalmedium, modifiziert nach Wilms et al., BIOTECHNOLOGY AND BIOENGI-NEERING, 2001 VOL. 73, NO. 2, 95-103

**Expressionsvektoren und Vektorkonstrukte**

Zur Expression rekombinanter Proteine wurden folgende an sich bekannte Expressionsvektoren verwendet:

**pET21a(+),** (vgl. Fig. 3a)
**pET22b(+)** (vgl. Fig. 3b)
**pET28a(+)** (vgl. Fig. 3d)und
**pCOLADuet-1**
(jeweils Novagen, Madison, Wisconsin, USA).

Die Vektoren **pET21a(+)** und **pET22b(+)** besitzen jeweils eine *Multiple cloning site* (MCS), die jeweils unter der Kontrolle eines T7-Promotors mit nachgeschaltetem *lac*-Operator und der darauf folgenden Ribosomalen Bindestelle (rbs) stehen. Im C-terminalen Bereich der Expressionsregion befindet sich jeweils ein T7-Terminator. Beide Plasmide verfügen über ein ColE1-Replicon (pBR322-Replicon), ein Ampicillin-Resistenzgen (*bla*), einen f1-Ursprung und ein Gen, welches den *lac*-Inhibitor codiert (*lac*I).

Zusätzlich verfügt das pET21a(+)-Plasmid über einen T7·Tag im *N*-terminalen Bereich der MCS und einen optionalen His·Tag *C*-terminal der MCS. Das pET22b(+)-Plasmid verfügt im *N*-terminalen Bereich der MCS über eine pelB-Signalsequenz und einen His·Tag *C*-terminal von der MCS.

Der **pCOLADuet-1-**Vektor verfügt über zwei MCS, die jeweils unter der Kontrolle eines T7-Promotors mit nachgeschaltetem *lac*-Operator und nachfolgender Ribosomalen Bindestelle (rbs) stehen. *C*-terminal der beiden MCS befindet sich ein T7-Terminator. Des Weiteren verfügt dieser Vektor über ein Gen, welches den *lac*-Inhibitor codiert sowie über ein COLA-Replicon (ColA-Replicon).

In der vorliegenden Arbeit wurde eine modifizierte Variante des kommerziell erhältlichen pCOLADuet-1-Vektors verwendet. Bei dieser modifizierten Plasmidvariante (bezeichnet als **pCOLA(mod); vgl. Fig. 3c))** wurde das Kanamycin-Resistenzgen des ursprünglichen Vektors durch ein Chloramphenicol-Resistenzgen ersetzt. Zudem wurde aus dem Chloramphenicol-Resistenzgen eine *Nco*I-Restriktionsschnittstelle mittels positionsgerichtete Punktmutagenese entfernt. Im *N*-terminalen Bereich der ersten MCS befindet sich ein His·Tag, während sich *C*-terminal der zweiten MCS ein S·Tag befindet.

Die ColE1-Replicons der pET-Plasmide und das COLA-Replicon des pCOLA-Plasmids sind miteinander kompatibel. Das ermöglicht das gleichzeitige stabile Einbringen eines pET-Plasmids und eines pCOLA-Plasmids in *Escherichia coli.* Somit können Kombinationen verschiedener Gene in *Escherichia coli* einkloniert werden, ohne dass diese auf demselben Operon platziert sind. Bedingt durch unterschiedliche Kopienzahlen von pET-Vektoren (~40) und pCOLA-Vektoren (20-40) lassen sich zudem die Expressionslevel kotransformierter Gene beeinflussen.

Folgende Vektorkonstrukte wurden verwendet

**pET22b(+) 7β-HSDH:** ein pET22b(+)-Vektor in das die 7β-HSDH aus *Collinsella aerofaciens* ATCC 25986 über die Schnittstellen *Nde* I und *Hind* III in üblicher Weise einkloniert wurde.

**pET22b(+) 3α-HSDH:** ein pET22b(+)-Vektor in das die 3α-HSDH aus *Comamonas testosteroni* über die Schnittstellen *Nde* I und EcoR I in üblicher Weise einkloniert wurde (Oppermann et al.,J Biochem, 1996, 241(3):744-749).

**pET21a(+) FDH D221G** (vgl. Fig. 4a): ein pET21a(+)-Vektor in das die Formiatdehydrogenase aus *Mycobacterium vaccae* N10 über die Schnittstellen *Nde* I und EcoR I einkloniert wurde. Durch ortsgerichtete Mutagenese wurde der Aspartatrest (D) an Position 221 (ohne Berücksichtigung von Methionin in Position 1) bzw. Position 222 (gerechnet ab Metionin in Position 1; vgl SEQ ID NO: 15, 19, 35) der Formiatdehydrogenase durch einen Glycinrest ersetzt (vgl. Herstellungsbeispiel 4, unten) Die Formiatdehydrogenase trägt an der Position 1202 der Nukleotidsequenz eine einzelne Basendeletion, was zum Austausch der letzten Aminosäure Valin gegen ein Alanin führt. Gleichzeitig führt diese Basendeletion zum Ausschalten des Stoppcodons und zur Aktivierung des ursprünglich

außerhalb des Leserahmens liegenden His·Tags (vgl. SEQ ID NO: 34 und 35).

**pET21a(+) 7β-HSDH:** ein pET21a(+)-Vektor, in den die 7β-HSDH aus *Collinsella aerofaciens* ATCC 25986 über die Schnittstellen *Nde* I und *Xho* I in üblicher Weise einkloniert wurde

**pET21a(+) FDH D221G 7β-HSDH** (vgl. Fig. 4b)

**pET21a(+) FDH 7β-HSDH(G39A) 3α-HSDH** (vgl. Fig. 8)

**pCOLA(mod) 3α-HSDH** (vgl. Fig. 5)

## Mikroorganismen

| Stamm | Genotyp |
|---|---|
| Escherichia coli DH5α | $F^-$ endA1 glnV44 thi-1 recA1 relA1 gyrA96 deoR nupG Φ80dlacZΔM15 Δ(lacZYA-argF)U169, hsdR17($r_K{}^-$ $m_K$+),λ- |
| *Escherichia coli* BL21 (DE3) | $F^-$ ompT gal dem lon $hsdS_B(r_B{}^- m_B{}^-)$ λ(DE3 [lacl lacUV5-T7 gene 1 ind1 sam7 nin5]) |
| Escherichia coli BL21 (DE3) $hdhA^-$ $KanR^+$ (7α-HSDH Knock-out Stamm) (vgl. Herstellungsbeispiel 5) | $F^-$ ompT gal dem lon $hsdS_B(r_B{}^- m_B{}^-)$ λ(DE3 [lacl lacUV5-T7 gene 1 ind1 sam7 nin5]) $hdhA^-$ $KanR^+$ |

Der Escherichia. coli Stamm DH5α (Novagen, Madison, Wisconsin, USA) wurde bei 37°C in LB-Medium, enthaltend geeignete Antibiotika vermehrt.

Der *Escherichia coli* Stamm BL21(DE3) (Novagen, Madison, Wisconsin, USA) wurde bei 37°C in LB-Medium, enthaltend geeignete Antibiotika, vermehrt und wurde nach Induktion bei $OD_{600}$ = 0.8 mit 0.5 mM IPTG bei 25°C und 140 Upm gehalten.

## Methoden

1. Standardbedingungen für 7ß-HSDH Aktivitätsbestimmung

Die Reaktionsmischung enthält ein Gesamtvolumen von 1 ml:

| | |
|---|---|
| 880 μl | 50 mM Kaliumphosphat Puffer, pH 8.0 |
| 10 μl | 10 mM UDCS (gelöst in Wasser, pH 8) |
| 10 μl | Enzymlösung (in Puffer wie oben, im Bereich von 1 zu 10 U/ml) |
| 100 μl | 1 mM NADP+ (in Puffer wie oben) |

Die Zunahme der Extinktion bei 340 nm wird gemessen und die Aktivität wird als Enzym-Unit (U, i.e. μmol/min) unter Verwendung des molaren Extinktionskoeffizienten von 6.22 $mM^{-1} \times cm^{-1}$, berechnet.

Standardbedingungen für 7ß-HSDH Aktivitätsbestimmung gemäß Herstellungsbeispiel 7

Die Reaktionsmischung enthält ein Gesamtvolumen von 1 ml

| | |
|---|---|
| 870 μl | 50 mM Kaliumphosphat (KPi) Puffer, pH 8.0 |
| 100 μl | 100 mM DHCA (gelöst in 50 mM KPi, pH 8) |
| 10 μl | Enzymlösung (in Puffer wie oben, im Bereich von 2 zu 6 U/ml) |
| 20 μl | 12,5 mM NADPH (gelöst in $ddH_2O$) |

Die Zunahme der Extinktion bei 340 nm wird gemessen und die Aktivität wird als Enzym-Unit (U, i.e. μmol/min) unter Verwendung des molaren Extinktionskoeffizienten von 6.22 $mM^{-1} \times cm^{-1}$, berechnet

2. Proteinbestimmung mittels BCA-Assay

Die Proben wurden mit BCA-Reagenz (von Interchim) gemischt und bei 37°C für 45 min inkubiert. Der Proteingehalt wurde bei 562 nm gegen einen Kalibrationskurve (BSA) im Konzentrationsbereich des verwendeten Assay bestimmt.

3. Dünnschichtchromatographie

5 bis 10 μg Probe wurden auf eine DC-Folie Kieselgel 60 (Merck) aufgetragen. Authentische Substanzen wurden als Referenz aufgetragen. Ein Ende der DC-Folie wurde in Laufmittel eingetaucht bis die Mobilphase oben erreicht. Die DC-Folie wurde getrocknet und mittels Molybdatophosphorsäure entwickelt.

4. Molekularbiologische Arbeitsmethoden:

Molekularbiologische Arbeiten erfolgen, soweit keine anderen Angaben gemacht werden, in Anlehnung an etablierte Methoden, z.B. beschrieben in:
Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989; Ausubel et al. (eds.), Current Protocols in Molecular Biology, John Wiley &Sons, NY (1993); Kriegler, Gene Transfer and Expression, A Laboratory Manual, Stockton Press, NY (1990).

## B. Beispiele

### Herstellungsbeispiel 1: Identifizierung der 7β-HSDH Aktivität

Die genomische DNA-Sequenz von *Collinsella aerofaciens* ATCC 25986 wurde im Jahr 2007 von "Washington University Genome Sequencing Center" für das "human gut microbiome project" bei GenBank veröffentlicht. HSDHs gehören zu den "Short-Chain Dehydrogenasen". Da die biochemische Funktion der "Short-Chain Dehydrogenasen" aus *Collinsella aerofaciens* ATCC 25986 nicht in GenBank annotiert wurde, wurden 9 Kandidaten in Vektor pET22b+ einkloniert, und anschließend in *E. coli* BL21(DE3) exprimiert.

Dazu wurden 7ß-HSDH kodierende Sequenzen PCR-amplifiziert. Die PCR-Produkte erhielt man unter Verwendung der genomischen DNA von *Collinsella aerofaciens* ATCC 25986 (DSM 3979) als Template und den Primer 5'-gggaattcCATATGAACCTGAGGGAGAAGTA-3' (SEQ ID NO:3) und 5'- cccAAGCTT-CTAGTCGCGGTAGAACGA-3' (SEQ ID NO:4). Die *Nde*I und *Hin*dIII Schnittstellen in den Primer-Sequenzen sind unterstrichen. Das PCR-Produkt wurde mittels PCR-Purification-Kit (Qiagen) aufgereinigt und dann mit den Enzymen *Nde*I und *Hin*dIII geschnitten. Der entsprechende Vektor wurde auch mit den *Nde*I und *Hin*dIII geschnitten. Die Produkte wurden auf ein Agarose-Gel aufgetragen, aufgetrennt, aus diesem ausgeschnitten und aufgereinigt. Das geschnittene PCR-Produkt und der geschnittene Vektor wurden mittels T4-Ligase ligiert. Das Ligant wurde in *E. coli* DH5α transformiert. Der entstehende Vektor (enthält das Gen von 7β-HSDH) wurde durch Sequenzierung bestätigt und in *E. coli* BL21(DE3) transformiert und mit IPTG induziert und exprimiert.

Die Expression wurde in 50 ml LB-Medium durchgeführt. Zur Vorbereitung von Vorkultur wurde eine Kolonie auf LB-Agar-Platte in 5 ml LB-Medium (enthält entsprechende Antibiotika) gepickt und bei 37°C und 160 Upm über Nacht inkubiert. Das 50 ml LB-Medium (enthält entsprechende Antibiotika) wurde mit 500 μl Vorkultur angeimpft. Die Kultur wurde bei 37°C und 160 Upm inkubiert. Bis OD600 ca. 0,8 wurde die Expression durch Zugabe von 0,5 mM IPTG induziert. Nach 6 h oder über Nacht wurden die Zellen abzentrifugiert. Die Pellets wurden in 6 ml Kaliumphosphat-Puffer (50 mM, pH 8, enthält 0,1 mM PMSF) resuspendiert und mittels Ultraschall aufgeschlossen. Die Zelltrümmer wurden durch Zentrifugation entfernt.

Zur Identifizierung 7β-HSDH-Aktivität wurde die Aktivität mittels photometrischer Methode untersucht. In einer 1 ml Küvette wurde hierzu Enzym und 0,1 mM Testsubstanz (UDCS) in Kaliumphosphat Puffer (50 mM, pH8) gemischt. Nach Zugabe von NADPH(NADH) bzw. $NADP^+$($NAD^+$) wurde die Abbau oder Bildung von NAD(P)H gemessen. Ein Enzym von den 9 Kandidaten zeigt Aktivität (60 U/ml) gegen UDCS in Anwesenheit von $NADP^+$, aber keine Aktivität gegen CS. Die NADPH-abhängige 7β-HSDH-Aktivität von diesem Enzym wurde identifiziert.

In der photometrischen Untersuchung zeigte die 7β-HSDH die Aktivität 60 U/ml gegen UDCS, Aktivität 35 U/ml gegen 7-Keto-LCS und Aktivität 119 U/ml gegen DHCS in Anwesenheit von $NADP^+$ bzw. NADPH. Die Aktivität gegen CS ist nicht nachweisbar.

Das Gen, das die 7β-HSDH codiert, wurde in pET28a+ mit His-Tag umkloniert, um eine schnelle Reinigung zu ermöglichen. Diese 7β-HSDH mit His-Tag wurde aktiv in *E. coli* BL21(DE3) exprimiert wie oben beschrieben wurde. Die Aufreinigung wurde mit einer Talon-Säule durchgeführt. Die Säule wurde zuerst mit Kaliumphosphat-Puffer (50 mM, pH 8, mit 300 mM NaCl) equilibriert. Nachdem das Zelllysat geladen worden ist, wurde die Säule mit Kaliumphosphat-Puffer (50 mM, pH 8, mit 300 mM NaCl) gewaschen. Die 7β-HSDH wurde mit Kaliumphosphat-Puffer (50 mM, pH 8, mit 300 mM NaCl und 200 mM Imidazol) eluiert. Das Imidazol im Eluat wurde mittels Dialyse entfernt. Die Ausbeute der Aufreinigung betrug 76% mit Reinheit ca. 90%.

**Herstellungsbeispiel 2: Klonierung, Expression und Reinigung 7ß-HSDH aus *Collinsella aerofaciens* ATCC 25986 in präparativem Maßstab sowie weitere Charakterisierung des Enzyms**

**2.1 Klonierung und Herstellung eines Expressionskontruktes**

Das für 7β-HSDH kodierende Gen wurde wiederum aus der genomischen DNA durch PCR und unter Verwendung von Primern, wie oben für Herstellungsbeispiel 1 beschrieben, amplifiziert:
Das PCR-Produkt wurde wiederum wie oben beschrieben gereinigt und mit den Restriktionsendonukleasen NdeI und HindIII verdaut. Das verdaute PCR-Produkt wurde wiederum gereinigt und in den pET-28a(+)-Vektor unter Verwendung der T4-Ligase kloniert, um einen Expressionsvektor zu erzeugen. Das resultierende Expressionskonstrukt wurde anschließend in *E. coli* DH5α-Zellen transformiert. Das zu erwartende Protein sollte 20 Aminosäurereste umfassendes ein Signalpeptid und einen N-terminalen 6xHis-Tag sowie eine Thrombin-Schnittstelle aufweisen. Die Sequenz der insertierten DNA wurde durch Sequenzierung überprüft.

**2.2 Überexpression und Reinigung von 7β-HSDH**

*E. coli* BL21(DE3) wurde mit dem Expressionskonstrukt transformiert. Dazu wurde der das Expressionskonstrukt enthaltende *E. coli* BL21(DE3)-Stamm in LB-Medium (2 × 400 ml in 2 Liter-Schüttelflaschen) enthaltend 30 μg/ml Kanamycin vermehrt. Die Zellen wurden durch Zentrifugation (10,000×g, 15 min, 4°C) geerntet. Das Pellet wurde in 20 ml Phosphatpuffer (50 mM, pH 8, enthaltend 0.1 mM PMSF) resuspendiert. Die Zellen wurden unter konstanter Kühlung durch einminütige Ultraschallbehandlung (40 W Leistung, 40% Arbeitsintervall und 1 min Pause) unter Verwendung eines Ultraschallgeräts Sonifier 250 (Branson, Deutschland) aufgeschlossen. Der Aufschluss wurde dreimal wiederholt. Das Zellextrakt wurde zentrifugiert (22,000×g, 20 min, 4°C). Der Überstand wurde auf eine Talon-Säule (Clontech, USA), äquilibriert mit Beladungspuffer (50 mM Kaliumphosphat, 300 mM NaCl, pH 8) beladen. Das Verfahren wurde bei 24°C durchgeführt. Nicht gebundenes Material wurde durch Waschen der Säule mit Beladungspuffer (3 Säulenvolumina) ausgewaschen. Schwach bindendes Protein wurde durch Waschen mit Waschpuffer (20 mM Imidazol im Beladungspuffer; 3 Säulenvolumina) entfernt. Das His-Tag-7β-HSDH-Protein wurde mit Elutionspuffer (200 mM Imidazol im Beladungspuffer) eluiert. Das Eluat wurde in einem Dialyseschlauch mit einer molekularen Ausschlussgrenze von 5 kDa (Sigma, USA) in 2 Liter Kaliumphosphatpuffer (50 mM, pH 8) bei 4°C über Nacht dialysiert. Schließlich wurde die Probe in einen neuen Schlauch überführt und bei -20°C zur weiteren Analyse gelagert. Die Proteinkonzentration wurde unter Verwendung eines BCA Testkits (Thermo, USA) nach Herstellerangaben bestimmt. Außerdem wurde die Probe durch 12.5%ige SDS-PAGE und Färben mit Coomassie Brilliant Blue analysiert. Die Reinheit des Proteins wurde densitometrisch mit Hilfe von Scion Image Beta 4.0.2 (Scion, USA) bestimmt.

**2.3 Gelfiltration**

Gelfiltration wurde auf einem Pharmacia ÄKTA-Protein-Reinigungssystem durchgeführt, um das Molekulargewicht von 7β-HSDH zu bestimmen. Das gereinigte Enzym wurde auf eine Sephadex G-200-Säule aufgetragen, welche zuvor mit 50 mM Tris-HCl (pH 8), enthaltend 200 mM Natriumchlorid, äquilibriert worden war. Das Protein wurde mit dem gleichen Puffer bei einer Flussrate von 1 ml/min eluiert. Das Molekulargewicht von 7β-HSDH wurde durch Vergleich seines Elutionsvolumens mit demjenigen von Proteinstandards (Serum Albumin (66 kDa), α-Amylase aus *Aspergillus oryzae* (52 kDa), Trypsin aus Schweinepankreas (24 kDa) und Lysozym aus Hühnerei (14.4 kDa)) bestimmt.

**2.4 Enzymtest und kinetische Analyse**

Das Reaktionsgemisch für den Enzymtest enthielt in einem Gesamtvolumen von 1 ml 50 μmol Kaliumphosphat (pH 8), 0.1 μmol NAD(P)H oder $NAD(P)^+$, Substrate und Protein. Das Reaktionsgemisch war in Küvetten mit einer Lichtpfadlänge von 1 cm enthalten. Die 7β-HSDH-Aktivität wurde durch Aufzeichnung der Veränderung in der NAD(P)H-Konzentration über die Extinktion bei 340 nm mit Hilfe eines Spektrophotometers (Ultraspec 3000, Pharmacia Biotech, Großbritannien) bestimmt. Die Enzymaktivitäten wurden als Enzymeinheiten (U, d.h. μmol/min) unter Verwendung des

molaren Extinktionskoeffizienten von 6,22 $mM^{-1} \times cm^{-1}$ bei 25°C bestimmt. Mehrere unterschiedliche Messungen mit den Variablen Substrat, Koenzym, Konzentration, pH-Wert, Puffer und Inkubationstemperatur wurden durchgeführt. Die kinetischen Konstanten wurden unter Verwendung von Standardmethoden bestimmt.

**2.5 Biotransformation von 7-keto-Lithocholsäure durch die 7β-HSDH**

Die Umwandlung von 7-Keto-LCS durch 7β-HSDH wurde durchgeführt, um die biochemische Funktion von 7β-HSDH zu verifizieren. 0.4 g 7-Keto-LCS wurden in 10 ml Kaliumphosphatpuffer (50 mM, pH 8) suspendiert und der pH-Wert wurde auf pH 8 durch Zugabe von 2 M Natriumhydroxid eingestellt. 0.2 ml Isopropanol, 100 U 7β-HSDH und 80 U Alkoholdehydrogenase (ADH-TE) aus *Thermoanaerobacter ethanolicus* (freundliche Zuwendung von Frau Dr. K. Momoi, ITB Universität, Stuttgart) und 1 μmol $NADP^+$wurden zugesetzt. Der gleiche Puffer wurde zugesetzt, um ein gesamtes Reaktionsvolumen von 20 ml zu erhalten. Das Reaktionsgemisch wurde bei 24°C inkubiert und 24 Stunden gerührt. Dabei wurde NADPH mit Hilfe der ADH über die Oxidation von 2-Propanol regeneriert. Das Produkt wurde mit 1 ml 2 M Salzsäure angesäuert und 5 × mit 5 ml Ethylacetat extrahiert. Die organische Lösung wurde anschließend destilliert.

**2.6 Chromatographische Produktbestimmung**

Eine HPLC-Analyse wurde auf einer Säule vom Typ Purospher® STAR RP-18 (Hitbar® RT 125-4 Pre-Packed Column, Purospher® STAR RP-18 endcapped, Merck, Deutschland), versehen mit einer Vorsäule des Typs LiChroCART® STAR RP18 (endcapped, Merck, Deutschland) auf einem HPLC-System LC20AD (Shimadzu, Japan) bei einer Flussrate von 1 ml/min durchgeführt. Die mobile Phase bestand aus zwei Eluenten. Eluent A enthielt Acetonitril und Eluent B destilliertes Wasser (pH 2.6, eingestellt mit Orthophosphorsäure, 85%). Folgender Gradient wurde verwendet: A 35% (8 min) - 35%-43% (1% $min^{-1}$) - 43%-70% (1% $min^{-1}$) - 70% (5 min) - 70%-35% (17.5% $min^{-1}$) - 35% (5 min); eluent A 65% (8 min) - 65%-57% (1% $min^{-1}$) - 57%-30% (1% $min^{-1}$) - 30% (5 min) - 30%-65% (17.5% $min^{-1}$) - 65% (5 min). 20 μl Probe (1 mg/ml) wurden analysiert. Authentische UDCS, 7-Keto-LCS und CDCS wurden bei gleicher Konzentration als Standards verwendet. Die Aufzeichnung erfolgte durch UV-Detektion bei 200 nm.

**2.7 Sequenzalignment und phylogenetische Analyse**

Multiple Sequenz-Alignments wurden unter Verwendung der Clustal X-Software (Thompson et al., 1997, Nucleic Acid Research 25: 4876-82) erzeugt und modifiziert unter Verwendung der Jalview-Software (Clamp et al., 2004, Bioinformatics 20:426-7). Der phylogenetische Stammbaum wurde unter Verwendung des Programms TreeView 1.6.6 (Roderic 2001, http://taxonomy.zoology.gla.ac.uk/rod/rod.html) erzeugt.

**2.8 Versuchsergebnisse:**

**2.8.1 Identifizierung der 7β-HSDH-Aktivität in einer präparativen Biotransformation**

Um die Funktion des Enzyms zu bestätigen, wurde eine Biotransformation von 7-Keto-LCS im 10 ml-Maßstab durchgeführt, wobei das isolierte Enzym in Kombination mit ADH zur Regeneration von NADPH unter Verwendung von 2-Propanol, wie bereits beschrieben, eingesetzt wurde. Die HPLC-Analyse ergab, dass UDCS das einzige von dem Enzym produzierte Reaktionsprodukt (90% Umsatz) war. CDCS (Retentionszeit 19,4 min) wurde im Reaktionsgemisch nicht nachgewiesen. Das Ergebnis zeigt, dass das Enzym eine NADPH-abhängige 7β-HSDH darstellt und zur selektiven Reduktion der 7-Carbonyl-Gruppe von 7-Keto-LCS in eine 7β-Hydroxy-Gruppe befähigt ist.

Retentionszeit UDCS: 15,5 min
Retentionszeit 7-keto-LCS: 18,3 min

**2.8.2. Reinigung und Gelfiltration**

Nach Klonierung des 7β-HSDH-Gens aus *Collinsella aerofaciens* DSM 3979 in den Expressionsvektor pET28a(+) und anschließende Überexpression erhielt man ein am N-terminus mit einem His-Tag versehenes Fusionsprotein mit einer 7β-HSDH-Ausbeute von 332.5 mg (5828 U) pro Literkultur. Das mit dem His-Tag versehene 7β-HSDH wurde in einer Stufe mit Hilfe einer Immobilisierte-Metallionen-Affinitätschromatographie gereinigt (Reinheit >90%, Ausbeute 76%, vgl. Fig. 2.). Die Hauptbanden der Bahnen 1 und 2 repräsentieren das erwartete Expressionsprodukt bei 30 kDa, welches dem aus der Aminosäuresequenz des Gens abgeleiteten vorhergesagten Molekulargewicht entspricht. Durch Gelfiltration ermittelt man jedoch für die 7β-HSDH ein Molekulargewicht von 56.1 kDa. Dies belegt die dimere

Beschaffenheit der 7β-HSDH aus *Collinsella aerofaciens* DSM 3979.

**2.8.3. Sequenzalignments**

Die Aminosäuresequenz der erfindungsgemäß verwendeten 7β-HSDH wurde mit bekannten HSDH-Sequenzen verglichen (Alignment nicht gezeigt). Die beobachtete Sequenzähnlichkeit weist darauf hin, dass das erfindungsgemäße Enzym zur Familie der kurzkettigen Dehydrogenasen (SDR) gehört. Es ist bekannt, dass SDRs sehr niedrige Homologie und Sequenzidentität aufweisen (Jornvall, H., B. Persson, M. Krook, S. Atrian, R. Gonzalez-Duarte, J. Jeffery, and D. Ghosh. 1995. Short-chain dehydrogenases/reductases (SDR). Biochemistry 34:6003-13 und Persson, B., M. Krook, and H. Jornvall. 1991. Characteristics of short-chain alcohol dehydrogenases and related enzymes. Eur J Biochem 200:537-43). Das Sequenzalignment ergibt jedoch deutlich die konservierten Domänen in der SDR Primärstruktur. Das N-terminale Motiv Gly-X-X-X-Gly-X-Gly (entsprechend Gly-41, Gly-45 and Gly-47, Nummerierung entsprechend dem Alignment) entspricht dem charakteristischen Dinukleotid-Bindungsmotiv der SDR-Superfamilie. Darüber hinaus sind drei stark konservierte Reste Ser-177, Tri-190 und Lys-194 zu beobachten, die der katalytischen Triade der SDR-Enzyme entsprechen.

**2.8.4. Phylogenetische Analyse**

7α-HSDH aus *Clostridium sordellii, Brucella melitensis* und *Escherichia coli* gehören zur gleichen Untergruppe. Die beiden 3α-HSDHs zeigen eine ausgeprägtere Verwandtschaft als andere HSDHs. Interessanterweise ist die prokaryotische 7β-HSDH mit der tierischen 11 β-HSDH-Untergruppe, umfassend *Cavia porcellus, Homo sapiens* und *Mus musulus,* verwandt.

**2.8.5. Kinetische Konstanten**

Kinetische Gleichgewichtsanalysen wurden durchgeführt, um die absoluten Werte für $V_{max}$ und $K_M$ für UDCS, 7-Keto-LCS, DHCS, $NADP^+$ und NADPH mit Hilfe von Lineweaver-Burk-Plots zu bestimmen. In folgender Tabelle sind sämtliche kinetische Daten für die getesteten Substrate und Coenzyme, die aus Substratsättigungskurven und reziproke Auftragungen erhalten wurden, zusammengefasst. Die $V_{max}$, $K_M$ and $k_{cat}$-Werte für sämtliche Substrate und Coenzyme liegen im gleichen Bereich, wohingegen der $K_M$-Wert für DHCS signifikant höher lag als bei den anderen Substraten, eventuell bedingt durch die geringe Löslichkeit in Wasser. Das Enzym ist NADPH-abhängig und kinetische Konstanten für $NAD^+$ und NADH konnten aufgrund der sehr geringen Aktivität nicht bestimmt werden.

Zusammenfassung von kinetischen Konstanten für 7β-HSDH aus *Collinsella aerofaciens* DSM 3979.

| | $K_M$ (μM) | $V_{max}$ (U/mg Protein)[b] | $k_{cat}$ (1 μmol/(μmol$_X$min)) |
|---|---|---|---|
| $NADP^+$ | 5.32 | 30.58 | 944.95 |
| NADPH | 4.50 | 33.44 | 1033.44 |
| UDCS | 6.23 | 38.17 | 1179.39 |
| 7-Keto-LCS | 5.20 | 30.77 | 950.77 |
| DHCS | 9.23 | 28.33 | 875.35 |
| $NAD^+$ | -[a] | - | Spuren |
| NADH | - | - | Spuren |

[a] konnten aufgrund der sehr geringen Aktivität nicht bestimmt werden
[b] 1 U = 1 μmol/min

**2.8.6. pH-Optimum**

Weiterhin wurde die 7β-HSDH-Aktivität für verschiedene Substrate in Abhängigkeit vom pH-Wert mit gereinigtem Enzym bestimmt. Für die Oxidation von UDCS mit 7β-HSDH wurde eine optimale Aktivität im Bereich von pH 9 bis 10 bei allmählichem Abfall auf der sauren Seite beobachtet. Im Gegensatz dazu ergab sich für die Reduktion von DHCS und 7-keto-LCS durch die 7β-HSDH eine optimale Aktivität im Bereich von pH 4 bis 6 mit einem scharfen Abfall auf der sauren Seite und einem allmählichen Abfall auf der alkalischen Seite. Unterschiedliche Puffer haben nur einen geringen Einfluss auf die Aktivität der 7β-HSDH bei gleichem pH-Wert.

**2.8.7. Thermische Stabilität**

Die erfindungsgemäß verwendete, NADP-abhängige 7β-HSDH zeigt folgendes Stabilitätsverhalten: Nach 400 min war die Aktivität bei 30°C etwa 30% niedriger als bei 23°C. Das Enzym war bei 30°C nach 1500 min vollständig inaktiviert, während bei 23°C und 1500 min die verbleibende Aktivität 20% betrug. Kein signifikanter Aktivitätsverlust wurde während der Lagerung bei -20°C in Kaliumphosphatpuffer (50 mM, pH 8) über einen Zeitraum von einigen Monaten nach mehrmaligem Einfrieren und Auftauen beobachtet.

**Herstellungsbeispiel 3: Herstellung von 7β-HSDH Mutanten und deren Charakterisierung (nicht erfindungsgemäß)**

Es wurde die Position 39 der Aminosäuresequenz (beinhaltend Start-Methionin) (vgl. SEQ ID NO: 2) mutiert.

**3.1 Primer**

Für die positionsgerichtete Mutagenese der 7ß-HSDH wurden die unten angegeben Mutageneseprimer verwendet. Die Primer wurden auf Grundlage der 7ß-HSDH-Gensequenz dahingehend ausgewählt, dass sie den gewünschten Aminosäurenaustausch bewirken. Es wurde darauf geachtet, dass die zu mutierende Base im Primer mittig lokalisiert ist, und dass die Schmelztemperaturen der Primerpaare sich im gleichen Bereich befinden.

Zur Erzeugung der G39A-Mutante wurde das Primerpaar 7beta_mut G39A finid und 7beta *mut_G39A_rev* verwendet. Zur Erzeugung der G39S-Mutante wurde das Primerpaar 7beta_mut_G39S_fwd und 7beta_mut_G39S_rev verwendet.

*Glycin → Alanin*

Forward: 7beta_mut_G39A_fwd: CGTCGTCATGGTCGCCCGTCGCGAGG. SEQ ID NO: 9)

Revers: 7beta_mut_G39A_rev: CCTCGCGACGGGCGACCATGACGACG. SEQ ID NO: 10)

*Glycin → Serin*

Forward: 7beta_mut_G39S_fwd: CGTCGTCATGGTCAGCCGTCGCGAGG. SEQ ID NO: 11)

Revers: 7beta_mut_G39S_rev: CCTCGCGACGGCTGACCATGACGACG. SEQ ID NO: 12)

**3.2 PCR-Programm**

Bei der Reaktion erfolgte zuerst ein 2 min langer initialer Denaturierungsschritt bei 98°C. Danach wurden 25 Zyklen von Denaturierung (30 s bei 98°C), Primerhybridisierung (2,5 min bei 58°C) und Elongation (6 min bei 72°C) durchgeführt. Als letzter Schritt erfolgte eine finale Elongation von 15 min bei 72°C bevor die Polymerasekettenreaktion durch Kühlen auf 4°C beendet wurde.

**3.3 PCR-Ansatz**

| | |
|---|---|
| HF-Puffer (5x) | 4 μl |
| dNTP-Mix (10 mM) | 0,4 μl |
| *Forward*-Primer (10 μM) | 2 μl |
| *Revers*-Primer (10 μM) | 2 μl |
| Templat | 1 μl |

(fortgesetzt)

| | |
|---|---|
| *Phusion* Polymerase (2 U µL$^{-1}$) | 0,2 µl |
| DMSO | 1 µl |
| dd$H_2O$ | 9,4 µl |
| | **20 µl** |

Als Templat wurde ein pET22b-Vektor mit der 7β-HSDH verwendet

### 3.4 Durchführung

Um Aminosäuren in Proteinsequenzen gezielt austauschen zu können wird die DNA-Sequenz des entsprechenden Gens positionsgerichtet mutiert. Hierzu werden zueinander komplementäre Primer genutzt, die die gewünschte Mutation in ihrer Sequenz tragen. Als Templat dient N6-adeninmethylierte, doppelsträngige Plasmid-DNA, die das zu mutierende Gen tragen. N6-adeninmethylierte Plasmid-DNA werden aus dam$^+$ *E. coli*-Stamm wie zum Beispiel *E. coli* DH5 isoliert.

Die Polymerasekettenreaktion wird wie oben beschrieben durchgeführt. Dabei werden die Primer komplementär zum Templat verlängert, wodurch Plasmide mit der gewünschten Mutation entstehen, die einen Strangbruch aufweisen. Anders als bei anderen PCR-Reaktionen steigt die DNA-Ausbeute hierbei nur linear, da neu gebildete DNA-Moleküle nicht als Templat für die PCR-Reaktion dienen können.

Nach Abschluss der PCR-Reaktion wird die parentale, N6-adeninmethylierte mit Hilfe des Restriktionsenzyms Dpnl verdaut. Dieses Enzym hat die Besonderheit, dass es unspezifisch N6-adeninmethylierte DNA restringiert, jedoch nicht die neu gebildete, nichtmethylierte DNA. Die Restriktion erfolgte, indem 1 µL Dpnl zum PCR-Reaktionsansatz hinzu gegeben und 1 h bei 37°C inkubiert wurde.

10 µl dieses Ansatzes wurden zur Transformation von 200 µl chemisch-kompetenten DH5α-Zellen verwendet. Nach Plasmidisolation wurde die erfolgreiche Mutation mittels Sequenzierung bestätigt.

### 3.5 Charakterisierung

Für jede der Mutanten sowie für das Wildtyp-Enzym wurden Kinetikmessungen im Mikrotiterplattenphotometer durchgeführt, indem die Umsatzraten der Enzyme sowohl mit gleichbleibenden Substratkonzentrationen bei variierten Cofaktorkonzentrationen als auch *vice versa* aufgezeichnet wurden. Zur Bestimmung der spezifischen Enzymaktivität wurden die Enzymkonzentrationen im Zelllysat densitometrisch bestimmt.

Zur Ermittlung der Abhängigkeit der Substratumsatzgeschwindigkeit von der Substratkonzentration wurde eine Cofaktorkonzentration von 100 µM NADPH bezogen auf das Reaktionsvolumen eingesetzt, während die Substratkonzentration über einen Bereich von 7 µM) bis 10 mM Dehydrocholsäure mit 31 verschiedenen Konzentrationen variiert wurde (jeweils bezogen auf den Reaktionsansatz).

Zur Ermittlung der Abhängigkeit der Substratumsatzgeschwindigkeit von der Cofaktorkonzentration wurde eine Substratkonzentration von 0,3 mM Dehydrocholsäure bezogen auf das Reaktionsvolumen eingesetzt, während die Cofaktorkonzentration über einen Bereich von 25 µM) bis 100 µM) NADPH mit 8 verschiedenen Konzentrationen beim Wildtypprotein bzw. über einen Bereich von 6 µM) bis 100 µM) NADPH mit 16 verschiedenen Konzentrationen bei den beiden Mutanten variiert wurde (jeweils bezogen auf den Reaktionsansatz). Die Reaktionsgeschwindigkeit wurde bei diesen Messreihen durch lineare Regression über die ersten 4 Messungen (0 s - 18 s) ermittelt.

Die erhaltenen Daten wurden mit IGOR Pro ausgewertet. Aus den Auftragungen der Substrat- bzw. Cofaktorkonzentration gegen die spezifische Enzymaktivität lässt sich für die Messreihen bei konstanten Substrat- und variierten Cofaktorkonzentrationen der typische Kurvenverlauf einer Michaelis-Menten-Kinetik erkennen. Aus den Auftragungen der Messreihen mit konstanten Cofaktor- und variierten Substratkonzentrationen können hingegen typische Kurvenverläufe einer Michaelis-Menten-Kinetik mit Substratinhibierung erkannt werden (vgl. Figur 6). Aus dem Grund wurde das klassische Michaelis-Menten-Modell für die Auswertung der Messreihen bei konstanten Substrat- und variierten Kosubstratkonzentrationen und das Michaelis-Menten-Modell mit Substratinhibierung für die Messreihen mit konstanten Kosubstrat- und variierten Substratkonzentrationen verwendet.

$V_{max}$, $K_m$ und $K_i$ wurden mittels nichtlinearer Regression ermittelt.

$$v = v_{max} \cdot \frac{c_S}{K_m + \left(1 + \frac{c_S}{K_i}\right) c_S}$$

| | |
|---|---|
| $v$ | spezifische Enzymaktivität, $U\ mg^{-1} = \mu mol\ min^{-1}\ mg^{-1}$ |
| $v_{max}$ | maximale spezifische Enzymaktivität, $U\ mg^{-1} = \mu mol\ min^{-1}\ mg^{-1}$ |
| $c_S$ | Substrat- bzw. Kofaktorkonzentration, $mol\ L^{-1}$ |
| $K_m$ | Halbsättigungskonzentration, $mol\ L^{-1}$ |
| $K_i$ | Inhibitionskonstante, $mol\ L^{-1}$ |

Die ermittelten Parameter sind in Tabelle aufgeführt.

**Tabelle** : Ermittelte Parameter der drei verschiedenen 7β-HSDH-Varianten.

| | $K_{m,DCS}$, μM | $K_{i,DCS}$, mM | $K_{m,NADPH}$, μM | $V_{max}$, U/mg |
|---|---|---|---|---|
| 7β-HSDH WT | 31 ± 5 | 8,6 ±1,5 | 46 ± 7 | 14,6 ± 1,0 |
| 7β-HSDH G39A | 33 ± 6 | 17 ± 4 | 16,4 ± 1,9 | 21,0 ± 1,1 |
| 7β-HSDH G39S | 75 ± 9 | 80 ± 50 | 13,1 ± 2,9 | 20,7 ± 1,0 |

Bei Betrachtung der gemessenen Daten fällt auf, dass die 7β-HSDH eine Substratinhibierung durch Dehydrocholsäure zeigt, die bei dem Wildtypprotein am stärksten auftritt und bei der G39S-Mutante am schwächsten ist. Vor allem bei dem letztgenannten Protein lässt sich die Frage stellen, ob überhaupt eine Substratinhibierung vorliegt, wie der hohe $K_i$ und dessen großer Fehler zeigen. Die Halbsättigungskonzentrationen für Dehydrocholsäure liegen im zweistelligen mikromolaren Bereich. Während sich diese für das Wildtypprotein und die G39A-Mutante mit 31±5 μM) bzw. 33±6 μM nicht signifikant unterscheiden, liegt dieser Wert bei der G39S-Mutante mit 75±9 μM) in etwa bei dem doppelten Wert der anderen zwei Enzyme. Bei den Halbsättigungskonzentrationen für den NADPH zeigen sich bei den Mutantenenzymen niedrigere Werte als bei dem Wildtyp-Enzym (46±7 μM) beim Wildtyp gegenüber 16,4±1,9 μM bei der G39A-Mutante bzw. 13,1±2,9 μM bei der G39S-Mutante).

Die Werte für $V_{max}$ liegen bei beiden Mutanten in etwa bei dem 1,5-fachen Wert des Wildtypproteins (21,0±1,1 U/mg bei der G39A-Mutante bzw. 20,1±1,0 U/mg bei der G38S-Mutante gegenüber 14,6±1,0 U/mg bei dem Wildtypprotein). Anders als bei dem klassischen Michaelis-Menten-Modell sind bei dem Michaelis-Menten-Modell mit Substratinhibierung die $v_{max}$ Werte jedoch nicht als maximal erreichbare spezifische Enzymaktivitäten zu sehen, da sich die spezifischen Enzymaktivitäten mit zunehmender Substratkonzentration nicht asymptotisch an $v_{max}$ annähern, sondern wieder abnehmen. Die maximal erreichbaren Enzymaktivitäten, d.h. die spezifischen Enzymaktivitäten bei den Maxima der Kinetikkurven, liegen bei dem Wildtyp-Protein bei ~13 U/mg und bei den Mutantenproteinen bei ~19 U/mg (G39A) bzw. ~20 U/mg(G39S). Interessanter als die maximal erreichbaren Enzymaktivitäten sind die Enzymaktivitäten, die bei der für eine Ganzzellbiotransformation eingesetzten Substratkonzentrationen erreicht werden können. Diese sollen exemplarisch für die Substratkonzentration von 10 mM Dehydrocholsäure verglichen werden und betragen bei dem Wildtyp-Protein ~6,7 U/mg, für die G39A-Mutante ~13 U/mg und für die G39S-Mutante ~18 U/mg. Bei 10 mM Substratkonzentration wäre die G39A-Mutante demnach doppelt so aktiv wie das Wildtyp-Protein, die G39S-Mutante hätte sogar knapp dessen 3-fache Aktivität. Diese Unterschiede sollten bei höheren Substratkonzentrationen noch stärker ausgeprägt sein, da das Wildtyp-Protein die stärkere Substratinhibierung als die G39A-Mutante zeigt, und die G39A-Mutante wiederum stärker substratinhibiert ist als die G39S-Mutante.

**Herstellungsbeispiel 4: Herstellung und Charakterisierung der FDH D221G Mutante**

**4.1 Klonierung pET21a(+) FDH**

**4.1.1** PCR-Amplifizierung der *Mycobacterium vaccae* Formiatdehydrogenase.

Als Templat der Amplifizierung dient genomische DNA von *Mycobacterium vaccae,* welche von der Deutschen Sammlung von Mikroorganismen und Zellkulturen GmbH (DMSZ), Braunschweig bezogen wurde. Primer für die Amplifizierung waren fdh_for (5'-CGATCATATGGCAAAGGTCCTGTGCGTTC-3') (SEQ ID NO:23) und fdh_rev (5'-GCTAGAATTCTCAGCCGCCTTCTTGAACT-3') (SEQ ID NO:24), bezogen von Eurofins MWG GmbH, Ebersberg. Die Erkennungsstellen für die Restriktionsenzyme sind unterstrichen. Der rev-Primer enthält die *EcoRI* Schnittstelle der for-Primer

enthält die *NdeI*-Schnittstelle.

Die PCR-Ansätze und PCR-Programme sind in den Tabellen angegeben.

Tabelle: PCR-Ansatz für die Amplifizierung der Formiatdehydrogenase aus *Mycobacterium vaccae*

| Komponente | Volumen [µl] |
|---|---|
| 10x Taq Puffer (mit $Mg^{2+}$) | 5 |
| dNTPs (10 mM) | 1 |
| Fdh_for (100 µM) | 0,5 |
| Fdh_rev (100 µM) | 0,5 |
| Templat-DNA (>=100 ng/µL) | 1 |
| Taq DNA Polymerase (5 U/mL) | 0,5 |
| Destilliertes Wasser | 41,5 |

Tabelle: PCR-Programm für die Amplifizierung der Formiatdehydrogenase aus *Mycobacterium vaccae*

| Segment | Zyklenzahl | Denaturierung | Annealing | Elongation |
|---|---|---|---|---|
| 1 | 1 | 94°C, 2 min | | |
| 2 | 5 | 94°C, 30 sec | 55,6°C, 30 sec | 72°C, 75 sec |
| 3 | 25 | 94°C, 30 sec | 58,6 °C, 30 sec | 72°C, 75 sec |
| 4 | 1 | | | 72°C, 75 sec |

**4.1.2** Restriktionsverdau von pET21a(+) und FDH-PCR-Produkt

1-5 µg in Wasser gelöste DNA (pET21a(+) bzw. FDH-PCR-Produkt) werden mit 5µl 10x NEBuffer *EcoR*I, 2,5 µl NdeI (20 U/mL) und 2,5 µl EcoRI (20 U/mL) jeweils New England Biolabs, Frankfurt) versetzt und mit destilliertem Wasser auf 50 µl Gesamtvolumen aufgefüllt. Die Ansätze wurden jeweils 1 h bei 37 °C inkubiert. Im Anschluss werden die geschnittenen DNA-Fragmente auf ein 1 %-iges Agarosegel (1 % (m/v) Agarose, 0,05 % (v/v) Ethidiumbromid) aufgetragen und die DNA-Fragmente 55 Minuten bei 120 V elektrophoretisch aufgetrennt. Anschließend wurden die Banden der richtigen Größe (1,2 kb bei dem FDH-Gen, 5,4 kb bei dem pET21a(+)-Plasmid) mit einem Skalpell aus dem Agarosegel ausgeschnitten und mit Hilfe des QIAQuick Gel Extraction Kit (QIAGEN, Hilden) gemäß Herstellerprotokoll isoliert.

**4.1.3** Ligation von geschnittenem pET21a(+) und FDH

100 ng geschnittene Vektor-DNA und 111 ng geschnittene FDH-DNA wurden mit 1 µl T4 Ligase (3 U/µL) und 1 µl 10x Ligase Puffer (jeweils New England Biolabs, Frankfurt) versetzt und mit destilliertem Wasser auf ein Gesamtvolumen von 10 µl aufgefüllt. Der Ligationsansatz wurde über Nacht bei 4°C inkubiert.

**4.1.4** Transformation des Ligationsansatzes in chemisch kompetente *E. coli* DH5α

Nach Beendigung des Ligationsschritts werden die 10 µL Ligationsansatz zu 200 µl nach Standardprotokoll hergestellte, chemisch kompetente *E. coli* DH5α gegeben. Danach erfolgt 30 Minuten lang ein Inkubationsschritt auf Eis, gefolgt von einem Hitzeschock bei 42°C (90 Sekunden). Anschließend werden 600 µl steriles LB-Medium zum Transformationsansatz zugegeben und die Zellen bei 200 rpm und 37°C in einem Schüttelinkubator 45 Minuten inkubiert. Im nächsten Schritt wird der Ansatz bei 3000 rpm 60 Sekunden in einer Tischzentrifuge abzentrifugiert, 700 µl vom Überstand werden verworfen, die Zellen im restlichen Überstand resuspendiert und auf einer LB-Agarplatte mit 100 mg/l Ampicillin ausplattiert. Die Agarplatte wird im Anschluss bei 37°C über Nacht inkubiert.

### 4.2. Herstellung pET21a(+) FDH D221G

Die Herstellung einer FDH Mutante, welche nicht nur NADH sondern auch NADHP regenerieren kann, wird anhand der Mutante D221G näher erläutert.

Bei Aspartat (D) 221 handelt es sich um eine Aminosäure mit einer negativ geladenen, großen Seitenkette, die direkt neben dem Arginin (R)-Rest, durch welchen $NADP^+$ gebunden werden soll, liegt. Dies kann zur Abstoßung der Phosphatgruppe im $NADP^+$, die ebenfalls negativ geladen ist, führen. Das Aspartat wird daher durch den kleinen ungeladenen Aminosäurerest Glycin (G) ersetzt

#### 4.2.1 Verwendete Primer

| | |
|---|---|
| mt1: | 5'- C CTG CAC TAC ACC G**G**C CGT CAC CGC CTG C - 3' (SEQ ID NO: 30) |
| NI_fdh_R: | 5'-GCTCGAATTCTCAGACCGCCTTC--3'(SEQ ID NO:31) |

#### 4.2.2 Durchführung

Zunächst wurde mit Hilfe des mt-Primers und des Primers NI_fdh_R ein Set von zwei komplementären Megaprimern erzeugt.

Als Template wurde Plasmid pET21a(+)FDH verwendet. Das verwendete PCR-Programm ist folgender Tabelle zu entnehmen:

Tabelle: PCR-Programm Megaprimer

| Segment | Zyklenzahl | Denaturierung | Annealing | Elongation |
|---|---|---|---|---|
| 1 | 1 | 94°C, 2 min | | |
| 2 | 30 | 94°C, 30 sec | 60°C, 30 sec | 72°C, 40 sec |
| 3 | 1 | 72°C, 5 min | | |

Durch Kombination von Primer mt1 mit dem Primer NI_fdh_R ergibt sich die Länge des Megaprimer zu 650 bp. Mit diesem PCR-Produkt der ersten PCR erfolgt eine Gelelektrophorese und Isolierung der gewünschten Bande aus dem Gel. Eine zweite PCR wird als Whole Plasmid PCR mit den Megaprimern als Primer und der Plasmid-DNA (pETfdh) als Template durchgeführt. Den Reaktionsansatz und das Temperaturschema für die Whole Plasmid PCR zeigen die folgenden Tabellen. Der 2x EZClone enzyme mix, die EZClone Solution 1, der 1,1 kb Marker und das *DpnI* stammten aus dem GeneMorph II EZClone Domain Mutagenesis Kit (Stratagene).

Tabelle: Ansatz für eine MEGA WHOP PCR (Gesamtvolumen 50 μL)

| Komponente | |
|---|---|
| Megaprimer | 250 ng (~ 2,5 μL aus einer Standard-PCR) |
| Template (pETfdh) | 50 ng |
| 2x EZClone enzyme mix | 25 μL |
| EZClone Solution 1 | 3 μL |
| dest. Wasser | ad 50 μL |

Der erste Schritt im PCR-Programm (68°C, 5 min) dient dazu, die durch die *Taq*-Polymerase unspezifisch angehängten Basen durch die 3'->5' Exonukleaseaktivität der in der MEGA WHOP PCR eingesetzten Polymerase zu entfernen.

Tabelle: PCR-Programm MEGA WHOP

| Segment | Zyklenzahl | Denaturierung | Annealing | Elongation |
|---|---|---|---|---|
| 1 | 1 | | | 68°C, 5 min |

(fortgesetzt)

| Segment | Zyklenzahl | Denaturierung | Annealing | Elongation |
|---|---|---|---|---|
| 2 | 1 | 95°C, 1 min | | |
| 3 | 25 | 95°C, 50 sec | 60°C, 50 sec | 68°C, 13 min |

Das PCR-Produkt ist ein doppelsträngiges Plasmid mit Einzelstrangbrüchen, die erst in *E. coli* geschlossen werden. Zu dem 50 µL PCR-Produkt wurden 10 U *DpnI* zugesetzt und der Ansatz bei 37°C für zwei Stunden inkubiert. *DpnI* baut nur methylierte DNA ab, d.h. die eingesetzte Template-DNA, nicht aber den Megaprimer oder das synthetisierte Plasmid. Das Template-Plasmid muss mit einem $dam^+$-Stamm (wie DH10B oder JM109) erzeugt werden, um methylierte Ausgangs-DNA zu erhalten.

**4.3 Expression und Isolierung der Mutante D221G**

Zunächst wurde eine LB-Vorkultur aus einer Gefrierkonserve oder mit einer Kolonie von einer Agarplatte inokuliert und die Vorkultur über Nacht inkubiert. Die Inkubation erfolgte bei 37°C und 250 Upm. Die OD der Vorkultur wurde bestimmt und die Kultur auf eine OD von 0,1 angeimpft. Beim Erreichen einer OD von 0,5-1 (nach etwa 2,5 h) wurde mit IPTG (Endkonzentration 1 mM) induziert. Die Zellen wurden drei Stunden nach der Induktion geerntet.
Zum Zellaufschluss wurde die mechanistische Methode mit Glasperlen eingesetzt. Dazu wurden zu 0,5 mL Zellprobe in einem 1,5 mL Reaktionsgefäß 0,5 mL Glasperlen zugegeben und das Reaktionsgefäß für 3 min bei maximaler Frequenz (30 $sec^{-1}$) in der Retsch-Schwingmühle geschüttelt. Nach dem Zellaufschluss wurden die Glasperlen abzentrifugiert (2 min, 13000 rpm). Die Kultur wurde vor und nach dem Aufschluss auf Eisgestellt, um die Proteindenaturierung durch die Erwärmung der Probe in der Schwingmühle so gering wie möglich zu halten. Dieses Aufschlussprotokoll ist für die Verwendung von bei -20°C eingefrorenen Proben optimiert.

**4.4 Charakterisierung der Mutante D221G**

Zusammenfassung kinetischer Konstanten der FDH bei pH 6, 7 und 8..

| Enzym | pH | Substrat | Cofaktor | Spezifische Aktivität (U × $mg^{-1}$) | $K_M$ (mM) Substrat | $K_M$ (mM) Cofaktor |
|---|---|---|---|---|---|---|
| FDH | 6 | Natriumformiat | $NAD^+$ | 4.6 | 46.86 | 0.83 |
| | 6 | Natriumformiat | $NADP^+$ | 1 | | 0.3 |
| | 7 | Natriumformiat | $NAD^+$ | 5.1 | 45.56 | 0.61 |
| | 7 | Natriumformiat | $NADP^+$ | 1.2 | | 0.51 |
| | 8 | Natriumformiat | $NAD^+$ | 4.7 | 59.26 | 0.52 |
| | 8 | Natriumformiat | $NADP^+$ | 1 | | 1.01 |

Die Daten belegen, dass die hergestellte Mutante sowohl $NAD^+$ als auch $NADP^+$ als Cofakor verwenden kann.

**Herstellungsbeispiel 5: Herstellung einer *E. coli* 7α-HSDH Knockout-Mutante (*E. coli* BL21 (DE3) hdhA$^-$ KanR$^+$)**

Ziel ist die Deletion der störenden 7α-HSDH Aktivität im Expressionsstamm *E. coli* BL21 (DE3).
Mit der im Folgenden beschriebenen Methode wird in das Zielgen der 7α-HSDH ein Antibiotika-Resistenzgen insertiert, wodurch das Zielgen ausgeschalten wird.

**5.1 Sequenzinformation zur 7α-HSDH aus *E. coli* BL21(DE3)**

Aminosäuresequenz: (SEQ ID NO:26)
Nukleotidsequenz (SEQ ID NO:25)
Accession: NC_012971 REGION: 1642470..1643237

### 5.2 Verwendete Primer

Folgende Primer für das Ausschalten der 7α-HSDH aus *E. coli* BL21(DE3) wurden hergestellt:

Primer für das Retargeting des LI.LtrB Introns:

467|468a-IBS (SEQ ID NO:27)
AAAAAAGCTTATAATTATCCTTATAGGACGTCATGGTGCGCCCAGATAGGGTG

467|468a-EBS1d (SEQ ID NO:28)
CAGATTGTACAAATGTGGTGATAACAGATAAGTCGTCATGTTTAACTTACCTTTCTTTGT

467|468a-EBS2 (SEQ ID NO:29)
TGAACGCAAGTTTCTAATTTCGGTTTCCTATCGATAGAGGAAAGTGTCT

Insertion
Location
467|468a GCAGCTTTAGATGATGCATAGGAAGTCATG - intron - TTTATATTTTTATTT

### 5.3 Herstellung der Knockout-Mutante

Die Herstellung der Knockout-Mutante wurde mit Hilfe des Kits TargeTron™ Gene Knockout System von Sigma Aldrich nach Herstellerangaben durchgeführt. Es wurde zur Aufreinigung des PCR-Produkts gemäß Schritt B.6. des TargeTron™ Gene Knockout System der QIAquick PCR Purification Kit von Qiagen genutzt.

Die Ligation des HindIII/BsrGI-verdauten Intron-PCR-Produkts in den linearisierten pACD4K-C Vektor wurde wie folgt durchgeführt: Die Reaktion erfolgte über Nacht bei 16°C.

20µl-Ansatz:

| | |
|---|---|
| 2 µl | pACD4K-C linear vector (40 ng) |
| 6 µl | HindIII/BsrGI-digested intron PCR product |
| 2 µl | ATP (10 mM) |
| 2 µl | Ligase-Puffer (10 x) (Fermentas) |
| 2 µl | T4-Ligase (Fermentas) |
| 6 µl | $H_2O$ |

5 µl Ligationsreaktionslösung wurden zu 200 µl chemisch-kompetenten Zellen *E. coli* BL21 (DE3) gegeben und für 20 min auf Eis inkubiert. Die weitere Transformation erfolgte wie von Herstellerseite beschrieben.

Die Transformations-Ansätzewurden auf LB-Agar-Platten, enthaltend 33 µg/mL Kanamycin, ausplattiert. Es wurden Kanamycin-resistente Zellen gepickt und diese jeweils über mehrere Nächte in 5 ml LB-Übernacht-Kulturen überimpft (jeweils mit 5 µl einer Kanamycin-Lösung (33 mg/ml)). Abschließend wurde eine 200 ml LB-Kultur (mit 200 µl Kanamycin-Lösung (33 mg/mL)) mit einer Übernachtkultur angeimpft und für 5 h bei 37°C und 180 rpm im Schüttelinkubator inkubiert. Anschließend wurde die Temperatur auf 42°C für 1 Stunde erhöht. Mit dieser Kultur wurde eine 5 mL LB-Übernacht-Kulturen angeimpft (jeweils mit 5 µl einer Kanamycin-Lösung (33 mg/mL)). Nach Inkubation über Nacht bei 37°C und 180 rpm wurde die Kultur auf einer LB-Agar-Platte mit 33 µg/mL Kanamycin ausgestrichen. Nach Übernacht-Inkubation bei 37°C wurden Kolonien gepickt und auf LB-Agar-Platte mit 33 µg/mL Kanamycin und 34 µg/mL Chloramphenicol ausgestrichen.

Nach Übernacht-Inkubation bei 37°C wurden Chloramphenicol-sensitive Mutanten gefunden. Dies ist erforderlich, um den Verlust des Plasmids, welches das induzierbare Knockout-System trägt und nach erfolgreichem Knockout nicht mehr benötigt wird, zu bestätigen.

### 5.4. Nachweis des Knockouts

Das 7α-HSDH-Gen wurde über Kolonie-PCR mit den Primern
7alpha-ko-check_fwd (5' - TTAATTGAGCTCCTGTACCCCACCACC - 3') SEQ ID NO:32 und
7alpha-ko-check_rev (5' - GTGTTTAATTCTGACAACCTGAGACTCGAC - 3') SEQ ID NO:33
amplifiziert. Das entstandene Fragment hatte eine Länge von ca. 2,5-3 kb und wurde mit dem Primer 7alpha-ko-check_fwd sequenziert. Anhand der Sequenzierung konnte nachgewiesen werden, dass die DNA-Sequenz der 7α-HSDH durch ein Insert aus dem pACD4K-Vektor unterbrochen ist, was den Knockout der 7α-HSDH zur Folge hat. (Sequenzierdaten nicht gezeigt)

**Umsetzungsbeispiel 1: Enzymatische Umsetzung von 7-Keto-LCS durch die 7β-HSDH (nicht erfindungsgemäß)**

Zur Überprüfung der biochemischen Funktion der 7β-HSDH, wurde eine Umsetzung von 7-Keto-LCS durch die 7β-HSDH durchgeführt. Die 20 ml Umsetzung enthält 50 mM 7-Keto-LCS (ca. 0,4 g), 5 U/ml 7β-HSDH und 0,05 mM $NADP^+$. Zur Regenerierung von NADPH wurde 4 U/ml ADH und 1% Isopropanol eingesetzt (siehe Schema 1). Die Reaktion wurde bei pH8 und 24°C unter Rühren im Abzug durchgeführt. Da Aceton schneller als Isopropanol verdunstet, wird die Reaktion in Bildung von UDCS geschoben. 1% Isopropanol wurde nach 24 h, 48 h und 72 h nachgegeben. Das Produkt wurde mittels DC analysiert (Kieselgel 60, Merck, Laufmittel Petrolether und Ethylacetat 1:10, vol:vol). Auf DC wurde das Produkt mit authentischen Referenzen 7-Keto-LCS, UDCS und CDCS verglichen. Die DC-Analyse zeigt, dass UDCS aus 7-Keto-LCS von der 7β-HSDH gebildet wurde. Das Enantiomer CDCS ist auf DC nicht nachweisbar.

Schema 1: Schematische Darstellung von Reduktion von 7-Keto-LCS durch die 7β-HSDH. Die ADH regeneriert den Cofaktor NADPH.

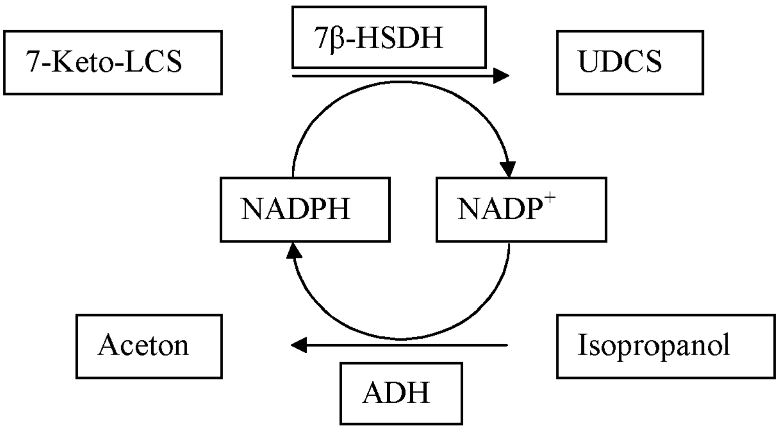


**Umsetzungsbeispiel 2: Enzymatische Herstellung von 3,12-Diketo-7β-CS aus DHCS durch 7β-HSDH (nicht erfindungsgemäß)**

Zur Überprüfung der Einsetzbarkeit der 7β-HSDH zu Herstellung von 12-Keto-UDCS aus DHCS, wurde eine Umsetzung von DHCS durch die 7β-HSDH durchgeführt. Die 50 ml Umsetzung enthält 50 mM DHCS (1 g), 5 U/ml 7β-HSDH und 0,05 mM $NADP^+$. Zur Regenerierung von NADPH wurde 4 U/ml ADH und 1% Isopropanol eingesetzt (siehe Schema 2). Die Reaktion wurde bei pH8 und 24°C unter Rühren im Abzug durchgeführt. Da Aceton schneller als Isopropanol verdunstet, wird die Reaktion in Bildung von 3,12-Diketo-7β-CS geschoben. Um vollständige Umsetzung zu erreichen, wurde 1% Isopropanol nach 24 h, 48 h und 72 h nachgegeben. Das Zwischenprodukt 3,12-Diketo-7β-CS wurde mittels DC analysiert. Das Edukt DHCS war nicht mehr nachweisbar auf DC (Kieselgel 60, Merck; Laufmittel Chlorform:Methanol:Essigsäure 10:1:0,08 vol:vol:vol).

Schema 2: Schematische Darstellung von Reduktion von DHCS durch die 7β-HSDH. Die ADH regeneriert den Cofaktor NADPH.

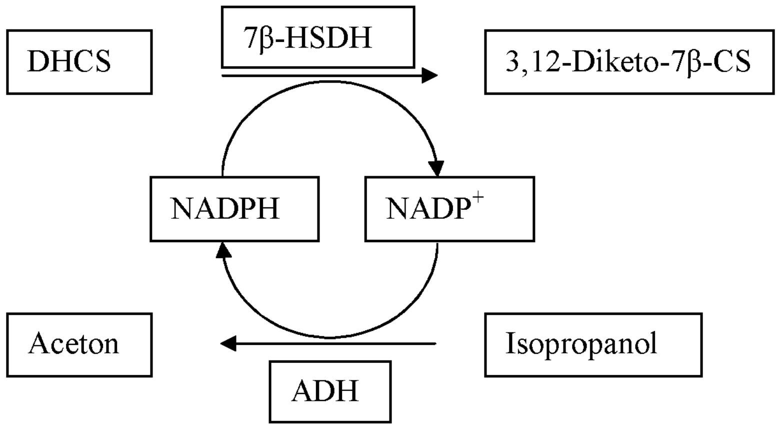


**Umsetzungsbeispiel 3: Enzymatische Umsetzung von 3,12-Diketo-7β-CS zu 12-Keto-UDCS (nicht erfindungsgemäß)**

Das Zwischenprodukt 3,12-Diketo-7β-CS (hergestellt gemäß Umsetzungsbeispiel 2) wurde von einer 3α-HSDH (SEQ ID NO:5 und 6) aus *Comamonas testosteroni* (Mobus, E. and E. Maser, Molecular cloning, overexpression, and characterization of steroid-inducible 3alpha-hydroxysteroid dehydrogenaselcarbonyl reductase from Comamonas testosteroni. A novel member of the short-chain dehydrogenaselreductase superfamily. J Biol Chem, 1998. 273(47): p. 30888-96) weiter zu 12-Keto-UDCS umgesetzt. Diese 3α-HSDH benötigt Cofaktor NADH, der von der FDH regeneriert wurde (siehe Abb. 3). Zu der Reaktion wurden 4U/ml 3α-HSDH, 1 U/ml FDH (NADH-abhängig, Codexis), 200 mM Natiumformiat und 0,05 mM $NAD^+$ zugegeben. Nach 40 h wurde das Produkt mit 2 M HCl bis pH2 angesäuert und mit 6 mal 10 ml Ethylacetat extrahiert. Nach Evaporation wurden 1,07 g Produkt erhalten. Das Produkt 12-Keto-UDCS wurde mittels DC und NMR analysiert und bestätigt. Die 3alpha-HSDH wurde analog zur Herstellung von 7ß-HSDH, jedoch mit dem Plasmid pET22b+ hergestellt und ohne weitere Aufreinigung verwendet.

Schema 3: Schematische Darstellung von Reduktion von 3,12-Diketo-7β-CS durch die 3α-HSDH. Die FDH regeneriert den Cofaktor NADH.

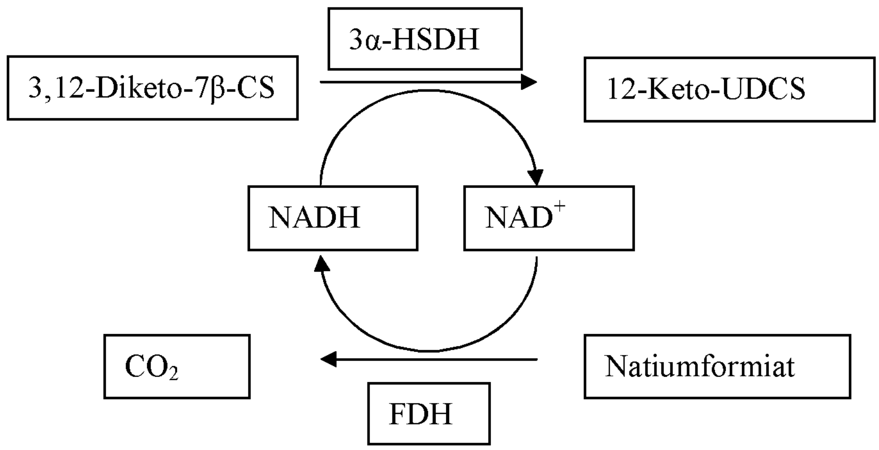

**Umsetzungsbeispiel 4: Chemische Umsetzung von CS zu DHCS**

In einem 2000L Rührwerksbehälter werden 1320L Eisessig vorgelegt und darin 110 kg (260mol) Cholsäure (CS) gelöst. Zu dieser Lösung werden 422 L Natriumhypochlorid Lösung (2,3 molar) bei 20 bis 40 °C zudosiert und die Reaktionslösung anschließend noch mindestens 1 Stunde zur Vervollständigung der Reaktion nachgerührt. Die Dehydocholsäure (DHCS)wird in einer Ausbeute von 100 kg (90%) durch Zentrifugieren isoliert.

**Umsetzungsbeispiel 5: Chemische Umsetzung von 12-Keto-UDCS zu UDCS**

105g (0,258mol) 12-Keto-UDCS werden in 384ml Triethylenglycol, 52,2g (1,304 mol) Natriumhydroxid und 75,95ml (1,563 mol) Hydrazinhydrat gelöst und langsam auf 180°C erwärmt. Dabei bildet sich das Hydrazon, das ab 160°C unter Abspaltung von Stickstoff in die UDCS umwandelt. Die Reaktion wird zur Vervollständigung der Umsetzung 8 Stunden bei 180°C gehalten. Die Reaktionsmischung wird unter 100°C gekühlt, mit 1500 ml Wasser versetzt. Anschließend durch Ansäuern mit Salzsäure die UDCS ausgefällt. Das Produkt wird in einer Ausbeute von 96,2 g bis 99,2 g (bis 95% - 98%) erhalten.

**Umsetzungsbeispiel 6: Enzymatische Umsetzung von DHCS zu 12-Keto-UDCS durch die 7β-HSDH, FHD D221G und 3α-HSDH (nicht erfindungsgemäß)**

In diesem Beispiel soll untersucht werden, ob eine zweistufige enzymatische Umsetzung von DHCS zu 12-Keto-UDCS bei gleichzeitiger Cofaktorregenerierung mit einer erfindungsgemäß verwendeten FDH-Mutante möglich ist. Da die verwendete FDH Mutante D221G sowohl $NADP^+$ als auch $NAD^+$ als Cofaktor akzeptiert, muss für die NADH-abhängige 3α-HSDH kein zusätzliches Cofaktorregenerierungssystem in den Reaktionsanssatz eingebracht werden.

Beispiele für zwei Teilreaktionen sind im Folgenden grafisch veranschaulicht. FDH* bezeichnet die Mutante FDH D221G.

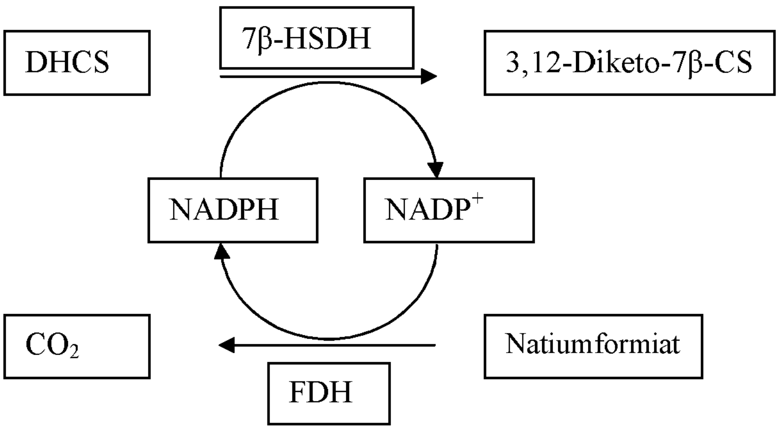


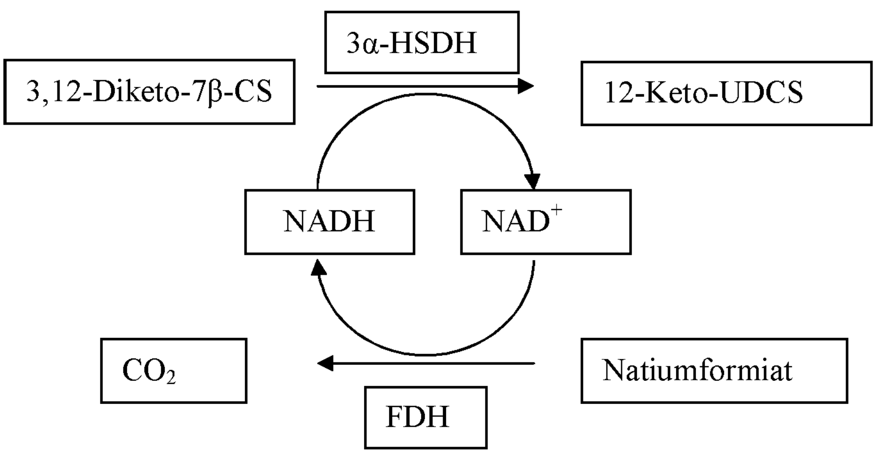


Hierzu wurden die Enzyme 7β-HSDH aus *Collinsella aerofaciens,* 3α-HSDH aus *Comamonas testosteroni* und

die FDH-Mutante D221G abgeleitet von FDH aus *Mycobacterium vaccae* getrennt voneinander in einem modifizierten *E. coli* Expressionsstamm exprimiert und zur Umsetzung verwendet. Der dabei für die Expression verwendete *E. coli* Stamm wurde so modifiziert, dass er keine 7α-HSDH-Enzymaktivität exprimiert. Diese in E. coli verbreitete Nebenaktivität kann bei Umsetzungen des vorliegenden Typs (stereospezifische Umsetzung der 7-Keto-Gruppe) zu unerwünschten Nebenreaktionen und damit zu Verunreinigungen des herzustellenden Umsetzungsproduktes führen.

In dem folgenden Experiment wurde der Knock-out Stamm *E. coli* BL21(DE3)Δ7α-HSDH verwendet, der in der älteren Europäischen Patentanmeldung EP 10164003.5 der Anmelderin beschrieben wird. Auf die Offenbarung dieser Patentanmeldung wird hiermit ausdrücklich Bezug genommen.

**6.1 Verwendete Plasmide,**

Plasmide für die Expression von 7β-HSDH, 3α-HSDH und FHD D221G:

pET28a(+)-7β-HSDH,
pET22b(+)-3α-HSDH und
pET21a(+)-FDH-D221G.

**6.2 Bakterienstämme und Kulturbedingungen:**

Der oben bezeichnete Knock-out Stamm *E. coli* BL21(DE3)Δ7α-HSDH wurde bei 37°C in LB-Medium, enthaltend die erforderlichen Antibiotika, gezüchtet. Nach Induktion mit 0,5 mM IPTG nach Erreichen von $OD_{600}$ = 0,8 wurde die Inkubation bei 140 Upm über einen Zeitraum von 12 Stunden bei 25°C fortgeführt.

**6.3 Enzymüberexpression und Reinigung**

Überexpression von 7β-HSDH, 3α-HSDH und der FDH-Mutante D221G in *E. coli* BL21(DE3)Δ7α-HSDH und Enzym-Reinigung erfolgten unter den gleichen Bedingungen wie oben in Herstellungsbeispiel 2 für die Überexpression und Reinigung von 7β-HSDH in *E. coli* BL21(DE3) beschrieben. Die Ausbeuten je Liter Kulturmedium (Schüttelkolben bei $OD_{600}$ ~ 6) waren wie folgt:

7β-HSDH: 3883U (für DHCS und NADPH)
3a-HSDH: 6853 U (für DHCS und NADH)
FDH-Mutante: 47 U (für Natriumformiat und $NAD^+$).

Gehalt und Reinheit der Proteine wurde mittels SDS-PAGE und Densitometerscanning mittels Scieon Image Beta 4.0.2 (Scieon, USA) bestimmt.

**6.4 Enzymatische Synthese von 12-Keto-UDCS im präparativen Maßstab**

Ein 800 ml Reaktionsansatz, enthaltend 7β-HSDH (2,4 U × $ml^{-1}$), 3α-HSDH (2,4 U × $ml^{-1}$), FDH D221G (0,325 U × $ml^{-1}$), $NADP^+$ (10 μM), $NAD^+$ (10 μM), Natriumformiat (250 mM), DHCS (10 mM, 3,2 g) und Kaliumphosphat-Puffer (50 mM, pH 6), wurde bei 24°C gerührt. Sämtliche drei Enzyme, die in diesem Experiment verwendet wurden, wurden als Zellrohextrakte ohne zusätzlichen Reinigungsschritt eingesetzt. Nach 12 Stunden wurde die Reaktion durch Entfernung der Enzyme mittels Ultrafiltration unter Verwendung einer Membran mit einer Porengröße von 10 kDa (Millipore, USA) gestoppt. Das Produkt im Filtrat wurde durch Ansäuern mit Salzsäure auf pH 2 und anschließende Papierfiltration gereinigt. Nach Trocknen des Produktes bei 60°C über Nacht erhielt man 2,9 g des gewünschten Produktes.

Das Produkt wurde mittels HPLC und NMR analysiert.

Analysendaten (partiell):

$^1H$ NMR (deuteriertes DMSO, 500 MHz) δ = 3,92 (2H, m, H-3α und H-7β) und
$^1H$- NMR (deuteriertes DMSO, 125 MHz) δ =69,38 (CH, 3-C); δ = 69,09 (CH, 7-C); δ = = 213,86 (C, 12-C)
Ausbeute: 90,6 %
Reinheit: 99 %

**Umsetzungsbeispiel 7: Ganzzellbiotransformation von DHCS zu 12-Keto-UDCS durch Coexpression von FDH D221G,** 7ß-HSDH **und 3α-HSDH im Zweiplasmid-System**

Es soll untersucht werden, ob eine zweistufige Ganzzellreduktion von Dehydrocholsäure (DHCS) zu 12-keto-Ursodesoxycholsäure (12-Keto-UDCS) (vgl. Schema gemäß Umsetzungsbeispiel 6, oben) möglich ist.

Hierzu wurde der oben hergestellte Knockout-Stamm *E. coli* BL21 (DE3) hdhA$^-$KanR$^+$ pET21a(+) FDH 7β-HSDH pCOLA(mod) 3α-HSDH verwendet, in dem zusätzlich zur 7β-HSDH und der Mutante FDH D221G eine 3α-HSDH aus *Comamonas testosteroni* rekombinant exprimiert wird. Da die verwendete FDH Mutante sowohl NADP$^+$ als auch NAD$^+$ als Cofaktor akzeptiert musste für die NADH-abhängige 3α-HSDH kein zusätzliches Cofaktorregenerierungssystem in den Biotransformationsstamm eingebracht werden.

**7.1 Verwendeter Stamm:**

Escherichia coli BL21 (DE3) hdhA$^-$ KanR$^+$

**7.2 Verwendete Molekularbiologische Arbeitsmethoden**

7.2.1 Polymerasekettenreaktion

Zur Klonierung der 7β-HSDH wurde die Polymerasekettenreaktion (PCR) eingesetzt. Als Templat zur Amplifikation der 7β-HSDH diente das Plasmid pET22b(+) 7β-HSDH

PCR-Reaktionen wurden in 500 μL PCR-Tubes mit 20 μL Reaktionsvolumen durchgeführt. Die Reaktionen wurden in einem Thermocycler der Firma Eppendorf durchgeführt. Bei der Amplifikation von 7β-HSDH wurden jeweils 4 μL HF-Buffer, 1 μL Templat DNA, jeweils 1 μL Vorwärts- und Revers-Primer (10 μM), 0,4 μL Desoxynukleotidtriphosphat-Lösung (10 mM) sowie 0,2 μL Phusion DNA-Polymerase (2 U/μL) zugegeben. Das Ansatzvolumen wurde mit RNAse-freiem Wasser auf 20 μL eingestellt.

Bei der Reaktion erfolgte zuerst ein 2 min langer initialer Denaturierungsschritt bei 98°C. Danach wurden 34 Zyklen von Denaturierung (30 s bei 98°C), Primerhybridisierung (2 min bei 48°C) und Elongation (2 min bei 72°C) durchgeführt. Als letzter Schritt erfolgte eine finale Elongation von 10 min bei 72°C bevor die Polymerasekettenreaktion durch Kühlen auf 4°C beendet wurde.

7.2.2 Aufreinigung von DNA-Fragmenten mittels Gelextraktion

Um DNA-Fragmente aufzureinigen wurden diese zunächst mittels Agarose-Gelelektrophorese aufgetrennt. Die entsprechenden Banden wurden mittels UV-Licht sichtbar gemacht, anhand ihrer Größe identifiziert und mit einem Skalpell aus dem Gel geschnitten. Die Extraktion erfolgte mit dem QIAquick Gel Extraction Kit nach Protokoll des Herstellers. Zur Elution der aufgereinigten DNA wurden 30-50 μL H2O verwendet.

7.2.3 Restriktion mit Endonukleasen

Die Restriktionsreaktionen wurden in einem Gesamtvolumen von 20-50 μL durchgeführt. Dabei wurde die zu schneidende DNA mit 10-20 U der jeweiligen Restriktionsenzyme versetzt. Zusätzlich wurde der laut Hersteller empfohlene Reaktionspuffer verwendet und je nach Herstellerempfehlung gegebenenfalls 0,5 μL Bovines Serumalbumin (BSA, 10 mg/mL) zugegeben. Der Restriktionsverdau erfolgte für 2 h bei 37°C. Anschließend wurden die restringierten Fragmente entweder über Gelextraktion (Vektorverdau-Produkte) oder mit Hilfe des QIAquick PCR Purification Kits (verdaute PCR-Produkte) aufgereinigt.

7.2.4 Aufreinigung von DNA-Fragmenten unter Verwendung des QIAquick PCR Purification Kits

Um restringierte PCR-Fragmente aufzureinigen wurde DNA mit Hilfe des QIAquick PCR Purification Kits aufgereinigt. Die Aufreinigung erfolgte nach Herstellerangaben, wobei zur Elution der aufgereinigten DNA 30-50 μL $H_2O$ verwendet wurde.

7.2.5 Ligation von DNA-Fragmenten

Für die Einklonierung restringierter DNA-Fragmente in Expressionsvektoren, wurden beide DNA-Moleküle mit denselben Restriktionsenzymen geschnitten. Durch die Verwendung zweier verschiedener Enzyme kann zum einen die Religation des Vektors verhindert werden und zum anderen das Insert in einer definierten Orientierung eingebaut werden. Als Enzym wurde die T4-DNA-Ligase verwendet, die die Bildung einer Phosphordiesterbindung zwischen einer freien 5'-Phosphatgruppe und einer freien 3'-OH-Ende einer Desoxyribonukleinsäure katalysiert.

Für die Klonierung der Expressionskonstrukte wurden jeweils 12 μL eines restringierten, aufgereinigten Vektors mit 4 μL eines gencodierenden, aufgereinigten DNA-Fragments mit restringierten Enden versetzt. Hinzu wurden 2 μL

10x Ligase-Puffer, 1 μL Adenosintriphosphat (ATP, 1 mM) und 1 μL T4-DNA-Ligase (400 U/μL) gegeben. Die Reaktionen verliefen über Nacht bei 16°C.

### 7.3 Verwendete Vektorkonstrukte

7.3.1 pET21a(+) FDH 7β-HSDH

Bei diesem Vektorkonstrukt wurde die 7ß-HSDH in pET21a(+) FDH einkloniert, so dass das 7beta-HSDH kodierende Gen *downstream* von der FDH gelegen ist. Zu diesem Zweck musste der FDH am 5'-Ende ein Stoppcodon eingefügt werden. Verglichen mit der Originalsequenz (Lys-Lys-Ala-Val-Stopp) wurde in diesem Konstrukt der C-terminale ValinRest durch einen Alanin-Rest ersetzt und weitere drei Aminosäuren angehängt, so dass folgende C-terminale Sequenz entsteht: Lys-Lys-Ala-Ala-Gly-Asn-Ser-Stopp. Des Weiteren wurde für eine gesteigerte Translation der 7ß-HSDH zwischen FDH und 7beta-HSDH eine zusätzliche Ribosomale Bindestelle eingefügt.

Zur PCR-Amplifikation der 7ß-HSDH wurden die Primer 7beta_fwd_EcoRI und S_7beta_rev_HindIII verwendet.

7beta_fwd_EcoRI (SEQ ID NO: 13:

5'-GC**GAATTC**GTGAAAGGAG*ATATAC*ATGAACCTGAGGGAGAAGTACGG-3'

S 7beta_rev_HindIII (SEQ ID NO: 3):

5'-CCCAAGCTTCTAGTCGCGGTAGAACGA-3'

Dargestellt sind die hybridisierende Sequenzbereiche (schwarz), Restriktionsschnittstellen (fett unterstrichen), Ribosomale Bindestellen (unterstrichen), Füllnukleotide (kursiv). Im Primer 7beta_fwd_EcoRI wurden zusätzlich ein Stoppcodon (doppelt unterstrichen) und ein Nukleotid zur Verschiebung des Leserahmens (fett) hinzugefügt.

Die Einklonierung erfolgte über die Schnittstellen *Eco*RI und *Hind*III.

7.3.2 pCOLA(mod) 3α-HSDH

Um Dehydrocholsäure zweistufig zu 12-keto-Ursodesoxycholsäure reduzieren zu können, müssen neben dem Cofaktorregenerierungssystem FDH auch die Enzyme 7β-HSDH und 3α-HSDH in einer Zelle vorliegen. Um das zu ermöglichen, wurde das Vektorkonstrukt pCOLA(mod) 3α-HSDH, ein modifiziertes Derivat des pCOLA-duet-Vektor, hergestellt, welches mit pET-Vektoren kotransformierbar ist.

Das 3α-HSDH codierende Gen wurde hierzu über die Schnittstellen *Nde*I und *Blp*I aus dem Vektor pET22b(+) 3α-HSDH ausgeschnitten und über dieselben Schnittstellen in MCS2 des pCOLA(mod)-Vektors einkloniert. Nach der Sequenzierung zeigte sich, dass an Position 45 der DNA-Sequenz ein Guanin durch ein Cytosin ersetzt wurde, woraus jedoch eine stille Mutation resultiert. Diese Mutation hat jedoch keine Auswirkung auf die Aminosäuresequenz, weshalb es sich hier um eine sogenannte Stille Mutation handelt.

Beide Vektoren wurden in den oben genannten Stamm wie unten beschrieben kotransformiert. Die Gene sind IPTG-induzierbar.

### 7.4 Hitzeschock-Transformation von *E. coli*-Zellen

Hierzu wurden 200 μL chemisch-kompetente *E. coli* BL21 (DE3) hdhA$^-$ KanR$^+$ oder DH5α aufgetaut und mit 1 μL DNA versetzt. Diese wurden zunächst 45 min auf Eis inkubiert. Danach wurden die Zellen 45 s bei 42°C einem Hitzeschock ausgesetzt. Anschließend wurde den Zellen 600 μL LB-Medium zugegeben und diese bei 37°C und 200 rpm geschüttelt, damit sie die gewünschte Antibiotikaresistenz ausbilden können. Daraufhin wurden die Zellen in einer Tischzentrifuge bei 3000 rpm 2 min zentrifugiert, in deren Anschluss 150 μL vom Überstand verworfen wurde. Die Zellen wurden mit dem restlichen Überstand resuspendiert und auf LB-Agarplatten mit dem entsprechenden Antibiotikum ausplattiert. Danach wurden die Platten bei 37°C über Nacht inkubiert.

Sollte eine Kotransformation von zwei verschiedenen Plasmiden erfolgen, wie dies zur Erstellung des Stammes notwendig ist, der die Plasmide pCOLA(mod) 3α-HSDH und pET21a(+) FDH 7β-HSDH enthält, wurden 50 μL chemisch-kompetenter *E. coli* BL21 (DE3) hdhA$^-$ KanR$^+$ mit jeweils 2 μL der einzubringenden Plasmide versetzt. Nach der Transformation wurden die Zellen in Vorkulturröhrchen mit 5 mL LB-Medium mit dem entsprechenden Antibiotikum gegeben und bei 37°C auf dem Vorkulturenschüttler 16-48 h bei 200 rpm inkubiert, bis sich ein Bakterienwachstum im Vorkulturröhrchen erkennen ließ. Daraufhin wurden 5 μL der Bakteriensuspension auf LB-Agarplatten mit dem entsprechenden Antibiotikum ausplattiert und bei 37°C über Nacht inkubiert.

**7.5 Kultivierung des Stamms im Schüttelkolben:**

Zur Expression rekombinanter Proteine wurde der entsprechende *E. coli* BL21 (DE3) hdhA- KanR+ beinhaltend die beiden Expressionsplasmide, in 5 mL LB-Medium mit Zugabe des entsprechenden Antibiotikums über Nacht bei 37°C und 200 rpm inkubiert. Anschließend wurden 200 mL TB-Medium mit Zugabe des entsprechenden Antibiotikums mit dieser Vorkultur inokuliert und bei 37°C, 250 rpm inkubiert. Bei Erreichen einer OD600 von 0,6-0,8 wurde die Expression des rekombinanten Proteins mit 1 mM Isopropyl-β-D-thiogalactopyranosid (IPTG) induziert und die Kultur bei 25°C, 160 rpm für weitere 21 h inkubiert.

**7.6 Durchführung der Ganzzellbiotransformation und Versuchsergebnis**

Die Reaktionen wurden in 2 mL Reaktionsvolumen bei pH 6,0 in Suspension durchgeführt, wobei eine Zelldichte von $OD_{600}$ = 20 eingesetzt wurde. Als Substratkonzentration wurden 25 mM bzw. 50 mM Dehydrocholsäure gewählt, die eingesetzte Kosubstratkonzentration betrug 400 mM. Die Versuche erfolgten unter Luftabschluss bei 25°C. Proben wurden nach 48 h genommen.

Bei dem Reaktionsansatz mit 25 mM ist kein Substrat mehr nachweisbar, während bei dem Reaktionsansatz mit 50 mM Dehydrocholsäure zwar ein Substrat-Peak detektierbar ist, dessen Peakfläche aber unterhalb der Grenze von 1 µg/mL Substrat in der Probe ist, für die die HPLC-Methode kalibriert ist. Gleiches gilt für die Peaks der 3,12-diketo-Ursodesoxycholsäure. Der größte Peak in den Chromatogrammen ist der des Produktes 12-keto- Ursodesoxycholsäure.

Es kann also überraschend gezeigt werden, dass eine zweistufige Ganzzellreduktion von Dehydrocholsäure zu 3-Keto-Ursodesoxycholsäure möglich ist.

Die Chromatogramme der HPLC-Messung sind in Figur 7 dargestellt.

Die HPLC-Analyse wurde wie folgt durchgeführt: Als Chromatographiesäule wurde die Umkehrphasen-Chromatographiesäule Hi-bar Purospher 125-4 RP-18e (5 µm) der Firma Merck, Darmstadt verwendet. Hierbei dient eine unpolare Phase als stationäre Phase, während eine polare Phase die mobile Phase bildet. Für die HPLC-Analytik wurde das Verfahren der Gradientenelution verwendet. Dabei wird dem Eluent phosphorsaures Wasser (pH 2,6) ein steigender Anteil an Acetonitril zugesetzt, dabei bedingt die Ansäuerung des Laufmittels einen einheitlichen Protonierungsgrad der zu untersuchenden Analyten. Nach Elution sämtlicher Komponenten wird die Chromatographiesäule mit dem Ausgangsverhältnis beider Lösungsmittel äquilibriert. Die Gradientenelution wurde durchgeführt, indem zunächst für 3 min ein Laufmittelgemisch der konstanten Zusammensetzung von 65 % (v/v) phosphorsaurem Wasser und 35 % (v/v) Acetonitril durch die HPLC-Säure gepumpt wurde. Von Minute 3 bis 7,5 wir der Anteil an Acetonitril linear auf 39 % (v/v) erhöht. Zwischen Minute 7,5 und 10 erfolgt eine weitere lineare Erhöhung des Acetonitrilanteils auf 40 % (v/v). Zur Elution aller Probenkomponenten wird der Acetonitrilanteil zwischen Minute 10 und 11 ebenfalls linear auf 70 % (v/v) erhöht und weitere zwei Minuten lang auf diesem Wert konstant gehalten. Danach wird der Acetonitrilanteil von Minute 13 bis 15 wieder auf den Ausgangswert von 35 % reduziert und die Säule mit dieser Laufmittelzusammensetzung 3 min lang äquilibriert. Der Fluss wird über die gesamte Dauer auf 1,000 mL/min eingestellt. Eluierte Analyten werden durch einen UV-Detektor bei einer Wellenlänge 200 nm detektiert.

**Umsetzungsbeispiel 8: Ganzzellbiotransformation von DHCS zu 12-Keto-UDCS durch Coexpression von FDH D221G,** 7ß-HSDH **und 3α-HSDH im Einplasmid-System**

Es soll untersucht werden, ob eine zweistufige Ganzzellreduktion von Dehydrocholsäure zu 12-keto-Ursodesoxycholsäure in einem zellulären Einplasmid-System möglich ist.

8.1 Verwendete Plasmide:

| Bezeichnung | Sequenz | SEQ ID NO |
|---|---|---|
| 3alpha_fwd_HindIII | 5'-CCCAAGCTTAAGGAGATATACATGTCCATCATCGTGATAAGCG-3' | 16 |
| 3alpha_rev_NotI | 5'-ATAAGAATGCGGCCGCTCAGAACTGTGTCGG GCG-3' | 17 |
| 7beta_mut_G39A_fwd | 5'-CGTCGTCATGGTCGCCCGTCGCGAGG-3' | 9 |
| 7beta_mut_G39A_rev | 5'-CCTCGCGACGGGCGACCATGACGACG-3' | 10 |

Restriktionsschnittstellen sind unterstrichen, ribosomale Bindestellen sind grau hinterlegt.

**8.2 Herstellung des Vektors:**

Die Herstellung des Plasmidkonstrukts pET21a(+) FDH 7β-HSDH(G39A) 3α-HSDH (Figur 8) erfolgte wie folgt: Ausgehend vom Plasmid pET21a(+) FDH 7β-HSDH wurde die Wildtyp-3α-HSDH (C. testosteroni; SEQ ID NO: 5,6) über die Schnittstellen HindIII und NotI hinter die 7β-HSDH einkloniert. Zur Amplifizierung der 3α-HSDH wurden die Primer 3alpha finid_HindIII und 3alpha_rev_NotI verwendet, als Templat diente hierzu das Plasmid pET22b(+) 3α-HSDH. Anschließend wurde die G39A-Muation über positionsgerichtete Mutagenese nach dem QuikChange-Protokoll in 7β-HSDH eingeführt. Dabei wurden die Mutageneseprimer 7beta_mut_ G39A_fwd und 7beta_mut_G39A_rev verwendet.

**8.3 Umsetzung:**

Das erstellte Plasmid wurde in E. coli BL21 (DE3) hdhA$^-$ KanR$^+$ transformiert. Mit dem dadurch erzeugtem Stamm *E. coli* BL21 (DE3) hdhA$^-$ KanR$^+$ pET21a(+) FDH 7β-HSDH(G39A) 3α-HSDH wurden Ganzzellumsätze unter folgenden Bedingungen im 150 mL-Maßstab durchgeführt: Zelldichte OD 30, 50 mM DHCS, 750 mM Natriumformiat, suspendiert mit 50 mM Kaliumphosphatpuffer (pH 6,5) als Zell- und Substratsuspension. Die Reaktionen wurden 3,5 h bei 25°C durchgeführt. Die Ergebnisse der HPLC Analyse (Durchführung siehe Umsetzungsbeispiel 7, oben) sind in Figur 9 dargestellt.

**Herstellungsbeispiel 6: Herstellung weiterer 7β-HSDS Mutanten und deren Charakterisierung (nicht erfindungsgemäß)**

Eine Möglichkeit zur Generierung einer NADH-spezifischen 7β-HSDH besteht in der Modifikation des verfügbaren Enzyms durch verschiedene Mutageneseverfahren. Daher sollte mittels positionsgerichteter Mutagenese einzelne Aminosäuren der 7β-HSDH durch andere substituiert werden, welche die Änderung der Kofaktorspezifität der 7β-HSDH von NADPH zu NADH bedingen, um NADP vorteilhaft durch das kostengünstigere NAD ersetzen zu können.

**6.1 Primer**

Insgesamt wurden die 7β-HSDH Mutanten G39D, G39D/R40L, G39D/R40I und G39D/R40V erzeugt. Die G39D-Mutante wird durch Quickchange-Mutagenese erzeugt, die anderen Mutanten nach der PCR-Methode von Sanchis et al. (Sanchis J, Fernändez L, Carballeira JD, Drone J, Gumulya Y, Höbenreich H, Kahakeaw D, Kille S, Lohmer R, Peyralans JJ, Podtetenieff J, Prasad S, Soni P, Taglieber A, Wu S, Zilly FE, Reetz MT. Improved PCR method for the creation of saturation mutagenesis libraries in directed evolution: application to difficult-to-amplify templates. Appl Microbiol Biotechnol. 2008 Nov;81(2):387-97). Ein Sequenzvergleich des Wildtyps und der erzeugten Mutanten ist in Figur 10 gezeigt. Folgende Primer wurden für die Mutagenesen verwendet, wobei die kursiv dargestellten Basen jeweils für die ausgetauschte Aminosäure kodieren:

**G39D**

7beta mut G39D fwd (SEQ ID NO:41):CGTCGTCATGGTCGACCGTCGCGAGG

7beta mut G39D rev (SEQ ID NO:42): CCTCGCGACG*GTC*GACCATGACGACG

**G39D R40L**

7beta mut G39D R40L fwd (SEQ ID NO:43): CGTCGTCATGGTC*GACCT*GCGCGAGG

3alphamut AntiMid_rev (SEQ ID NO:44): CCGCCGCATCCATACCGCCAGTTGTTTACCC

**G39D R40I**

7beta mut G39D R40I fwd (SEQ ID NO:45): CGTCGTCATGGTC*GACATT*CGCGAGG

3alphamut AntiMid_rev (SEQ ID NO:44): CCGCCGCATCCATACCGCCAGTTGTTTACCC

**G39D R40V**

7beta mut G39D R40V fwd (SEQ ID NO:46): CGTCGTCATGGTC*GACGTT*CGCGAGG
3alphamut AntiMid_rev (SEQ ID NO:44): CCGCCGCATCCATACCGCCAGTTGTTTACCC

### 6.2 Enzymkinetische Untersuchung der 7β-HSDH Mutanten

Die Bewertung der erstellten Mutanten erfolgt mittels enzymkinetischer Untersuchungen, deren Ergebnisse in Figur 11 grafisch dargestellt sind. Hierbei werden für die Untersuchung der unmodifizierten 7β-HSDH 0,1 mM NADPH als Kofaktor eingesetzt, für die Untersuchung der 7β-HSDH-Mutanten hingegen 0,5 mM NADH. Die Notwendigkeit zur Erhöhung der Kofaktorkonzentration bei der enzymkinetischen Untersuchung der 7β-HSDH Mutanten sind durch die bei den Mutanten erhöhten Halbsättigungskonzentrationen für den Kofaktor NADH bedingt. Aus den Auftragungen der Substratkonzentration gegen die spezifische Enzymaktivität lassen sich für die 7β-HSDH Mutanten die charakteristischen Kurvenverläufe der Michaelis- Menten-Kinetik erkennen, während sich aus der Auftragung für die unmodifiziert Wildtyp 7β-HSDH der Kurvenverlauf einer Michaelis-Menten-Kinetik mit Substratinhibierung erkennen lässt. Dementsprechend wurde das klassische Michaelis-Menten-Modell (Gleichung 2) für die Auswertung der Messreihen der 7β- HSDH Mutanten und das Michaelis-Menten-Modell mit Substratinhibierung für die Messreihe des unmodifizierten Wildtyp 7β-HSDH verwendet (Gleichung 2).

Gleichung 1 (Michaelis-Menten-Gleichung)

$$EA_X = v_{\max} \cdot \frac{c_s}{K_m + c_s}$$

EA$_x$: spezifische Enzymaktivität, U mg$^{-1}$= μmol min $^{-1}$ mg$^{-1}$
$v_{max}$: maximale spezifische Enzymaktivität, U mg$^{-1}$= μmol min $^{-1}$ mg$^{-1}$
$c_S$: Substrat- bzw. Kofaktorkonzentration, mol L$^{-1}$
$K_m$: Halbsättigungskonzentration, mol L$^{-1}$

Gleichung 2 (Michaelis-Menten-Gleichung mit Substratinhibierung)

$$EA_X = v_{\max} \cdot \frac{c_s}{K_m + (1 + \frac{c_s}{K_i})c_s}$$

$EA_x$: spezifische Enzymaktivität, U mg-'= μmol min $^{-1}$ mg$^{-1}$

$v_{max}$: maximale spezifische Enzymaktivität, U mg-'= μmol min $^{-1}$ mg$^{-1}$

$c_S$: Substrat- bzw. Kofaktorkonzentration, mol L$^{-1}$

$K_m$: Halbsättigungskonzentration, mol L$^{-1}$

$K_i$: Inhibitionskonstante, mol L$^{-1}$

In der nachfolgenden Tabelle sind enzymkinetische Parameter der unmodifizierten Wildtyp 7β-HSDH und deren Mutanten mit veränderter Kofaktorspezifität zusammengefasst. Für den Wildtyp wird NADPH als Kofaktor eingesetzt, während für die Mutanten NADH als Kofaktor eingesetzt wird.

| | $K_m$, DHCA, μM | $K_i$, DHCA, μM | Vmax, U mg-1 |
|---|---|---|---|
| **Wildtyp** | 31 ± 5 | 8600 ± 1500 | 14,6±1,0 |
| **G39D** | 660 ±120 | n. b. | 2,90±0,16 |
| **G39D R40I** | 920 ±170 | n. b. | 4,64±0,27 |
| **G39D R40V** | 880 ±120 | n. b. | 2,69±0,12 |
| **G39D R40L** | 560 ± 80 | n. b. | 1,60±0,07 |

**Herstellungsbeispiel 7: Herstellung weiterer 7β-HSDS Mutanten und deren Charakterisierung (nicht erfindungsgemäß)**

In einem zum Herstellungsbeispiel 6 parallelen Ansatz wurden nochmals 7β-HSDS-Mutanten hergestellt.

**7.1 Primer**

Für die positionsgerichtete Mutagenese der 7ß-HSDH wurden die unten angegeben Mutageneseprimer verwendet. Die Primer wurden auf Grundlage der 7ß-HSDH-Gensequenz dahingehend ausgewählt, dass sie den gewünschten Aminosäurenaustausch bewirken. Es wurde darauf geachtet, dass die zu mutierende Base im Primer mittig lokalisiert ist, und dass die Schmelztemperaturen der Primerpaare sich im gleichen Bereich befinden.
Zur Erzeugung der Mutanten wurden folgende Primerpaare verwendet:

**Tabelle:** Verwendete Primer für die positionsgerichtete Mutagenese der 7ß-HSDH

| ***Bezeichnung*** | ***Position*** | ***Primer*** | ***5' -> 3' Sequenz*** |
|---|---|---|---|
| G39D_for | G39D | forward | GTCGTCATGGTC*GA*CCGTCGCGAGGAG |
| G39D_rev | | reverse | CTCCTCGCGACG*GTC*GACCATGACGAC |
| G39D/R40I_for | G39D/R40I | forward | GTCATGGTC*GACATT*CGCGAGGAG |
| G39D/R40I_rev | | reverse | CTCCTCGCG*AATGTC*GACCATGAC |
| R40D_for | R40I | forward | GTCATGGTCGGC*GAT*CGCGAGGAGAAG |
| R40D_rev | | reverse | CTTCTCCTCGCG*ATC*GCCGACCATGAC |
| R40D/R411_for | R40D/R41I | forward | GTCATGGTCGGC*GATATC*GAGGAGAAGCTG |
| R40D/R41I_rev | | reverse | CAGCTTCTCCTC*GATATC*GCCGACCATGAC |
| DIN_for | G39D/R40I/R41N | forward | ATGGTC*GACATTAAC*GAGGAGAAGCTG |
| DIN_rev | | reverse | CAGCTT*CTCCTCGTT*AATGTCGACCAT |

**7.2 PCR-Programm**

Bei der Reaktion erfolgte zuerst ein 2 min langer initialer Denaturierungsschritt bei 98°C. Danach wurden 23 Zyklen von Denaturierung (30 s bei 98°C), Primerhybridisierung (1 min bei 60 - 68°C) und Elongation (3,5 min bei 72°C) durchgeführt. Als letzter Schritt erfolgte eine finale Elongation von 10 min bei 72°C bevor die Polymerasekettenreaktion durch Kühlen auf 4°C beendet wurde.

**7.3 PCR-Ansatz**

**Tabelle:** PCR-Ansatz für die Erzeugung der verschiedenen 7ß-HSDH Varianten

| | |
|---|---|
| HF-Puffer (5x) | 10 μl |
| dNTP-Mix (10 mM) | 1,0 μl |
| *Forward*-Primer (10 pmol/μl) | 5 μl |
| *Revers*-Primer (10 pmol/μl) | 5 μl |
| Templat | 1,5 μl |
| *Phusion* Polymerase | 0,5 μl |
| DMSO | 2,5 μl |
| $ddH_2O$ | 24,5 μl |
| | **50 μl** |

Als Templat wurde ein pET21a-Vektor mit der 7β-HSDH(Wildtyp) verwendet.

### 7.4 Durchführung

Um Aminosäuren in Proteinsequenzen gezielt austauschen zu können, wird die DNA-Sequenz des entsprechenden Gens positionsgerichtet mutiert. Hierzu werden zueinander komplementäre Primer genutzt, die die gewünschte Mutation in ihrer Sequenz tragen. Als Templat dient N6-adeninmethylierte, doppelsträngige Plasmid-DNA, die das zu mutierende Gen tragen. N6-adeninmethylierte Plasmid-DNA werden aus dam$^+$ *E. coli*-Stamm wie zum Beispiel *E. coli* DH5 isoliert.

Die Polymerasekettenreaktion wird wie oben beschrieben durchgeführt. Dabei werden die Primer komplementär zum Templat verlängert, wodurch Plasmide mit der gewünschten Mutation entstehen, die einen Strangbruch aufweisen. Anders als bei anderen PCR-Reaktionen steigt die DNA-Ausbeute hierbei nur linear, da neu gebildete DNA-Moleküle nicht als Templat für die PCR-Reaktion dienen können.

Nach Abschluss der PCR-Reaktion wurde das PCR-Produkt mittels PCR-Purification-Kit (Analytik Jena) aufgereinigt und die parentale, N6-adeninmethylierte mit Hilfe des Restriktionsenzyms *DpnI* verdaut. Dieses Enzym hat die Besonderheit, dass es unspezifisch N6-adeninmethylierte DNA restringiert, jedoch nicht die neu gebildete, nichtmethylierte DNA. Die Restriktion erfolgte, indem 1 μL *DpnI* zum PCR-Reaktionsansatz hinzu gegeben und für 2 h oder über Nacht bei 37°C inkubiert wurde.

5 μl dieses Ansatzes wurden zur Transformation von 100 μl chemisch-kompetenten DH5α-Zellen verwendet. Nach Plasmidisolation wurde die erfolgreiche Mutation mittels Sequenzierung bestätigt.

### 7.5 Charakterisierung

Die Mutation wurde über positionsgerichtete Mutagenese nach dem QuikChange-Protokoll in die 7β-HSDH eingeführt. Dabei wurden die Mutageneseprimer aus der im Kapitel 7.1 gezeigten Tabelle verwendet.

Mittels der im Kapitel "Methoden" genannten Assays für die photometrische Aktivitätmessung wurde die Aktivität der mutierten Enzyme in Gegewart von NADPH beziehungsweise NADH gemessen. Die ermittelten Aktivitäte sind in der nachfolgenden Tabelle zusammengefasst.

**Tabelle:** ermittelte Aktivität der verschiedenen 7β-HSDH-Varianten.

| **erzeugte Mutante** | *Volumetrische Aktivität* ***[U/ml]*** | | *Spezifische Aktivität* ***[U/mg]*** | |
|---|---|---|---|---|
| | NADPH | NADH | NADPH | NADH |
| G39D | 119 | 2 | 5,1 | 0,1 |
| G39D/R40I | 0 | 21 | 0 | 0,8 |
| R40D | 0 | 3 | 0 | 0,1 |
| R40D/R41I | 0 | 3 | 0 | 0,2 |
| G39D/R40I/R41N | 0 | 6 | 0 | 0,3 |
| 7ß-HSDH (WT) | 134 | 0 | 5,6 | 0 |

### Umsetzungsbeispiel 9: Einsatz von NADH abhängiger 7β-HSDH in der Ganzzellreduktion von Dehydrocholsäure zu 3,12-diketo-UDCA

Nachfolgend soll der Einsatz NADH-abhängiger 7β-HSDH, hergestellt im Herstellungsbeispiel 6. in der ganzzellbiokatalytischen Umsetzung von DHCA zu 3,12-diketo-UDCA demonstriert werden. Zur besseren Verständlichkeit ist eine Übersicht möglicher Umsetzungswege und Reaktionsprodukte in Figur 12 gezeigt.

Im vorliegenden Fall wurden die 7β-HSDH-Mutanten zusammen mit einer NADH-abhängigen Formiatdehydrogenase aus *Mycobacterium vaccae* in einen Expressionsvektor eingefügt. Hierbei trägt der Vektor, in den 7β-HSDH (G39D) eingefügt ist, die Bezeichnung pFr7(D), entsprechend SEQ ID NO:49; der Vektor, in den 7β-HSDH (G39D R40L) eingefügt ist, die Bezeichnung pFr7(DL), entsprechend SEQ ID NO:50; der Vektor, in den 7β-HSDH (G39D R40I) eingefügt ist, die Bezeichnung pFr7(DI), entsprechend SEQ ID NO:51, und der Vektor, in den 7β-HSDH (G39D R40V) eingefügt ist, die Bezeichnung pFr7(DV), entsprechend SEQ ID NO:52. Eine allgemeine Plasmidkarte dieser Vektoren ist in Figur 13 gezeigt.

Diese Vektoren wurden in den Stamm *E. coli* BL49 (identisch mit E.coli BL21(DE3) hdhA$^-$ KanR$^+$ ) transformiert. Von diesen Stämmen wurden zunächst 5 mL Übernachtkulturen in LB-Medium mit 100 mg $L^{-1}$ Ampicillin angezüchtet.

Jeweils 1 mL dieser Übernachtkulturen wurden am Folgetag in 200 mL TB-Medium in Schüttelkolben mit 100 mg $L^{-1}$ Ampicillin transferiert. Diese Kulturen wurden bei 37 °C und 250 rpm im Schüttelinkubator kultiviert, bis eine OD von 0,6 - 0,8 erreicht wurde. Anschließend erfolgte die Induktion durch 1 mM Isopropyl-β-D-thiogalactopyranosid (IPTG). Die anschließende Expressionsphase wurde 21 h lang bei 25 °C und 160 rpm durchgeführt. Die Zellernte erfolgte durch 10-minütige Zentrifugation bei 3220 *g*.

Mit den auf diese Weise hergestellten Zellen wurden Ganzzellbiotransformationsreaktionen angesetzt. Die Konzentrationen des Substrats DHCA, und des Produkts 3,12-diketo-UDCA im Reaktionsansatz zu verschiedenen Zeitpunkten wurde durch Hochleistungsflüssigkeitschromatographie (HPLC) ermittelt.

Die Reaktionsansätze für die Biotransformationen enthielten 17,7 g $L^{-1}$BTM Ganzzellbiokatalysatoren, 100 mM Substrat (DHCA), 500 mM Natriumformiat und sind in 50 mM Kaliumformiatpuffer (pH 6,5) suspendiert. Der Prozess lief im 20 mL Maßstab als Satzverfahren ohne pH-Kontrolle ab. Die Konzentrationen an Produkt und Substrat nach 24 h sind in Figur 14 dargestellt. Es ist zu entnehmen, dass sämtliche Mutanten für die ganzzellbiokatalytische Umsetzung von DHCA zu 3,12-diketo-UDCA geeignet sind.

**Umsetzungsbeispiel 10: Einsatz von NADH abhängiger 7ß-HSDH in der zweistufigen Ganzzellreduktion von Dehydrocholsäure zu 12-keto-UDCA**

Nachfolgend soll der Einsatz NADH-abhängiger 7β-HSDH in der zweistufigen ganzzellbiokatalytischen Umsetzung von DHCA zu 12-diketo-UDCA demonstriert werden. Die Mutante 7β-HSDH (G39D) wurden hierbei zusammen mit einer NADH-abhängigen Formiatdehydrogenase aus *Mycobacterium vaccae* und einer NADH-abhängigen 3α-HSDH aus *Comamonas testosteroni* in einen Expressionsvektor eingefügt. Hierbei ist die Verwendung von 7β-HSDH (G39D) exemplarisch für sämtliche NADH-abhängigen 7β-HSDH gezeigt. Der Vektor trägt die Bezeichnung pFr3T7(D) und ist schematisch in Figur 15 dargestellt.

Diese Vektoren wurden in den Stamm *E. coli* BL49 (identisch mit E.coli BL21(DE3) $hdhA^-$ $KanR^+$) transformiert, der entstehende Stamm trägt die Bezeichnung *E. coli* BL49 pFr3T7(D). Dieser Stamm wurde im 7 L-Bioreaktor bei einem Arbeitsvolumen von 4 L im definierten Minimalmedium kultiviert. Nach einer kurzen Satzphase bei 37 °C erfolgte eine substratlimitierte exponentielle Wachstumsphase bei 30 °C. Bei Erreichen einer Optischen Dichte von 25 - 30 wurde die Proteinexpression der Zellen durch Zugabe von 0,5 mM IPTG induziert. Die Expression erfolgte danach für 24 h bei 20 °C. Die Zellen (32,0 g L-1BTM) wurden durch Zentrifugation geerntet, mit Kaliumphosphatpuffer (pH 6,5) resuspendiert und nach Standardprotokoll bei -20 °C gelagert.

Die Biotransformation wurde im 20 mL-Maßstab unter folgenden Reaktionsbedingungen angesetzt: 100 mM DHCA, 17,7 g $L\text{-}1_{BTM}$ des gelagerten Biokatalysators, 500 mM Ammoniumformiat, 26 % Glycerin, 50 mM $MgCl_2$, suspendiert in 50 mM KPi-Puffer (pH 6,5). Durch manuelle pH-Einstellung wurde der pH während der ersten 5 Stunden des Biotransformationsprozesses auf pH 6,5 gehalten. Der zeitliche Verlauf der Gallensalzkonzentrationen ist in Figur 16 dargestellt. Nach 24 h konnten 92 % des Produkts 12-keto-UDCA gebildet werden. Die Umsetzung von DHCA zu 3,12-diketo-UDCA und die Umsetzung von 7,12-diketo-UDCA zu 12-keto-UDCA zeigt, dass sämtliche in Figur 12 dargestellten Umsetzungen der 7β-HSDH tatsächlich durch 7β-HSDH (G39D) katalysiert werden.

**Umsetzungsbeispiel 11: Zweistufige Reduktion von Dehydrocholsäure in einem Einzell-System**

Ein Syntheseweg für Ursodesoycholsäure beinhaltet die chemische Oxidation von Cholsäure zu Dehydrocholsäure mit den anschließenden asymmetrischen enzymatischen Reduktionen der 3-Carbonylgruppe zur 3α-Hydroxygruppe und der 7-Carbonylgruppe zur 7β-Hydroxygruppe gefolgt von der chemischen Entfernung der 12-Carbonylgruppe.

Für diesen Syntheseweg werden zur Katalyse der enzymatischen Reduktionsreaktionen die Enzyme 7ß-Hydroxysteroiddehydrogenase (7β-HSDH) und 3α-Hydroxysteroiddehydrogenase (3α-HSDH) benötigt. Bei der Umsetzung werden zudem Kofaktoren (NADH bzw. NADPH) stöchiometrisch verbraucht, die in einem wirtschaftlichen Prozess regeneriert werden müssen. Hierzu werden häufig enzymatische Regenerierungsmöglichkeiten bevorzugt. Geeignete Enzyme sind unter anderem Formiatdehydrogenasen (FDH), Glucosedehydrogenasen (GDH), Glucose-6-Phosphat-Dehydrogenasen (G6PDH), Alkoholdehydrogenasen (ADH) oder Phosphitdehydrogenasen (PtDH).

Anders als beim Einsatz von isolierten Enzymen ist es bei einer Ganzzellbiokatalyse, bei der erforderliche Enzyme innerhalb eines Wirtsorganismus, typischerweise einem einzelligen Mikroorganismus, nicht möglich, einzelne Enzyme in individuellen Mengen zum Reaktionsmedium zuzugeben. Hierbei muss die Ausbalancierung der Enzymaktivitäten durch Modifikation der Expressionsstärken aller beteiligten Enzyme entweder durch Kultivierungsmethoden oder durch gentechnische Modifikation des Ganzzellbiokatalysators erfolgen.

Im vorliegenden Beispiel wurden erfindungsgemäß Vektoren verwendet, die sämtliche drei Gene für 7β-HSDH, 3α-HSDH und FDH enthalten. Die Vektoren sind so konstruiert, dass die Expressionsstärke der drei Gene im designierten Wirtsstamm, insbesondere auf *Escherichia coli* BL21(DE3) basierende Ganzzellbiokatalysatorstämmen, in der Form

eingestellt sind, dass alle drei Enzyme möglichst ähnliche Enzymaktivitäten aufweisen.

Die drei an der Ganzzellreduktion von Dehydrocholsäure zu 12-keto-Ursodesoxycholsäure benötigten Gene liegen auf ein und demselben Vektor. Es handelt sich um Gene, die für folgende Enzyme kodieren: eine NADPH-abhängige 7β-HSDH aus *Collinella aerofaciens,* eine NADH-abhängige 3α-HSDH aus *Comamonas testosteroni,* eine sowohl NAD- als auch NADP-abhängige, mutierte FDH aus *Mycobacterium vaccae.* Die Expressionsstärken dieser drei Gene ist durch das genetische Konstrukt dahingehend ausbalanciert, dass eine möglichst optimale Ganzzellbiokatalyse durchführbar ist. Im vorliegenden Beispiel wurden die 7β-HSDH-Mutanten G39A und G39S statt eines unmodifizierten Wildtyp-Enzyms in einen Vektor für die Ganzzellbiokatalyse eingesetzt. Die Vektoren tragen die Bezeichnungen pF(G)r7(A)r3 und pF(G)r7(S)r3 und sind in den Figuren 17a und b dargestellt. Figur 18 zeigt eine schematische Darstellung des möglicher Umsetzungswege und Reaktionsprodukte.

Diese erfindungsgemäßen Vektoren wurden in Escherichia coli-Produktionsstämme transformiert. Diese Stämme stellen den Ganzzellbiokatalysator dar. Bei dem verwendeten Wirtstamm handelt es sich um einen modifizierten E. coli BL21(DE3) mit der Bezeichnung E. coli BL49. Allerdings ist der Wirtsorganismus nicht auf diesen Wirtsstamm beschränkt. Der mit pF(G)r7(A)r3 transformierte E. coli BL49 trägt die Bezeichnung E. coli BL49 pF(G)r7(A)r3, der mit pF(G)r7(S)r3 transformierte E. coli BL49 trägt die Bezeichnung E. coli BL49 pF(G)r7(S)r3.

### 11.1 Zweistufige Ganzzellreduktion von Dehydrocholsäure im 20 mL-Maßstab

Der Vergleich der Biokatalysatoren erfolgte durch Ganzzellbiotransformation im 20 mL-Maßstab. Hierzu wurde zunächst eine 5 mL Übernachtkultur in LB-Medium mit 100 mg L-1 Ampicillin angezüchtet. 1 mL dieser Übernachtkultur wurde am Folgetag in 200 mL TB-Medium in Schüttelkolben mit 100 mg L-1 Ampicillin transferiert. Diese Kulturen wurden bei 37 °C und 250 rpm im Schüttelinkubator kultiviert, bis eine OD von 0,6 - 0,8 erreicht wurde. Anschließend erfolgte die Induktion durch 1 mM Isopropyl-β-D-thiogalactopyranosid (IPTG). Die anschließende Expressionsphase wurde 21 h lang bei 25 °C und 160 rpm durchgeführt. Die Zellernte erfolgte durch 10-minütige Zentrifugation bei 3220 g.

Mit den auf diese Weise hergestellten Zellen wurden Ganzzellbiotransformationsreaktionen angesetzt. Maßgeblich für die Beurteilung der Zellen war die Menge des gebildeten Produktes (12-keto-UDCA) im Reaktionsansatz. Die Konzentrationen des Substrats DHCA, der Intermediate 3,12-diketo-UDCA und 7,12-diketo-UDCA sowie des Produkts 12-keto-UDCA im Reaktionsansatz wurden durch Hochleistungsflüssigkeitschromatographie (HPLC) ermittelt.

Die Reaktionsansätze für die Biotransformationen enthielten 11,8 g L-1BTM Ganzzellbiokatalysatoren, 100 mM Substrat (DHCA), 500 mM Natriumformiat und waren in 50 mM Kaliumformiatpuffer (pH 6,5) suspendiert. Der Prozess lief im 20 mL Maßstab als Satzverfahren ohne pH-Kontrolle ab. In der nachfolgenden Tabelle sind Ergebnisse der oben beschriebenen Ganzzellbiotransformation nach 5 h Prozess dargestellt. Hier sind beide neuen Stämme *E. coli* BL49 pF(G)r7(S)r3 und *E. coli* BL49 pF(G)r7(A)r3 einem Referenzstamm *E. coli* BL49 pF(G)r7 pC3 gegenübergestellt. Während beim Referenzstamm *E. coli* BL49 pF(G)r7 pC3 lediglich 5,7 ± 1,0 % Produkt (12-keto-UDCA) gebildet wurden, konnte mit den neuen Biokatalysatoren beim Stamm *E. coli* BL49 pF(G)r7(S)r3 38,4 ± 8,2 % 12-keto-UDCA und beim Stamm *E. coli* BL49 pF(G)r7(A)r3 47.7 ± 2,1 % 12-keto-UDCA gebildet werden. Damit wurde die Produktkonzentration im Reaktionsansatz gegenüber dem Referenzstamm auf das 6,7-fache (*E. coli* BL49 pF(G)r7(S)r3) beziehungsweise auf das 8,4-fache (*E. coli* BL49 pF(G)r7(A)r3) gesteigert.

Die folgende Tabelle zeigt dementsprechend Gallensalzanteile von Biotransformationsansätzen nach 5 h bei Einsatz von 100 mM Substrat (DHCA). Gezeigt sind die Ansätze mit den zwei neuen Biokatalysatorstämmen *E. coli* BL49 pF(G)r7(A)r3 und *E. coli* BL49 pF(G)r7(S)r3 im Vergleich mit dem Stamm nach dem bisherigen Stand der Technik *E. coli* BL49 pF(G)r7 pC3:

| | Gallensalzanteile, % | | | |
|---|---|---|---|---|
| | 12-keto-UDCA | 3,12-diketo-UDCA | 7,12-diketo-UDCA | DHCA |
| E. coli BL49 pF(G)r7 pC3 | 5,7 ± 1,0 % | 9,2 ± 0,2 % | 40,5 ± 1,9 % | 44,6 ± 3,1 % |
| E. coli BL49 pF(G)r7(A)r3 | 47.7 ± 2,1 % | 2,4 ± 0,5 % | 48,9 ± 1,6 % | 1,0 ± 0,0% |
| E. coli BL49 pF(G)r7(S)r3 | 38,4 ± 8,2 % | 2,7 ± 2,7 % | 57,4 ± 4,4 % | 1,5 ± 1,8 % |

### 11.2 Zweistufige Ganzzellreduktion von Dehydrocholsäure im 1 L-Maßstab

Durch Prozesskontrolle wurde die Produktbildung mit dem Biokatalysator *E. coli* BL49 pF(G)r7(A)r3 gegenüber

dem Millilitermaßstab gesteigert. Hierzu wurde der Biokatalysator im 7 L-Bioreaktor bei einem Arbeitsvolumen von 4 L im definierten Minimalmedium kultiviert. Nach einer kurzen Satzphase bei 37 °C erfolgte eine substratlimitierte exponentielle Wachstumsphase bei 30 °C. Bei Erreichen einer optischen Dichte von 25 - 30 wurde die Proteinexpression der Zellen durch Zugabe von 0,5 mM IPTG induziert. Die Expression erfolgte danach für 10 h bei 25 °C, bevor die Zellen durch Zentrifugation geerntet und bei -20 °C gelagert wurden.

Die Biotransformation wurde in einem 1 L-Bioreaktor unter folgenden Reaktionsbedingungen angesetzt: 70 mM DHCA, 17,7 g L-1BTM des gelagerten Biokatalysators, 500 mM Natriumformiat, 26 % (v/v) Glycerin, 50 mM MgCl2, suspendiert in 50 mM KPi-Puffer (pH 6,5). Durch pH-Regelung wurde der pH während der gesamten Biotransformation auf pH 6,5 gehalten. Die zeitlichen Verläufe der Gallensalzkonzentrationen sind in Figur 19 dargestellt. Nach 21 h konnten 99,4 % Produkt (12-keto-UDCA) gebildet werden und lediglich 0,6 % des Nebenprodukts 3,12-diketo-UDCA waren nachweisbar.

**Umsetzungsbeispiel 12: Zweistufige Reduktion von Dehydrocholsäure in einem Einzell-System unter Verwendung einer Glukose-Dehydrogenase zur Kofaktorregenerierung**

In diesem Beispiel wird in einem Ganzzellbiokatalysator (Einzell-System) für die zweistufige Reduktion von DHCA zu 12-keto-UDCA neben einer NADPH-abhängigen 7β-HSDH aus *Collinella aerofaciens* (Mutante G39A) und einer NADH-abhängigen 3α-HSDH aus *Comamonas testosteroni* eine sowohl NAD- als auch NADP-abhängige GDH aus *Bacillus subtilis* für die Kofaktorregenerierung exprimiert. Die Nukleinsäuresequenz und die zugehörige Aminosäuresequenz dieser GDH sind als SEQ ID NO:47 beziehungsweise SEQ ID NO:48 angegeben.

Die dafür verwendeten Vektoren tragen die Bezeichnungen p3T7(A)rG und p7(A)T3rG und sind in Figur 20 beziehungsweise Figur 21 dargestellt.

Die erfindungsgemäßen Vektoren wurden in *Escherichia coli*-Produktionsstämme transformiert. Diese Stämme stellen den Ganzzellbiokatalysator dar. Bei dem verwendeten Wirtstamm handelt es sich um einen modifizierten *E. coli* BL21(DE3) mit der Bezeichnung *E. coli* BL49 (identisch mit E.coli BL21(DE3) hdhA$^-$ KanR$^+$ ). Allerdings ist der Wirtsorganismus nicht auf diesen Wirtsstamm beschränkt. Der mit p7(A)T3rG transformierte *E. coli* BL49 trägt die Bezeichnung *E. coli* BL49 p7(A)T3rG, der mit p3T7(A)rG transformierte *E. coli* BL49 trägt die Bezeichnung *E. coli* BL49 p3T7(A)rG.

Mit den neu hergestellten Biokatalysatoren bei kann Einsatz von insgesamt 17,7 g/L $_{BTM}$ Biokatalysatoren 100 mM DHCA zu 98 % zu 12-keto-UDCA umgesetzt werden.

**12.1 Zweistufige Ganzzellreduktion von Dehydrocholsäure im 20 mL-Maßstab**

Der Vergleich der Biokatalysatoren erfolgte durch Ganzzellbiotransformation im 20 mL-Maßstab. Die Kultivierung und die Konzentrationsermittlung des Substrats DHCA, der Intermediate 3,12-diketo-UDCA und 7,12-diketo-UDCA sowie des Produkts 12-keto-UDCA im Reaktionsansatz erfolgte wie im Umsetzungsbeispiel 11.1 beschrieben.

Die Reaktionsansätze für die Biotransformationen enthielten 11,8 g/L BTM Ganzzellbiokatalysatoren, 100 mM Substrat (DHCA), 500 mM Glucose und waren in 50 mM Kaliumformiatpuffer (pH 7,3) suspendiert. Der Prozess lief im 20 mL Maßstab als Satzverfahren ohne pH-Kontrolle ab. Eine Perforation der Biokatalysatoren war nicht notwendig. In der nachfolgenden Tabelle sind Ergebnisse der oben beschriebenen Ganzzellbiotransformation nach 24 h Prozess dargestellt, wobei die Ergebnisse der Ganzzellbiotransformation mit den beiden Stämmen *E. coli* BL49 p7(A)T3rG und *E. coli* BL49 p3T7(A)rG gezeigt sind. Mit den neuen Biokatalysatoren kann unter Einsatz von 11,8 g L-1BTM Biokatalysator innerhalb von 24 h 100 mM Dehydrocholsäure (DHCA) zu 46 % (*E. coli* BL49 p3T7(A)rG) beziehungsweise zu 68 % (*E. coli* BL49 p7(A)T3rG) zu 12-keto-Ursodesoxycholsäure (12-keto-UDCA) umgesetzt werden. In der nachfolgenden Tabelle sind Gallensalzanteile von Biotransformationsansätzen nach 24 h bei Einsatz von 100 mM Substrat (DHCA) gezeigt (Ansätze mit den zwei neuen Biokatalysatorstämmen *E. coli* BL49 p3T7(A)rG und *E. coli* BL49 p7(A)T3rG):

| | Gallensalzanteile, % | | | |
|---|---|---|---|---|
| | 12-keto-UDCA | 3,12-diketo-UDCA | 7,12-diketo-UDCA | DHCA |
| *E. coli* BL49 p3T7(A)rG | 46 ± 6 % | 7 ± 1 % | 7 ± 1 % | 40,0 ± 2% |
| *E. coli* BL49 p7(A)T3rG | 68 ±19% | 15 ± 3 % | 1 ± 1 % | 12 ± 10 % |

### 12.2 Zweistufige Ganzzellreduktion von Dehydrocholsäure mit im Bioreaktor kultivierten Zellen des Stammes E.coli BL49 p7(A)T3rG im 20 mL-Maßstab

Der Stamm *E. coli* BL49 p7(A)T3rG wurde im 7 L-Bioreaktor bei einem Arbeitsvolumen von 4 L im definierten Minimalmedium kultiviert. Nach einer kurzen Satzphase bei 37 °C erfolgte eine substratlimitierte exponentielle Wachstumsphase bei 30 °C. Bei Erreichen einer optischen Dichte von 25 - 30 wurde die Proteinexpression der Zellen durch Zugabe von 0,5 mM IPTG induziert. Die Expression erfolgte danach für 24 h bei 20 °C. Die Zellen (46,7 g / L BTM) wurden durch Zentrifugation geerntet, mit Kaliumphosphatpuffer (pH 6,5) resuspendiert und nach Standardprotokoll bei -20 °C gelagert. Die Enzymaktivitäten betrugen zum Erntezeitpunkt: 16,4 U/mL 7β-HSDH, 3,6 U/mL 3α-HSDH, 44,9 U/mL GDH (NADP), 18,1 U/mL GDH (NAD).

Die Biotransformation wurde im 20 mL-Maßstab unter folgenden Reaktionsbedingungen angesetzt: 100 mM DHCA, 17,7 g/L BTM des gelagerten Biokatalysators, 500 mM Glucose, 10 mM $MgCl_2$, suspendiert in 50 mM KPi-Puffer (pH 7). Durch manuelle pH-Einstellung wurde der pH während der gesamten Biotransformation auf pH 7 gehalten. Die Reaktionen wurden entweder ohne Kofaktorzugabe oder mit Zugabe von 0,1 mM NAD durchgeführt. Die zeitlichen Verläufe der Gallensalzkonzentrationen sind in Figur 22 dargestellt. Nach 2 h wurden mit und ohne NAD-Zugabe jeweils ≥98 % Produkt (12-keto-UDCA) gebildet.

Bei Einsatz von insgesamt 17,7 g/L BTM Biokatalysatoren wurde 100 mM DHCA zu 98 % zu 12-keto-UDCA umgesetzt.

## Umsetzungsbeispiel 13: Zweistufige Reduktion von Dehydrocholsäure unter paralleler Verwendung von zwei verschiedenen Ganzzellbiokatalysatoren

Im vorliegenden Beispiel wurden zwei Ganzzellbiokatalysatoren anstelle von einem Ganzzellbiokatalysator verwendet. Für diesen Zweck wurden in einem dieser Biokatalysatoren eine NADP-spezifische FDH aus *Mycobacterium vaccae* und eine NADPH-spezifische 7β-HSDH aus *Collinsella aerofaciens* exprimiert, während im anderen Biokatalysator eine NAD-abhängige FDH aus *Mycobacterium vaccae* und eine NADH-abhängige 3α-HSDH aus *Comamonas testosteroni* exprimiert wurden

Im vorliegenden Beispiel wurden konkret der Vektor pF(G)r7(A), welcher Gene für eine NADP-spezifische FDH aus *Mycobacterium vaccae* und für eine NADPH-spezifische 7β-HSDH aus *Collinsella aerofaciens* beinhaltet, sowie der Vektor pFr3, welcher Gene für eine NAD-abhängige FDH aus *Mycobacterium vaccae* und für eine NADH-abhängige 3α-HSDH aus *Comamonas testosteroni* beinhaltet, verwendet. Die Vektoren pF(G)r7(A) und pFr3 sind in Figur 23 a und b dargestellt. Figur 24 zeigt eine Reaktionsschema mit möglichen Reaktionswegen und Produkten. Allerdings ist die Erfindung nicht auf diese zwei genannten Vektoren beschränkt, sondern kann sämtliche denkbaren Vektoren umfassen, die Gene für eine 7β-HSDH und ein geeignetes Kofaktorregenerationsenzym in Kombination mit Vektoren, die Gene für eine 3α-HSDH und ein geeignetes Kofaktorregenerationsenzym beinhalten.

### 13.1 Ganzzellreduktion von DHCA unter Einsatz zweier Biokatalysatoren in unterschiedlichen Mischungsverhältnissen

Im folgenden Beispiel ist die Ganzzellreduktion von DHCA zu 12-keto-UDCA mit unterschiedlichen Mischungsverhältnisse der Biokatalysatoren *E. coli* BL49 pF(G)r7(A) und *E. coli* BL49 pFr3 gezeigt. Hierbei wurden bei drei Ansätzen jeweils 17,7 g $L^{-1}{}_{BTM}$ Gesamtbiokatalysatormenge eingesetzt. Allerdings wurden in den Ansätzen unterschiedliche Anteile der zwei Biokatalysatoren eingesetzt. Diese sind

- 8,85 g L-1BTM *E. coli* BL49 pF(G)r7(A) und 8,85 g $L^{-1}{}_{BTM}$ *E. coli* BL49 pFr3
- 10,33 g L-1BTM *E. coli* BL49 pF(G)r7(A) und 7,38 g $L^{-1}{}_{BTM}$ *E. coli* BL49 pFr3
- 11,80 g L-1BTM *E. coli* BL49 pF(G)r7(A) und 5,90 g $L^{-1}{}_{BTM}$ *E. coli* BL49 pFr3

Diese Biokatalysereaktionen wurden bei einem Arbeitsvolumen von 20 mL durchgeführt. Weitere Bestandteile der Ansätze waren: 70 mM DHCA, 500 mL Natriumformiat, 26 % Glycerin, 50 mM MgCl2, suspendiert in 50 mL Kaliumphosphatpuffer (pH 6,5). Die Reaktionen wurden 24 h lang durchgeführt. Die Gallensalzanteile nach 24 h sind in Fig. 25 gezeigt.

Im Ansatz mit 8,85 g $L^{-1}{}_{BTM}$ E. coli BL49 pF(G)r7(A) und 8,85 g $L^{-1}{}_{BTM}$ E. coli BL49 pFr3 werden 79 % 12-keto-UDCA, 4 % 3,12-diketo-UDCA und 17 % 7,12-diketo-UDCA gebildet, im Ansatz mit 10,33 g $L^{-1}{}_{BTM}$ E. coli BL49 pF(G)r7(A) und 7,38 g $L^{-1}{}_{BTM}$ E. coli BL49 pFr3 werden 87 % 12-keto-UDCA, 9 % 3,12-diketo-UDCA und 4 % 7,12-diketo-UDCA gebildet und im Ansatz mit 11,80 g $L^{-1}{}_{BTM}$ E. coli BL49 pF(G)r7(A) und 5,90 g $L^{-1}{}_{BTM}$ E. coli BL49 pFr3 werden 71 % 12-keto-UDCA und 29 % 3,12-diketo-UDCA gebildet. An den Anteilen der gebildeten Intermediate ist zu erkennen, dass bei dem Ansatz mit 8,85 g $L^{-1}{}_{BTM}$ E. coli BL49 pF(G)r7(A) und 8,85 g $L^{-1}{}_{BTM}$ E. coli BL49 pFr3 die

Reaktion der 3α-HSDH mit erhöhter Geschwindigkeit abläuft, da hier ein hoher Anteil am Intermediat 7,12-diketo-UDCA gebildet wird, während bei dem Ansatz 11,80 g $L^{-1}{}_{BTM}$ E. coli BL49 pF(G)r7(A) und 5,90 g $L^{-1}{}_{BTM}$ E. coli BL49 pFr3 die Reaktion der 7β-HSDH mit erhöhter Geschwindigkeit abläuft und dementsprechend ein hoher Anteil am Intermediat 3,12-diketo-UDCA gebildet wird. Beim Ansatz mit 10,33 g $L^{-1}{}_{BTM}$ E. coli BL49 pF(G)r7(A) und 7,38 g $L^{-1}{}_{BTM}$ E. coli BL49 pFr3 sind die Reaktionen der 7β-HSDH und der 3α-HSDH so abgestimmt, dass beide ungefähr gleich schnell ablaufen, was an den annähernd gleichmäßigen Intermediatbildungen zu sehen ist. Infolge dessen kann bei diesem Ansatz der höchste Anteil am Produkt 12-keto-UDCA gebildet werden (87 %).

Dieses Beispiel zeigt, wie sich durch Anpassung der eingesetzten Biokatalysatoranteile die Geschwindigkeit einzelner Reaktionsschritte beeinflussen lassen.

**13.1 Ganzzellreduktion von DHCA unter Einsatz zweier Biokatalysatoren im 1 L-Maßstab**

Durch Prozesskontrolle konnte die Produktbildung unter Einsatz der Biokatalysatoren *E. coli* BL49 pF(G)r7(A) und *E. coli* BL49 pFr3 gegenüber dem Millilitermaßstab gesteigert werden. Die Biotransformation wurde in einem 1 L-Bioreaktor unter folgenden Reaktionsbedingungen angesetzt: 90 mM DHCA, mit 8,85 g $L^{-1}{}_{BTM}$ *E. coli* BL49 pF(G)r7(A) und 8,85 g $L^{-1}{}_{BTM}$ *E. coli* BL49 pFr3, 500 mM Ammoniumformiat, 26 % (v/v) Glycerin, 50 mM MgCl2, suspendiert in 50 mM KPi-Puffer (pH 6,5). Durch pH-Regelung mit Ameisensäure wurde der pH während der gesamten Biotransformation auf pH 6,5 gehalten. Die zeitlichen Verläufe der Gallensalzkonzentrationen sind in Fig. 26 dargestellt. Nach 20 h konnten 99,4 % Produkt (12-keto-UDCA) gebildet werden und lediglich insgesamt 0,6 % der Intermediate 3,12-diketo-UDCA und 7,12-diketo-UDCA waren nachweisbar.

Mit der Verwendung der erfindungsgemäßen Biokatalysatoren konnten hier bei Einsatz von insgesamt 17,7 g $L^{-1}{}_{BTM}$ Biokatalysatoren 90 mM DHCA zu 99,5 % zu 12-keto-UDCA umgesetzt werden.

Zuordnung von SEQ ID NOs:

| SEQ ID NO: | Beschreibung | Typ |
|---|---|---|
| 1 | 7ß-HSDH | NS |
| 2 | 7ß-HSDH | AS |
| 3 | Primer S 7beta_rev_HindIII | NS |
| 4 | Primer | NS |
| 5 | 3α-HSDH (C. testosteroni) | NS |
| 6 | 3α-HSDH (C. testosteroni) | AS |
| 7 | 3α-HSDH (R. norvegicus) | NS |
| 8 | 3α-HSDH (R. norvegicus) | AS |
| 9 | Primer 7beta_mut_G39A_fwd | NS |
| 10 | Primer 7beta_mut_G39A_rev | NS |
| 11 | Primer 7beta_mut_G39S_fwd | NS |
| 12 | Primer 7beta_mut_G39S_rev | NS |
| 13 | Primer 7beta_fwd_EcoRI | NS |
| 14 | FDH D221G | NS |
| 15 | FDH D221G | AS |
| 16 | Primer 3alpha_finid_HindIII | NS |
| 17 | Primer 3alpha_rev_Notl | NS |
| 18 | pET22a FDH D221G 7ß-HSDH | NS |
| 19 | FDH D221G | AS |
| 20 | 7beta-HSDH | AS |

(fortgesetzt)

| SEQ ID NO: | Beschreibung | Typ |
|---|---|---|
| 21 | pCOLA(mod) 3$\alpha$-HSDH | NS |
| 22 | 3$\alpha$-HSDH | AS |
| 23 | Primer fdh_for | NS |
| 24 | Primer fdh_rev | NS |
| 25 | 7$\alpha$-HSDH | NS |
| 26 | 7$\alpha$-HSDH | AS |
| 27 | Primer 467\|468a-IBS | NS |
| 28 | Primer 467\|468a-EBS1d | NS |
| 29 | Primer 467\|468a-EBS2 | NS |
| 30 | Primer mt1 | NS |
| 31 | Primer NI_fdh_R | NS |
| 32 | Primer 7alpha-ko-check_fwd | NS |
| 33 | Primer 7alpha-ko-check_rev | NS |
| 34 | FDH D221G mit Deletion und His-Tag | NS |
| 35 | FDH D221G mit Deletion und His-Tag | AS |
| 36 | FDH Wildtyp, M. vaccae | AS |
| 37 | 7$\beta$-HSDH G39D | AS |
| 38 | 7$\beta$-HSDH G39D/R40L | AS |
| 39 | 7$\beta$-HSDH G39D/R40I | AS |
| 40 | 7$\beta$-HSDH G39D/R40V | AS |
| 41 | Primer für G39D (7beta mut G39D fwd) | NS |
| 42 | Primer für G39D (7beta mut G39D rev) | NS |
| 43 | Primer für G39D R40L (7beta mut G39D R40L fwd) | NS |
| 44 | Primer 3alphamut_AntiMid_rev | NS |
| 45 | Primer für G39D R40I (7beta mut G39D R40I fwd) | NS |
| 46 | Primer 7beta mut G39D R40V fwd | NS |
| 47 | GDH, B. subtilis | NS |
| 48 | GDH B. subtilis | AS |
| 49 | Vektor pFr7(D) | NS |
| 50 | Vektor pFr7(DL) | NS |
| 51 | Vektor pFr7(DI) | NS |
| 52 | Vektor pFr7(DV) | NS |
| 53 | PCR Primer "G39D for" | NS |
| 54 | PCR Primer "G39D_rev" | NS |
| 55 | PCR Primer "G39D/R40I_for" | NS |
| 56 | PCR Primer "G39D/R40I_rev" | NS |
| 57 | PCR Primer "R40D_for" | NS |
| 58 | PCR Primer "R40D_rev" | NS |

(fortgesetzt)

| SEQ ID NO: | Beschreibung | Typ |
|---|---|---|
| 59 | PCR Primer "R40D/R41I_for" | NS |
| 60 | PCR Primer "R40D/R41I_rev" | NS |
| 61 | PCR Primer "DIN _ for" | NS |
| 62 | PCR Primer "DIN_rev" | NS |
| 63 | Plasmid pFr3T7(D), Fig. 15 | NS |
| 64 | Plasmid pF(G)r7(A)r3, Fig. 17a | NS |
| 65 | Plasmid pF(G)r7(S)r3, Fig. 17b | NS |
| 66 | Plasmid p3T7(A)rG, Fig. 20 | NS |
| 67 | Plasmid p7(A)T3rG, Fig. 21 | NS |
| 68 | Plasmid pF(G)r7(A), Fig. 23a | NS |
| 69 | Plasmid pFr3, Fig. 23b | NS |
| AS = Aminosäuresequenz<br>NS = Nukleinsäuresequenz | | |

**Patentansprüche**

1. Rekombinanter Mikroorganismus, der auf einem oder mehreren Expressionskonstrukten:

   (a) die Nukleinsäuresequenz kodierend für ein erstes Enzym, welches eine 7ß-Hydroxysteroiddehydrogenase (7ß-HSDH) ist,
   (b) die kodierende Sequenz für mindestens ein zweites Enzym, ausgewählt unter Hydroxysteroiddehydrogenasen, welche sich von der 7ß-HSDH unterscheiden, wobei das zweite Enzym eine 3α-Hydroxysteroiddehydrogenase (3α-HSDH) umfasst; und
   (c) gegebenenfalls die kodierende Sequenz für mindestens ein drittes Enzym, ausgewählt unter zur Kofaktorregenerierung geeigneten Dehydrogenasen

   trägt.

2. Rekombinanter Mikroorganismus nach einem der Ansprüche 1, welcher zur gleichzeitigen Expression der 7ß-HSDH, der zweiten, von 7ß-HSDH verschiedenen Hydroxysteroiddehydrogenase und gegebenenfalls der zur Kofaktorregenerierung geeigneten Dehydrogenase befähigt ist.

3. Rekombinanter Mikroorganismus nach einem der Ansprüche 1 oder 2, wobei die zur Kofaktorregenerierung geeigneten Dehydrogenasen ausgewählt sind unter:

   einem NADPH-regenerierenden Enzym, ausgewählt unter NADPH Dehydrogenasen, Alkoholdehydrogenasen (ADH), und NADPH regenerierenden Formiatdehydrogenasen (FDH), sowie NADPH-regenerierende Glucosedehydrogenasen (GDH), Glucose-6-Phosphat-Dehydrogenasen (G-6-PDH), oder Phosphit-Dehydrogenasen (PtDH); und
   einem NADH-regenerierenden Enzym, ausgewählt unter NADH-Dehydrogenasen, NADH-regenerierenden Formiatdehydrogenasen (FDH), NADH-regenerierenden Alkoholdehydrogenasen (ADH), NADH-regenerierenden Glucose-6-Phosphat-Dehydrogenasen (G6PDH), NADH-regenerierenden Phosphitdehydrogenasen (PtDH) sowie NADH-regenerierenden Glucosedehydrogenasen (GDH).

4. Rekombinanter Mikroorganismus nach einem der vorhergehenden Ansprüche, welcher zur gleichzeitigen Expression der 7ß-HSDH, einer FDH und einer 3α-HSDH befähigt ist; oder welcher zur gleichzeitigen Expression der 7β-HSDH, einer GDH und einer 3α-HSDH befähigt ist.

**5.** Rekombinanter Mikroorganismus nach Anspruch 4, wobei die FDH eine Mutante einer $NAD^+$-abhängigen FDH ist, welche wenigstens die enzymatische Oxidation von Ameisensäure zu $CO_2$ katalysiert, wobei die Mutante im Vergleich zum nicht-mutierten Enzym zusätzlich $NADP^+$ als Kofaktor akzeptiert.

**6.** Rekombinanter Mikroorganismus nach Anspruch 5, wobei die $NADP^+$-akzeptierende FDH wenigstens eine Mutation im Sequenzmotiv TDRHRL gemäß Position 221 bis 226 von SEQ ID NO:36 und wenigstens 95 % Sequenzidentität zu SEQ ID NO:36 aufweist.

**7.** Rekombinanter Mikroorganismus nach Anspruch 4 oder 6, wobei die FDH eine FDH aus *Mycobacterium vaccae* N10 gemäß SEQ ID NO: 36 oder eine davon abgeleitete FDH mit wenigstens 90 % Sequenzidentität ist.

**8.** Verfahren zur Synthese von 7ß-Hydroxysteroiden, wobei man das korrespondierende 7-Ketosteroid in Gegenwart eines rekombinanten Mikroorganismus gemäß der Definition in einem der Ansprüche 1 bis 7 umsetzt, und wenigstens ein gebildetes Reduktionsprodukt aus dem Reaktionsansatz gegebenenfalls isoliert.

**9.** Verfahren nach Anspruch 8, wobei die Reduktion in Gegenwart und insbesondere unter Verbrauch von NADPH und/oder NADH erfolgt.

**10.** Verfahren zur Herstellung von Ursodesoxycholsäure (UDCS) der Formel (1)

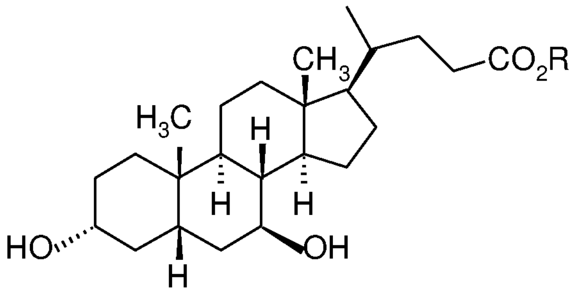

(1)

worin

R für Alkyl, H, ein Alkalimetallion oder $N(R^3)_4{}^+$steht, worin die Reste $R^3$ gleich oder verschieden sind und für H oder Alkyl stehen,
wobei man

a) gegebenenfalls eine Cholsäure (CS) der Formel (2)

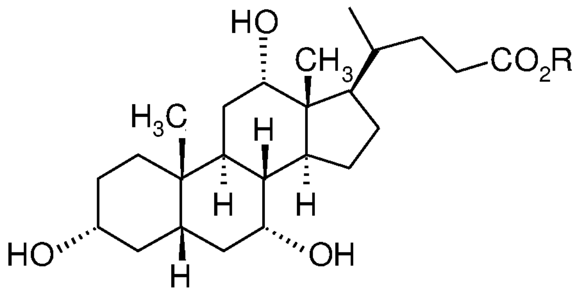

(2)

worin R die oben angegebenen Bedeutungen besitzt, zur Dehydrocholsäure (DHCS) der Formel (3)

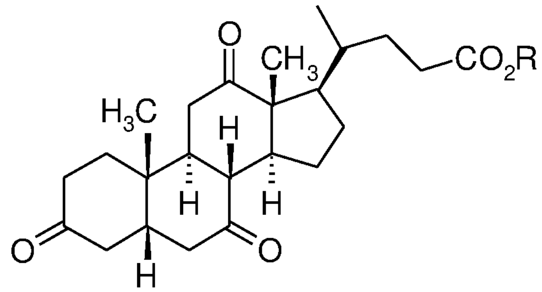

(3)

worin R die oben angegebenen Bedeutungen besitzt, chemisch oxidiert;

b) DHCS in Gegenwart wenigstens eines rekombinanten Mikroorganismus gemäß der Definition in einem der Ansprüche 1 bis 7 zur korrespondierenden 12-Ketoursodesoxychiolsäure (12-Keto UDCS) der Formel (5)

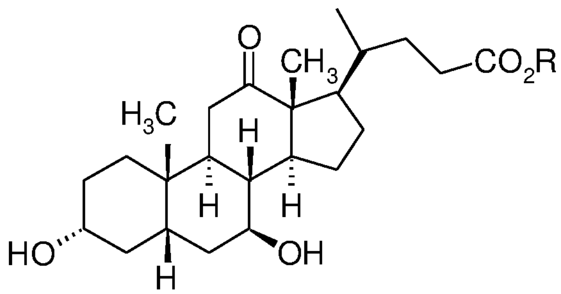

(5)

worin R die oben angegebenen Bedeutungen besitzt, insbesondere in Gegenwart und unter Verbrauch von NADH und/oder NADPH, reduziert und anschließend

c) 12-Keto-UDCS der Formel (5) chemisch zu UDCS reduziert; und

d) das Reaktionsprodukt gegebenenfalls weiter aufreinigt.

## Claims

1. Recombinant microorganism carrying on one or more expression constructs:

(a) the nucleic acid sequence encoding a first enzyme which is a 7β-hydroxysteroid dehydrogenase (7β-HSDH),

(b) the coding sequence for at least a second enzyme selected from hydroxysteroid dehydrogenases different from the 7β-HSDH, wherein said second enzyme comprises a 3 α-hydroxysteroid dehydrogenase (3α-HSDH); and

(c) optionally, the coding sequence for at least a third enzyme selected from dehydrogenases suitable for cofactor regeneration.

2. Recombinant microorganism according to one of claims 1, which is capable of simultaneously expressing 7β-HSDH, the second hydroxysteroid dehydrogenase different from 7β-HSDH and optionally the dehydrogenase suitable for cofactor regeneration.

3. The recombinant microorganism according to any one of claims 1 or 2, wherein the dehydrogenases suitable for cofactor regeneration are selected from: an NADPH-regenerating enzyme selected from NADPH dehydrogenases, alcohol dehydrogenases (ADH), and NADPH-regenerating formate dehydrogenases (FDH), as well as NADPH-regenerating glucose dehydrogenases (GDH), glucose-6-phosphate dehydrogenases (G-6-PDH), or phosphite dehydrogenases (PtDH); and

an NADH-regenerating enzyme selected from NADH dehydrogenases, NADH-regenerating formate dehydrogenases (FDH), NADH-regenerating alcohol dehydrogenases (ADH), NADH-regenerating glucose-6-phosphate dehydrogenases (G-6-PDH), NADH-regenerating phosphite dehydrogenases (PtDH) and NADH-regenerating glucose dehydrogenases (GDH).

**4.** A recombinant microorganism according to any one of the preceding claims, which is capable of simultaneously expressing the 7β-HSDH, an FDH and a 3α-HSDH; or which is capable of simultaneously expressing the 7β-HSDH, a GDH and a 3α- HSDH.

**5.** The recombinant microorganism according to claim 4, wherein the FDH is a mutant of an $NAD^+$-dependent FDH that at least catalyses the enzymatic oxidation of formic acid to $CO_2$, wherein the mutant, compared to the nonmutated enzyme additionally accepts $NADP^+$ as a cofactor.

**6.** The recombinant microorganism according to claim 5, wherein the $NADP^+$-accepting FDH has at least one mutation in the sequence motif TDRHRL according to position 221 to 226 of SEQ ID NO: 36 and at least 95% sequence identity to SEQ ID NO: 36.

**7.** The recombinant microorganism according to claim 4 or 6, wherein the FDH is an FDH from *Mycobacterium vaccae* N10 according to SEQ ID NO: 36 or an FDH derived therefrom having at least 90% sequence identity to SEQ ID NO: 36.

**8.** A process for the synthesis of 7β-hydroxysteroids, wherein the corresponding 7-ketosteroid is reacted in the presence of a recombinant microorganism as defined in any one of claims 1 to 7, and at least one reduction product formed is optionally isolated from the reaction mixture.

**9.** The process according to claim 8, wherein the reduction is carried out in the presence of and in particular with consumption of NADPH and/or NADH.

**10.** Process for the preparation of ursodeoxycholic acid (UDCA) of formula (1)

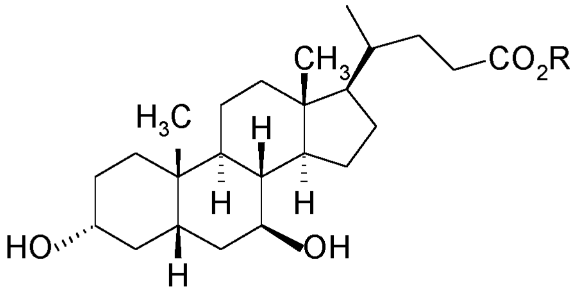

(1)

wherein R stands for alkyl, H, an alkali metal ion or $N(R^3)_4^+$, in which the residues $R^3$ may be identical or different and stand for H or alkyl,
wherein

a) optionally a cholic acid (CA) of formula (2)

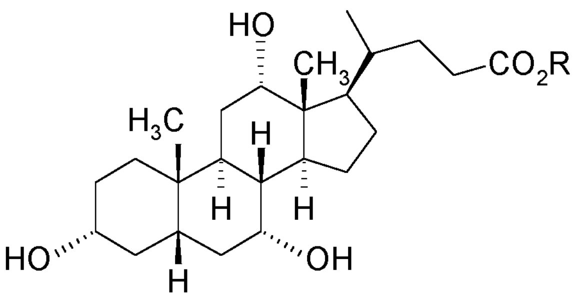

(2)

in which R has the meanings given above, is oxidized chemically to dehydrocholic acid (DHCA) of formula (3)

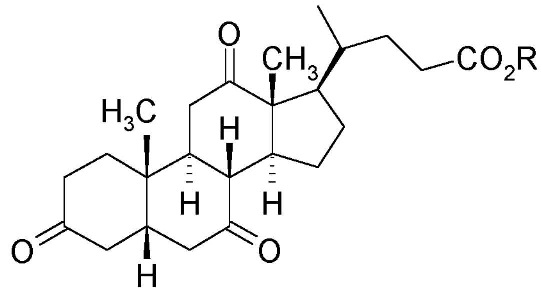

(3)

in which R has the meanings given above;

b) DHCA is reduced in the presence of at least one recombinant microorganism as defined in any one of claims 1 to 7 to the corresponding 12-keto-ursodeoxycholic acid (12-keto UDCA) of formula (5)

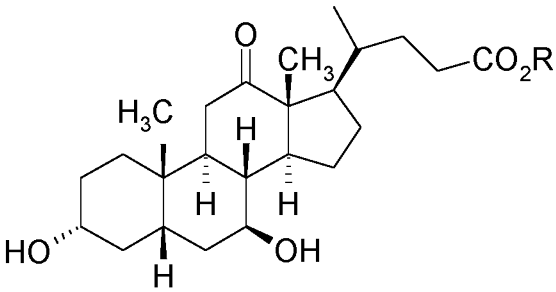

(5)

in which R has the meanings given above, in particular in the presence and with consumption of NADH and/or NADPH, and then

c) 12-keto-UDCA of the formula (5) is reduced chemically to UDCA; and

d) the reaction product optionally is further purified.

**Revendications**

1. Microorganisme recombinant portant, sur une ou plusieurs structures d'expression :

(a) la séquence d'acides nucléiques codant pour une première enzyme consistant en une 7β-hydroxystéroïde-déshydrogénase (7β-HSDH),

(b) la séquence codant pour au moins une deuxième enzyme, choisie parmi les hydroxystéroïde-déshydrogénases différentes de la 7β-HSDH, ladite deuxième enzyme comprenant une 3α-hydroxystéroïde-déshydrogénase (3α-HSDH), et

(c) éventuellement la séquence codant pour au moins une troisième enzyme, choisie parmi les déshydrogénases appropriées à la régénération du cofacteur.

2. Microorganisme recombinant selon la revendication 1, apte à l'expression simultanée de la 7β-HSDH, de la deuxième hydroxystéroïde-déshydrogénase différente de ladite 7β-HSDH et éventuellement de la déshydrogénase appropriée à la régénération du cofacteur.

3. Microorganisme recombinant selon l'une des revendications 1 et 2, dans lequel les déshydrogénases appropriées à la régénération du cofacteur sont choisies parmi :

une enzyme de régénération de NADPH choisie parmi les NADPH-déshydrogénases, les alcool-déshydrogénases (ADH), et les formiate-déshydrogénases (FDH) de régénération de NADPH, ainsi que les glucose-déshydrogénases (GDH) de régénération de NADPH, les glucose-6-phosphate-déshydrogénases (G-6-PDH) ou les phosphite-déshydrogénases (PtDH) ; et

une enzyme de régénération de NADH choisie parmi les NADH-déshydrogénases, les formiate-déshydrogénases (FDH) de régénération de NADH, les alcool-déshydrogénases (ADH) de régénération de NADH, les glucose-6-phosphate-déshydrogénases (G6PDH) de régénération de NADH, les phosphite-déshydrogénases

(PtDH) de régénération de NADH ainsi que les glucose-déshydrogénases (GDH) de régénération de NADH.

**4.** Microorganisme recombinant selon l'une des revendications précédentes, apte à l'expression simultanée de la 7β-HSDH, d'un mutant de FDH et d'une 3α-HSDH ; ou apte à l'expression simultanée de la 7β-HSDH, d'une GDH et d'une 3α-HSDH.

**5.** Microorganisme recombinant selon la revendication 4, dans lequel la FDH est un mutant d'une FDH dépendante de $NAD^+$ catalysant au moins l'oxydation enzymatique de l'acide formique en $CO_2$, ledit mutant acceptant en outre $NADP^+$ comme cofacteur, comparativement à l'enzyme non mutée.

**6.** Microorganisme recombinant selon la revendication 5, dans lequel la FDH acceptant $NADP^+$ présente au moins une mutation dans le motif séquentiel TDRHRL selon les positions 221 à 226 de SEQ ID NO:36 et au moins 95 % d'identité de séquence avec SEQ ID NO:36.

**7.** Microorganisme recombinant selon la revendication 4 ou 6, dans lequel la FDH est une FDH issue de *Mycobacterium vaccae* N10 selon SEQ ID NO:36 ou une FDH dérivée de celle-ci et présentant au moins 90 % d'identité de séquence.

**8.** Procédé de synthèse de 7β-hydroxystéroïdes, dans lequel on fait réagir le 7-cétostéroïde correspondant en présence d'un microorganisme recombinant selon la définition donnée dans l'une des revendications 1 à 7, et on isole éventuellement de la préparation de réaction au moins un produit de réduction formé.

**9.** Procédé selon la revendication 8, dans lequel la réduction a lieu en présence et notamment avec consommation de NADPH et/ou de NADH.

**10.** Procédé de fabrication d'acide ursodésoxycholique (AUDC) de formule (1)

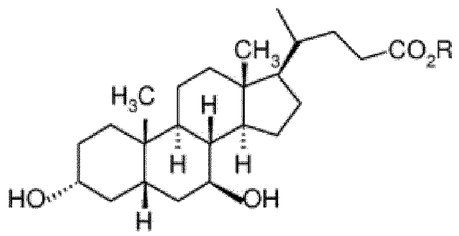

(1)

dans laquelle

R représente alkyle, H, un ion de métal alcalin ou $N(R^3)_4^+$, les radicaux $R^3$ étant identiques ou différents et représentant H ou alkyle,
selon lequel procédé

a) on oxyde chimiquement éventuellement un acide cholique (AC) de formule (2)

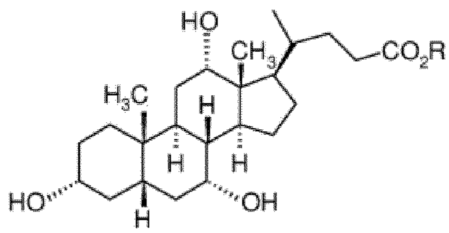

(2)

dans laquelle R a les significations indiquées précédemment, pour former l'acide déshydrocholique (ADHC) de formule (3)

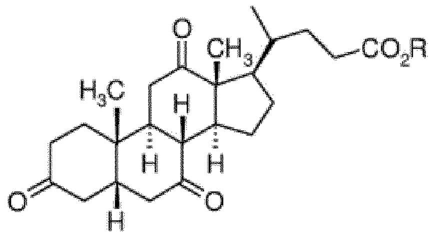

(3)

dans laquelle R a les significations indiquées précédemment ;

b) on réduit, notamment en présence et avec consommation de NADH et/ou de NADPH, l'ADHC en présence d'au moins un microorganisme recombinant selon la définition donnée dans l'une des revendications 1 à 7 pour former l'acide 12-céto-ursodesoxychiolique (12-céto AUDC) correspondant de formule (5)

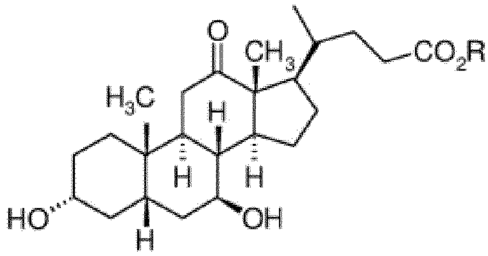

(5)

dans laquelle R a les significations indiquées précédemment, puis

c) on réduit chimiquement le 12-céto-AUDC de formule (5) en AUDC, et

d) on purifie éventuellement encore le produit de réaction.

Zugang: ZP_01773061

```
  1 mnlrekygew glilgategv gkafcekiaa ggmnvvmvgr reeklnvlag eiretygvet
 61 kvvradfsqp gaaetvfaat egldmgfmsy vaclhsfgki qdtpwekhea minvnvvtfl
121 kcfhhymrif aaqdrgavin vssmtgisss pwngqygagk afilkmteav acecegtgvd
181 vevitlgttl tpsllsnlpg gpqgeavmki altpeecvde afeklgkels viagqrnkds
241 vhdwkanhte deyirymgsf yrd
```

**Fig. 1a**

Zugangs-Nr.: NZ_AAVN02000010, Region: 52005..52796

```
  1 atgaacctga gggagaagta cggtgagtgg ggcctgatcc tgggcgcgac cgagggcgtc
 61 ggcaaggcgt tctgcgagaa gatcgccgcc ggcggcatga acgtcgtcat ggtcggccgt
121 cgcgaggaga agctgaacgt gctcgcaggc gagatccgcg agacctacgg cgtggagacc
181 aaggtcgtgc gcgccgactt tagccagccc ggcgctgccg agaccgtctt cgccgcgacc
241 gagggcctgg acatgggctt catgagctac gtggcctgcc tgcacagctt cggtaagatc
301 caggacaccc cctgggagaa gcacgaggcc atgatcaacg tcaacgtcgt gaccttcctc
361 aagtgcttcc accactacat gcggatcttt gccgcccagg accgcggcgc cgtgatcaac
421 gtctcgtcga tgaccggcat cagctccagc ccctggaacg gccagtacgg cgcgggcaag
481 gccttcatcc tcaagatgac cgaggccgtg gcctgcgagt gcgagggcac cggcgtcgac
541 gtcgaggtca tcaccctcgg caccacccta acccccagcc tgctgtccaa cctccccggc
601 ggcccgcagg gcgaggccgt catgaagatc gccctcaccc ccgaggagtg cgttgacgag
661 gcctttgaga agctgggtaa ggagctctcc gtcatcgccg gccagcgcaa caaggactcc
721 gtccacgact ggaaggcaaa ccacaccgag gacgagtaca tccgctacat ggggtcgttc
781 taccgcgact ag
```

**Fig.1b**

3α-HSDH

Quelle: *Comamonas testosteroni* ATCC 11996

```
  1 msiivisgca tgigaatrkv leaaghqivg idirdaevia dlstaegrkq aiadvlakcs
 61 kgmdglvlca glgpqtkvlg nvvsvnyfga telmdaflpa lkkghqpaav vissvasahl
121 afdknplala leageeakar aivehageqg gnlayagskn altvavrkra aawgeagvrl
181 ntiapgatet pllqaglqdp rygesiakfv ppmgrraeps emasviaflm spaasyvhga
241 qividggida vmrptqf
```

**Fig.1c**

Nukleinsäuresequenz codierend für 3α-HSDH

Quelle: *Comamonas testosteroni* ATCC 11996

Accession: AF092031, Region: 158..931

```
  1 atgtccatca tcgtgataag cggctgcgcc accggcattg gtgcggctac gcgcaaggtc
 61 ctggaggcgg ccggtcacca gatcgtaggc atcgatatac gcgatgcgga agtgattgcc
121 gatctctcga cggccgaagg tcgaaagcag gcgattgccg atgtactggc gaagtgcagc
181 aagggcatgg acggcctggt gctgtgcgcc ggcctgggac cgcagaccaa ggtgcttggc
241 aatgtggttt cggtcaatta ttttggcgcg accgagctga tggatgcctt tttgccagcg
301 ctgaaaaaag gccatcagcc cgcagccgtc gtcatctcgt ccgtggcttc cgcgcatctg
361 gcttttgaca agaacccact ggcgctggca ctggaagccg gcgaggaagc caaggcccgc
421 gccattgtcg aacatgcggg agagcagggc ggaaatctgg cctatgcggg cagcaagaat
481 gctttgacgg tggctgtgcg caaacgcgcc gccgcctggg gcgaggctgg cgtgcgcctg
541 aacaccatcg cccccggtgc aaccgagact cccttgctgc aggcgggcct gcaggacccg
601 cgctatggcg aatccattgc caagttcgtt cctcccatgg gccgccgtgc cgagccgtcc
661 gagatggcgt cggtcatcgc ctttttgatg agcccggccg caagctatgt gcatggcgcg
721 cagatcgtca ttgatggcgg cattgatgcg gtgatgcgcc cgacacagtt ctga
```

**Fig.1d**

3α-HSDH

Quelle: Rattus norvegicus (Norway rat)
Genbank ACCESSION M61937

MDSISLRVALNDGNFIPVLGFGTTVPEKVAKDEVIKATKIAIDN
GFRHFDSAYLYEVEEEVGQAIRSKIEDGTVKREDIFYTSKLWSTFHRPELVRTCLEKT
LKSTQLDYVDLYIIHFPMALQPGDIFFPRDEHGKLLFETVDICDTWEAMEKCKDAGLA
KSIGVSNFNCRQLERILNKPGLKYKPVCNQVECHLYLNQSKMLDYCKSKDIILVSYCT
LGSSRDKTWVDQKSPVLLDDPVLCAIAKKYKQTPALVALRYQLQRGVVPLIRSFNAKR
IKELTQVFEFQLASEDMKALDGLNRNFRYNNAKYFDDHPNHPFTDE

**Fig.1e**

atggattcca tatctctgcg tgtagcacta aatgatggta acttcattcc tgtactgggg
tttggaacca ctgtgcctga gaaggttgct aaggatgaag ttatcaaggc tactaaaata
gctatagata atggattccg ccattttgac tctgcttatt tgtacgaagt agaagaggaa
gtgggccaag ccattagaag caagattgaa gacggcactg tgaagagaga agatatattc
tatacttcaa agctttggag cactttccat agaccagagc tggtccgaac ttgcttggaa
aagacactga aaagcactca actggactat gtggatcttt atattattca tttcccaatg
gctttgcagc ctggagatat atttttccca cgagatgagc atggaaaact attgtttgaa
acagtggata tctgtgacac atgggaggcc atggaaaagt gtaaggatgc aggattggcc
aagtctattg gggtgtccaa ctttaactgc aggcagctgg agaggattct gaataagcca
gggctcaaat acaagcctgt gtgcaaccag gtggaatgtc acctttatct caaccagagc
aaaatgctgg actattgtaa gtcaaaagac atcattctgg tttcctactg cacgctggga
agttcacgag acaaaaacatg ggtggatcag aaaagtccag ttctcctaga tgatccagtt
ctttgtgcca tagcaaagaa gtacaagcaa accccagccc tagttgccct tcgctaccag
ctgcagcgtg ggtttgtgcc cctgatcagg agtttcaacg cgaagcggat caaagagcta
acacaggttt ttgaattcca gttggcttca gaggacatga aagccctgga tggcttgaac
agaaatttca gatacaacaa tgcaaaatat tttgatgacc atcccaatca tccatttact
gatgaatag

**Fig.1f**

FDH-Sequenz, D221G-Mutante:

atggcaaaggtcctgtgcgttctttacgatgatccggtcgacggctacccgaagacctatgcccgcgacgatcttccgaa
gatcgaccactatccgggcggccagatcttgccgacgccgaaggccatcgacttcacgcccgggcagttgctcggctccg
tctccggcgagctcggcctgcgcgaatatctcgaatccaacggccacaccctggtcgtgacctccgacaaggacggcccc
gactcggtgttcgagcgcgagctggtcgatgcggatgtcgtcatctcccagcccttctggccggcctatctgacgcccga
gcgcatcgccaaggccaagaacctgaagctcgcgctcaccgccggcatcggttccgaccacgtcgatcttcagtcggcta
tcgaccgcaacgtcaccgtggcggaagtcacctactgcaactcgatcagcgtcgccgagcatgtggtgatgatgatcctg
tcgctggtgcgcaactatctgccctcgcacgaatgggcgcggaagggcggctggaacatcgccgactgcgtctcccacgc
ctacgacctcgaggcgatgcatgtcggcaccgtggccgccggccgcatcggtctcgcggtgctgcgccgtctggcgccgt
tcgacgtgcacctgcactacaccggccgtcaccgcctgccggaatcggtcgagaaggagctcaacctcacctggcacgcg
acccgcgaggacatgtatccggtttgcgacgtggtgacgctgaactgcccgctgcaccccgaaaccgagcacatgatcaa
tgacgagacgctgaagctgttcaagcgtggcgcctacatcgtcaacaccgcccgcggcaagctgtgcgaccgcgatgccg
tggcacgtgcgctcgaatccggccggctggccggctatgccggcgacgtgtggttcccgcagccggcgccgaaggaccac
ccctggcggacgatgccctataacggcatgaccccgcacatctccggcaccacgctgaccgcgcaggcgcgttatgcggc
gggcacccgcgagatcctggagtgcttcttcgagggccgtccgatccgcgacgaatacctcatcgtgcagggcggcgctc
ttgccggcaccggcgcgcattcctactcgaagggcaatgccaccggcggttcggaagaggccgccaagttcaagaaggcg
gtctga

**Fig.1g**

Aminosäure-Sequenz der D221G-Mutante

MAKVLCVLYDDPVDGYPKTYARDDLPKIDHYPGGQILPTPKAIDFTPGQLLGSVSGELGLREYLESNGHTLVVTSDKDGPDSVFE
RELVDADVVISQPFWPAYLTPERIAKAKNLKLALTAGIGSDHVDLQSAIDRNVTVAEVTYCNSISVAEHVVMMILSLVRNYLPSH
EWARKGGWNIADCVSHAYDLEAMHVGTVAAGRIGLAVLRRLAPFDVHLHYTGRHRLPESVEKELNLTWHATREDMYPVCDVVTLN
CPLHPETEHMINDETLKLFKRGAYIVNTARGKLCDRDAVARALESGRLAGYAGDVWFPQPAPKDHPWRTMPYNGMTPHISGTTLT
AQARYAAGTREILECFFEGRPIRDEYLIVQGGALAGTGAHSYSKGNATGGSEEAAKFKKAV*

**Fig.1h**

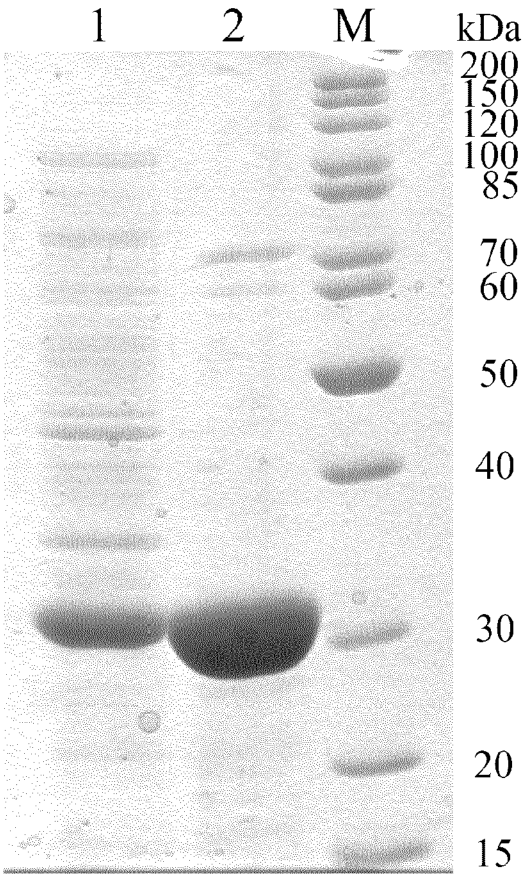


Fig. 2

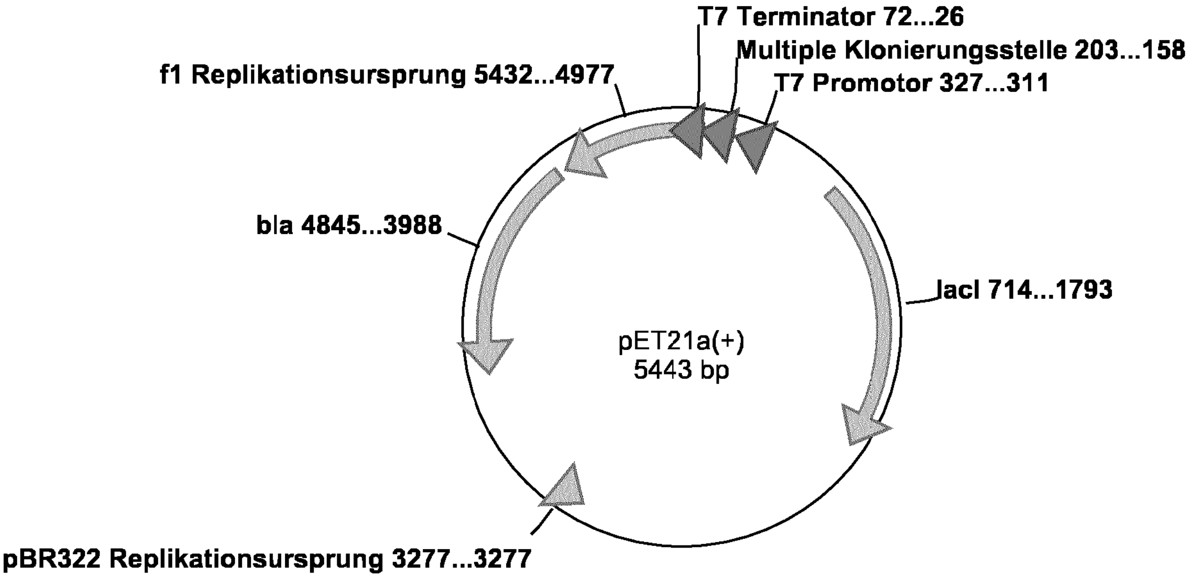
T7 Terminator 72...26
Multiple Klonierungsstelle 203...158
f1 Replikationsursprung 5432...4977
T7 Promotor 327...311
bla 4845...3988
lacI 714...1793
pET21a(+)
5443 bp
pBR322 Replikationsursprung 3277...3277

Fig. 3a

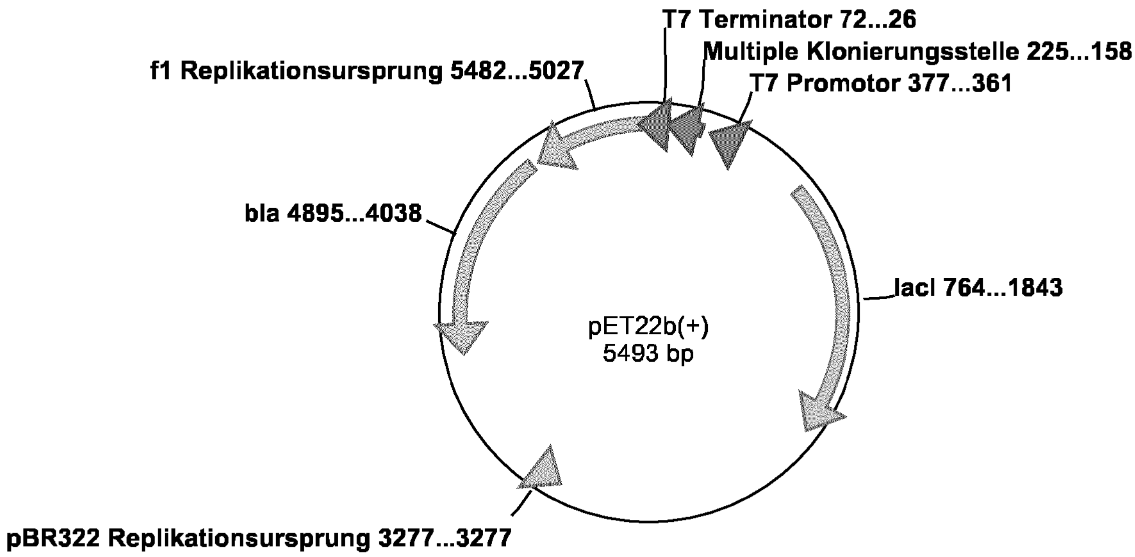
T7 Terminator 72...26
Multiple Klonierungsstelle 225...158
f1 Replikationsursprung 5482...5027
T7 Promotor 377...361
bla 4895...4038
lacI 764...1843
pET22b(+)
5493 bp
pBR322 Replikationsursprung 3277...3277

Fig. 3b

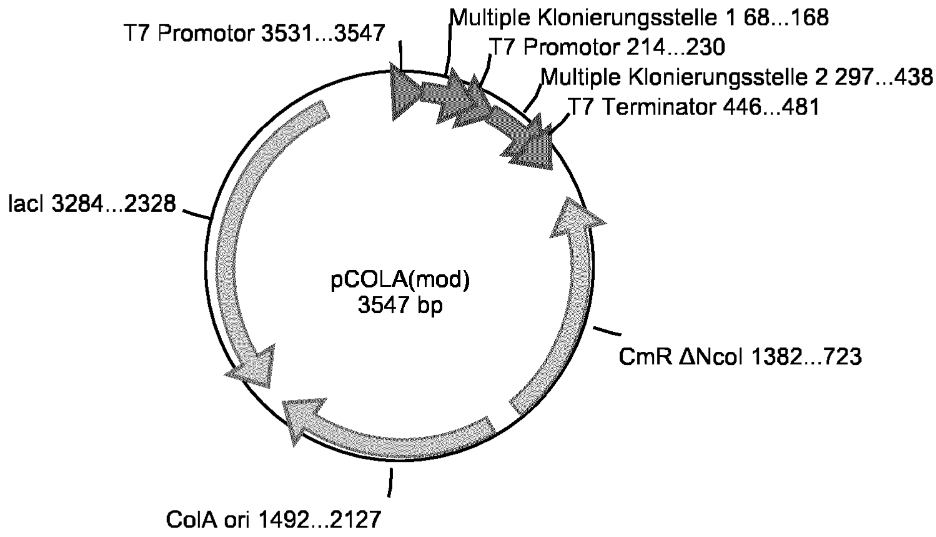
T7 Promotor 3531...3547
Multiple Klonierungsstelle 1 68...168
T7 Promotor 214...230
Multiple Klonierungsstelle 2 297...438
T7 Terminator 446...481
lacI 3284...2328
pCOLA(mod)
3547 bp
CmR ΔNcoI 1382...723
ColA ori 1492...2127

**Fig. 3c**

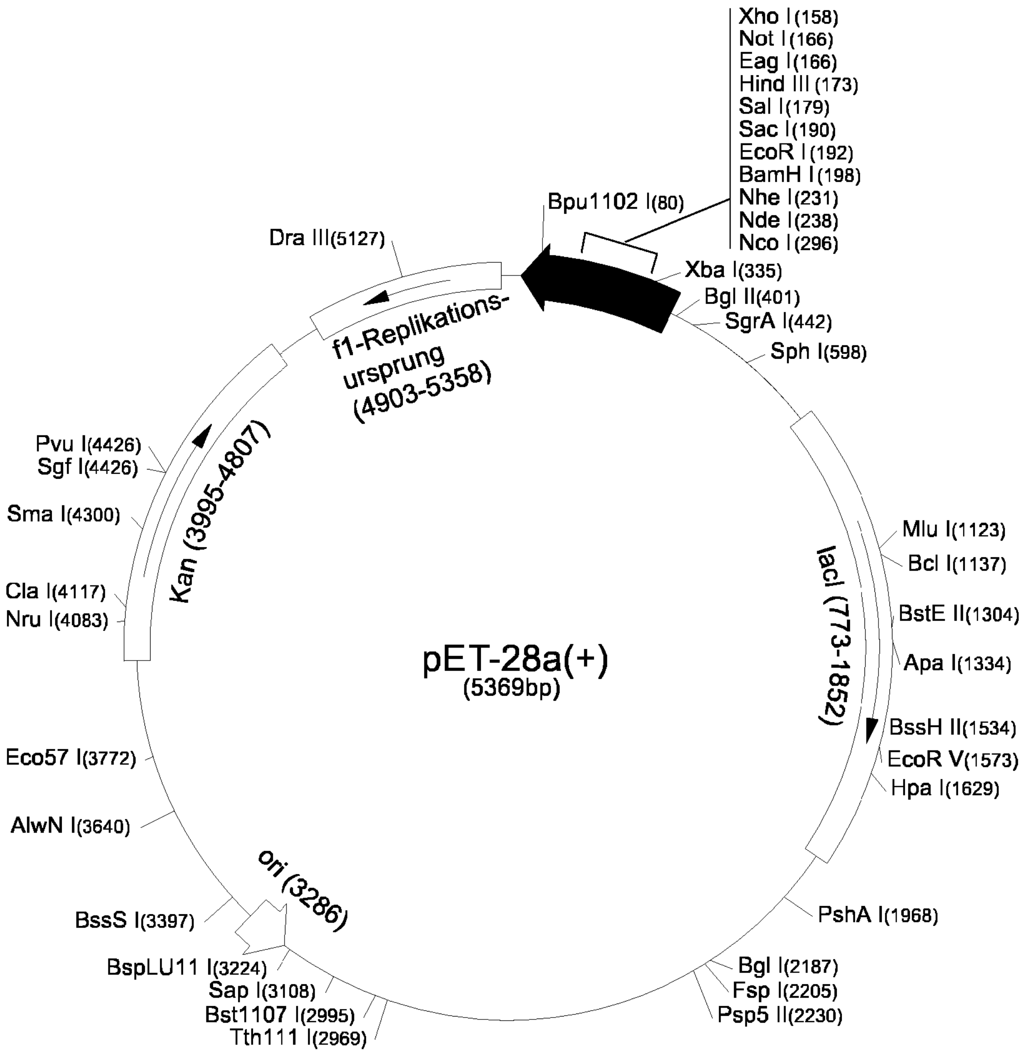
Xho I(158)
Not I(166)
Eag I(166)
Hind III (173)
Sal I(179)
Sac I(190)
EcoR I(192)
BamH I(198)
Nhe I(231)
Nde I(238)
Nco I(296)
Bpu1102 I(80)
Xba I(335)
Bgl II(401)
SgrA I(442)
Sph I(598)
Dra III(5127)
f1-Replikations-ursprung (4903-5358)
Pvu I(4426)
Sgf I(4426)
Kan (3995-4807)
Sma I(4300)
Cla I(4117)
Nru I(4083)
Mlu I(1123)
Bcl I(1137)
lacI (773-1852)
BstE II(1304)
Apa I(1334)
pET-28a(+)
(5369bp)
BssH II(1534)
EcoR V(1573)
Hpa I(1629)
Eco57 I(3772)
AlwN I(3640)
ori (3286)
PshA I(1968)
BssS I(3397)
BspLU11 I(3224)
Sap I(3108)
Bst1107 I(2995)
Tth111 I(2969)
Bgl I(2187)
Fsp I(2205)
Psp5 II(2230)

**Fig. 3d**

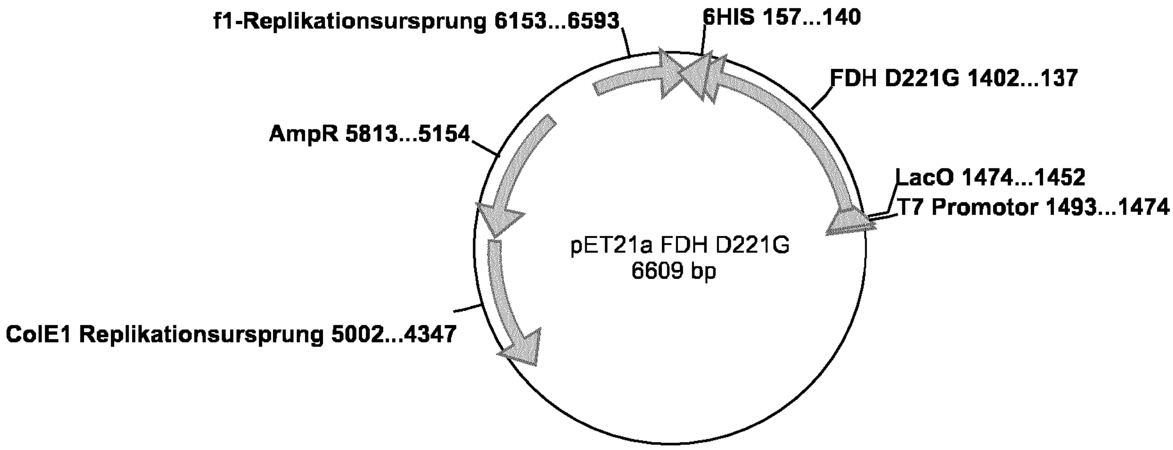
f1-Replikationsursprung 6153...6593
6HIS 157...140
FDH D221G 1402...137
AmpR 5813...5154
LacO 1474...1452
T7 Promotor 1493...1474
pET21a FDH D221G
6609 bp
ColE1 Replikationsursprung 5002...4347

**Fig. 4a**

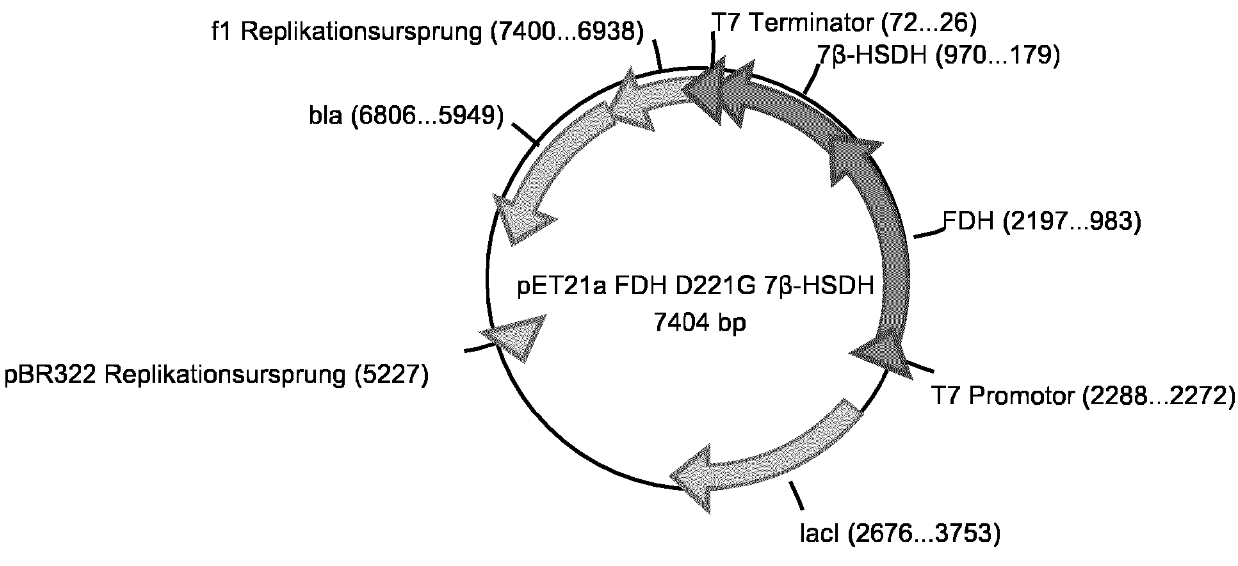
f1 Replikationsursprung (7400...6938)
T7 Terminator (72...26)
7β-HSDH (970...179)
bla (6806...5949)
FDH (2197...983)
pET21a FDH D221G 7β-HSDH
7404 bp
pBR322 Replikationsursprung (5227)
T7 Promotor (2288...2272)
lacI (2676...3753)

**Fig. 4b**

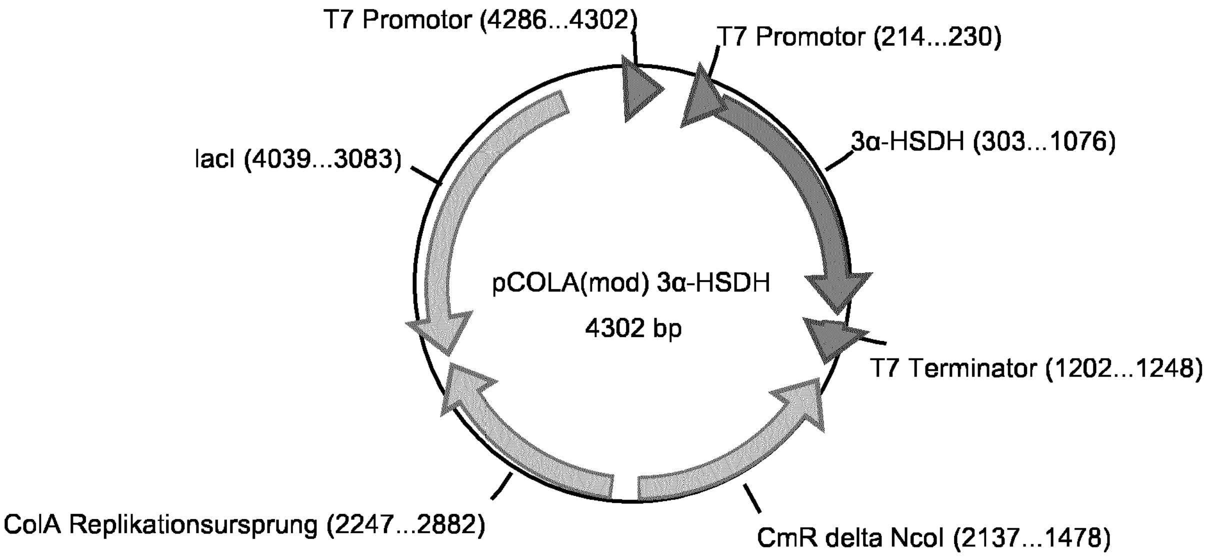
T7 Promotor (4286...4302)
T7 Promotor (214...230)
3α-HSDH (303...1076)
lacI (4039...3083)
pCOLA(mod) 3α-HSDH
4302 bp
T7 Terminator (1202...1248)
ColA Replikationsursprung (2247...2882)
CmR delta NcoI (2137...1478)

**Fig. 5**

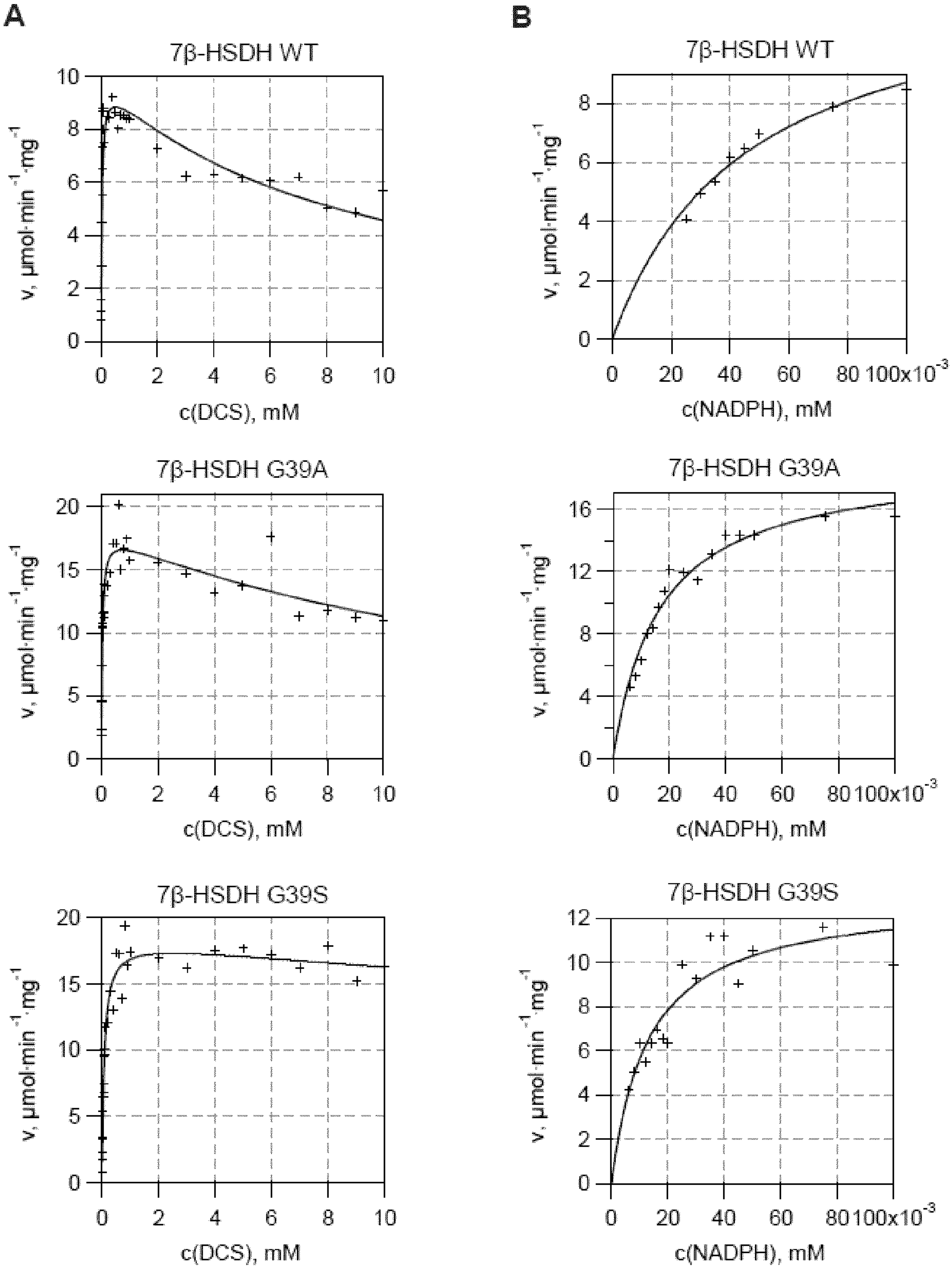
A
B
7β-HSDH WT
7β-HSDH G39A
7β-HSDH G39S
v, µmol·min -1·mg -1
c(DCS), mM
c(NADPH), mM
100x10-3

Fig. 6

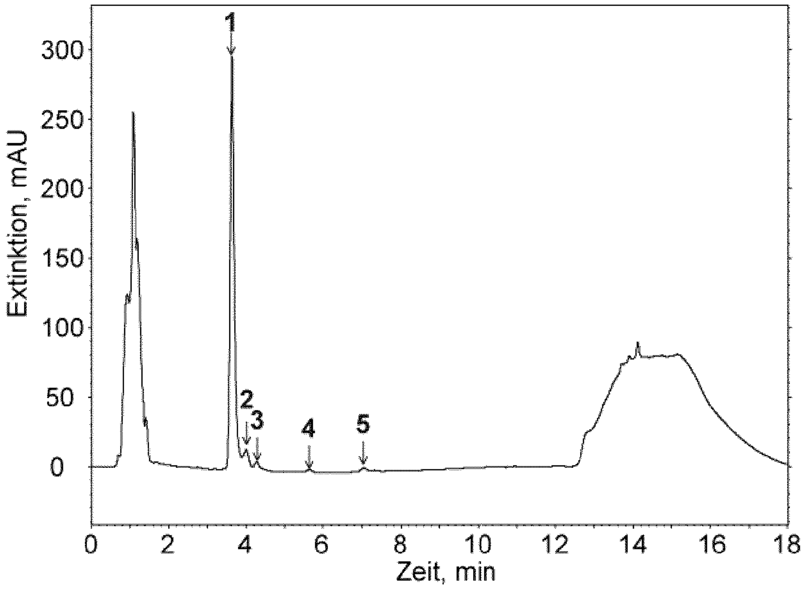
1
2
3
4
5
Extinktion, mAU
300
250
200
150
100
50
0
0
2
4
6
8
10
12
14
16
18
Zeit, min

Fig. 7

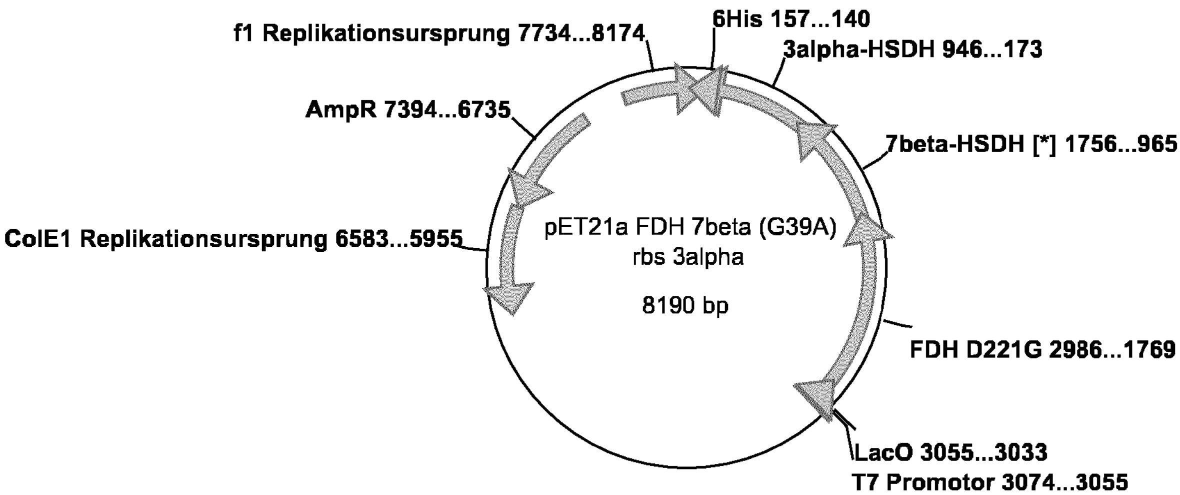
f1 Replikationsursprung 7734...8174
6His 157...140
3alpha-HSDH 946...173
AmpR 7394...6735
7beta-HSDH [*] 1756...965
ColE1 Replikationsursprung 6583...5955
pET21a FDH 7beta (G39A)
rbs 3alpha
8190 bp
FDH D221G 2986...1769
LacO 3055...3033
T7 Promotor 3074...3055

Fig. 8

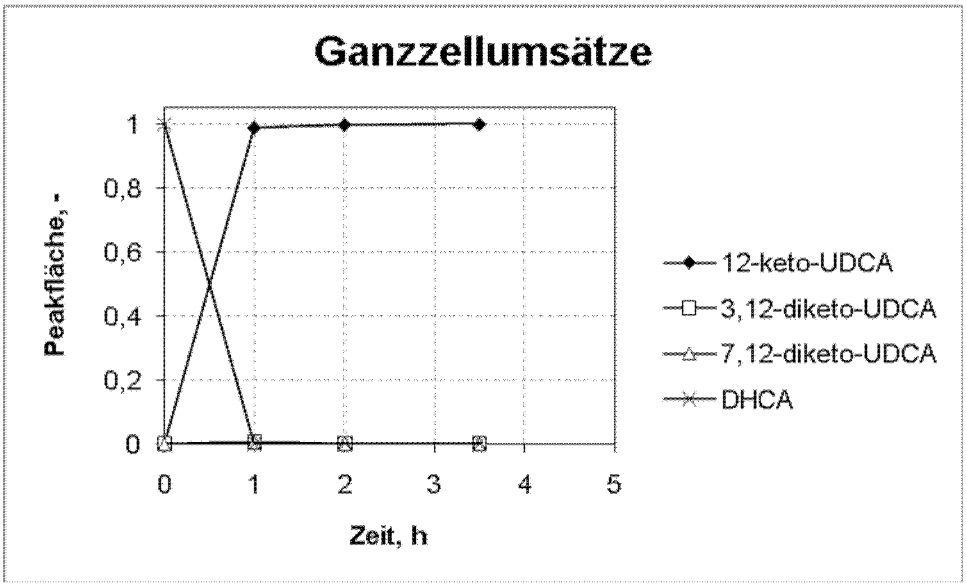


**Fig. 9**

```
7beta-HSDH Wildtyp         MNLREKYGEW GLILGATEGV GKAFCEKIAA GGMNVVMVGR REEKLNVLAG
7beta-HSDH G39D            MNLREKYGEW GLILGATEGV GKAFCEKIAA GGMNVVMVDR REEKLNVLAG
7beta-HSDH G39D R40L       MNLREKYGEW GLILGATEGV GKAFCEKIAA GGMNVVMVDL REEKLNVLAG
7beta-HSDH G39D R40I       MNLREKYGEW GLILGATEGV GKAFCEKIAA GGMNVVMVDI REEKLNVLAG
7beta-HSDH G39D R40V       MNLREKYGEW GLILGATEGV GKAFCEKIAA GGMNVVMVDV REEKLNVLAG

7beta-HSDH Wildtyp         EIRETYGVET KVVRADFSQP GAAETVFAAT EGLDMGFMSY VACLHSFGKI
7beta-HSDH G39D            EIRETYGVET KVVRADFSQP GAAETVFAAT EGLDMGFMSY VACLHSFGKI
7beta-HSDH G39D R40L       EIRETYGVET KVVRADFSQP GAAETVFAAT EGLDMGFMSY VACLHSFGKI
7beta-HSDH G39D R40I       EIRETYGVET KVVRADFSQP GAAETVFAAT EGLDMGFMSY VACLHSFGKI
7beta-HSDH G39D R40V       EIRETYGVET KVVRADFSQP GAAETVFAAT EGLDMGFMSY VACLHSFGKI

7beta-HSDH Wildtyp         QDTPWEKHEA MINVNVVTFL KCFHHYMRIF AAQDRGAVIN VSSMTGISSS
7beta-HSDH G39D            QDTPWEKHEA MINVNVVTFL KCFHHYMRIF AAQDRGAVIN VSSMTGISSS
7beta-HSDH G39D R40L       QDTPWEKHEA MINVNVVTFL KCFHHYMRIF AAQDRGAVIN VSSMTGISSS
7beta-HSDH G39D R40I       QDTPWEKHEA MINVNVVTFL KCFHHYMRIF AAQDRGAVIN VSSMTGISSS
7beta-HSDH G39D R40V       QDTPWEKHEA MINVNVVTFL KCFHHYMRIF AAQDRGAVIN VSSMTGISSS

7beta-HSDH Wildtyp         PWNGQYGAGK AFILKMTEAV ACECEGTGVD VEVITLGTTL TPSLLSNLPG
7beta-HSDH G39D            PWNGQYGAGK AFILKMTEAV ACECEGTGVD VEVITLGTTL TPSLLSNLPG
7beta-HSDH G39D R40L       PWNGQYGAGK AFILKMTEAV ACECEGTGVD VEVITLGTTL TPSLLSNLPG
7beta-HSDH G39D R40I       PWNGQYGAGK AFILKMTEAV ACECEGTGVD VEVITLGTTL TPSLLSNLPG
7beta-HSDH G39D R40V       PWNGQYGAGK AFILKMTEAV ACECEGTGVD VEVITLGTTL TPSLLSNLPG

7beta-HSDH Wildtyp         GPQGEAVMKI ALTPEECVDE AFEKLGKELS VIAGQRNKDS VHDWKANHTE
7beta-HSDH G39D            GPQGEAVMKI ALTPEECVDE AFEKLGKELS VIAGQRNKDS VHDWKANHTE
7beta-HSDH G39D R40L       GPQGEAVMKI ALTPEECVDE AFEKLGKELS VIAGQRNKDS VHDWKANHTE
7beta-HSDH G39D R40I       GPQGEAVMKI ALTPEECVDE AFEKLGKELS VIAGQRNKDS VHDWKANHTE
7beta-HSDH G39D R40V       GPQGEAVMKI ALTPEECVDE AFEKLGKELS VIAGQRNKDS VHDWKANHTE

7beta-HSDH Wildtyp         DEYIRYMGSF YRD
7beta-HSDH G39D            DEYIRYMGSF YRD
7beta-HSDH G39D R40L       DEYIRYMGSF YRD
7beta-HSDH G39D R40I       DEYIRYMGSF YRD
7beta-HSDH G39D R40V       DEYIRYMGSF YRD
```

**Fig. 10**

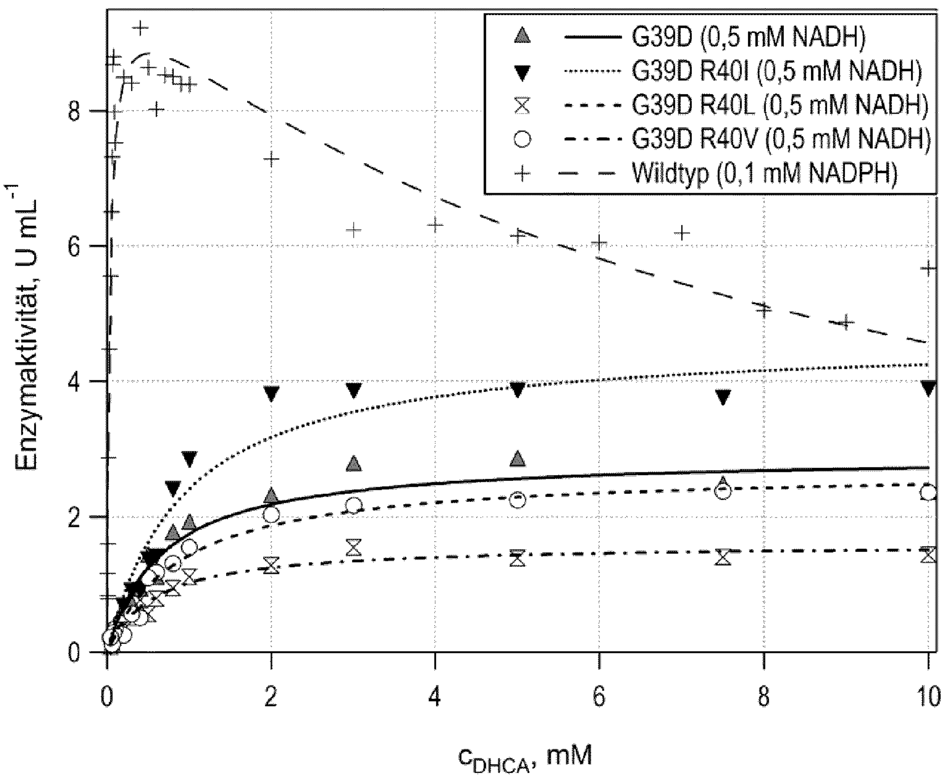


Fig. 11

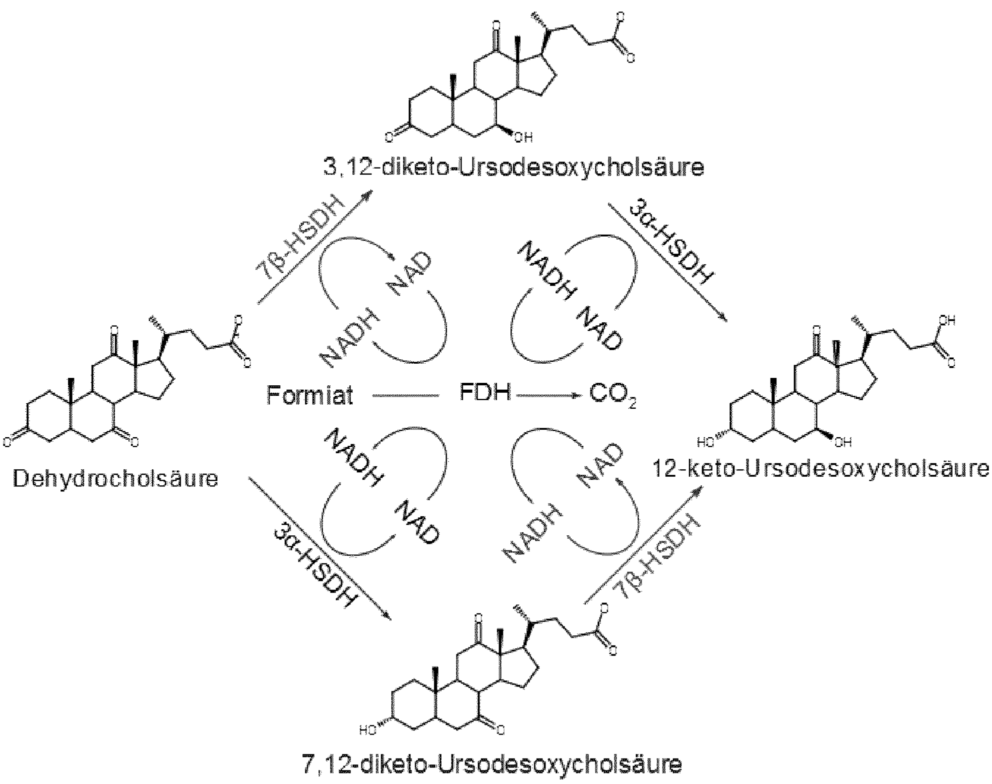


3,12-diketo-Ursodesoxycholsäure

Dehydrocholsäure

12-keto-Ursodesoxycholsäure

7,12-diketo-Ursodesoxycholsäure

Fig.12

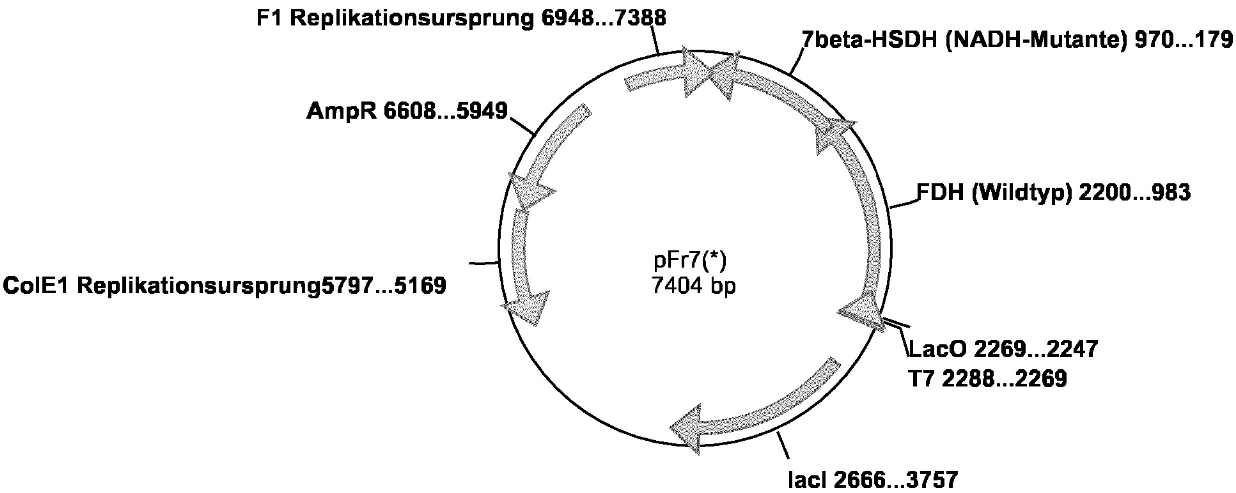
F1 Replikationsursprung 6948...7388
7beta-HSDH (NADH-Mutante) 970...179
AmpR 6608...5949
FDH (Wildtyp) 2200...983
ColE1 Replikationsursprung5797...5169
pFr7(*)
7404 bp
LacO 2269...2247
T7 2288...2269
lacI 2666...3757

Fig. 13

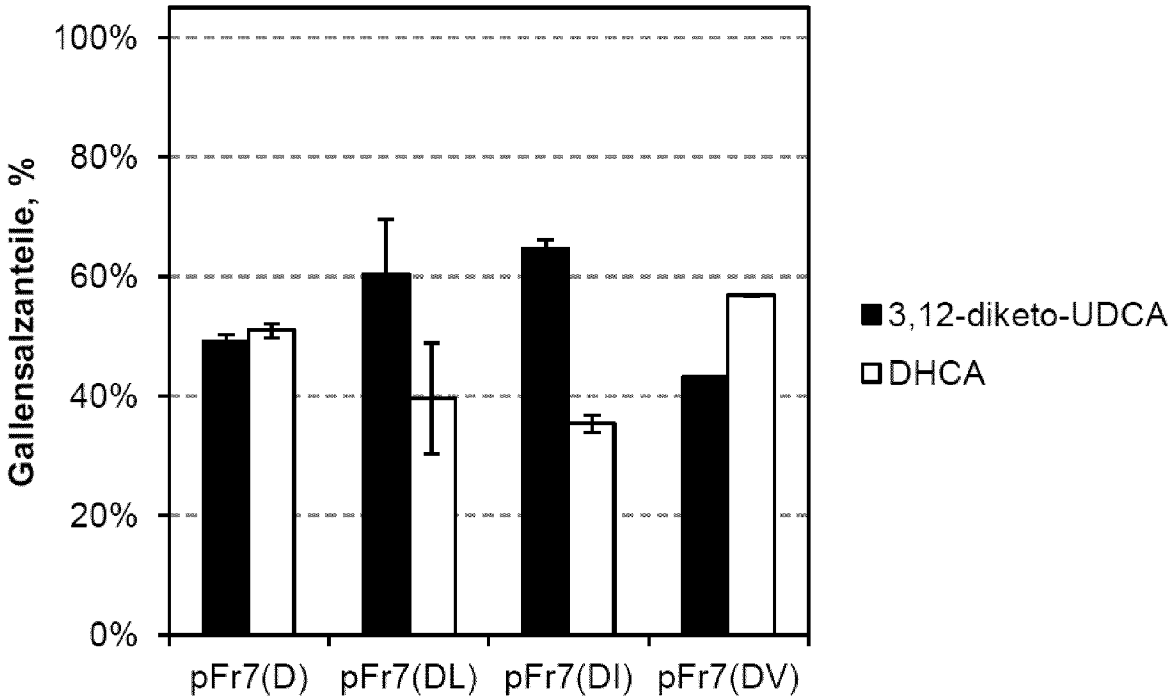
100%
80%
60%
40%
20%
0%
Gallensalzanteile, %
pFr7(D)
pFr7(DL)
pFr7(DI)
pFr7(DV)
3,12-diketo-UDCA
DHCA

Fig. 14

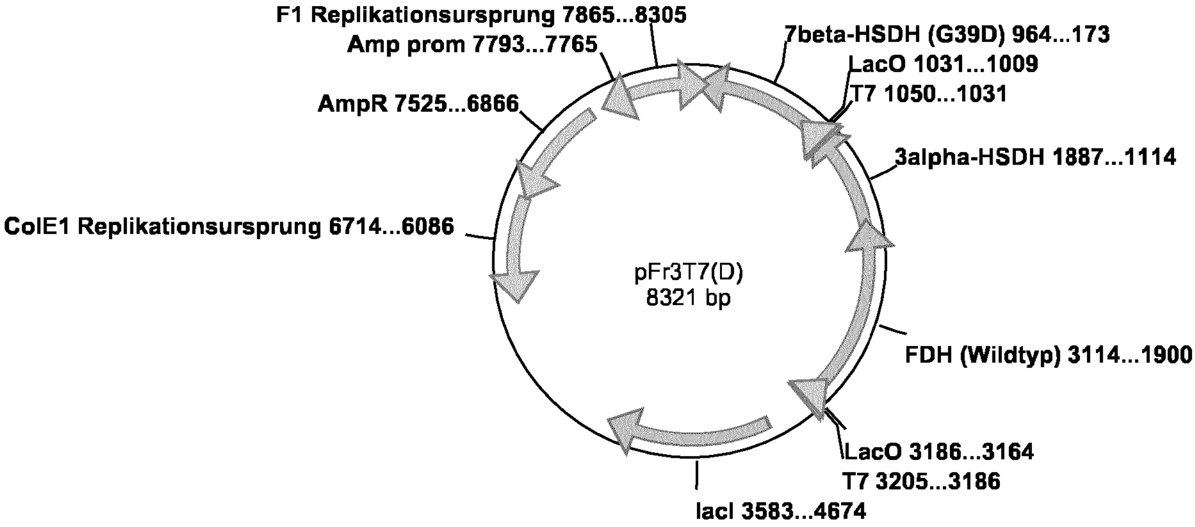
F1 Replikationsursprung 7865...8305
Amp prom 7793...7765
AmpR 7525...6866
ColE1 Replikationsursprung 6714...6086
7beta-HSDH (G39D) 964...173
LacO 1031...1009
T7 1050...1031
3alpha-HSDH 1887...1114
pFr3T7(D)
8321 bp
FDH (Wildtyp) 3114...1900
LacO 3186...3164
T7 3205...3186
lacI 3583...4674

Fig. 15

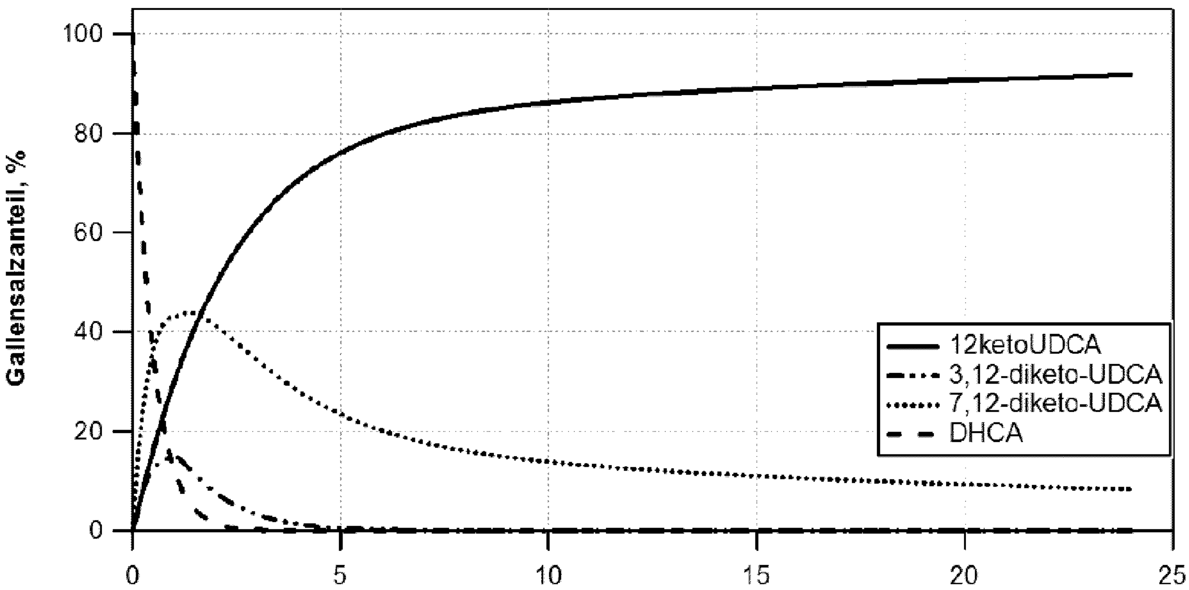
Gallensalzanteil, %
100
80
60
40
20
0
0
5
10
15
20
25
12ketoUDCA
3,12-diketo-UDCA
7,12-diketo-UDCA
DHCA

Fig. 16

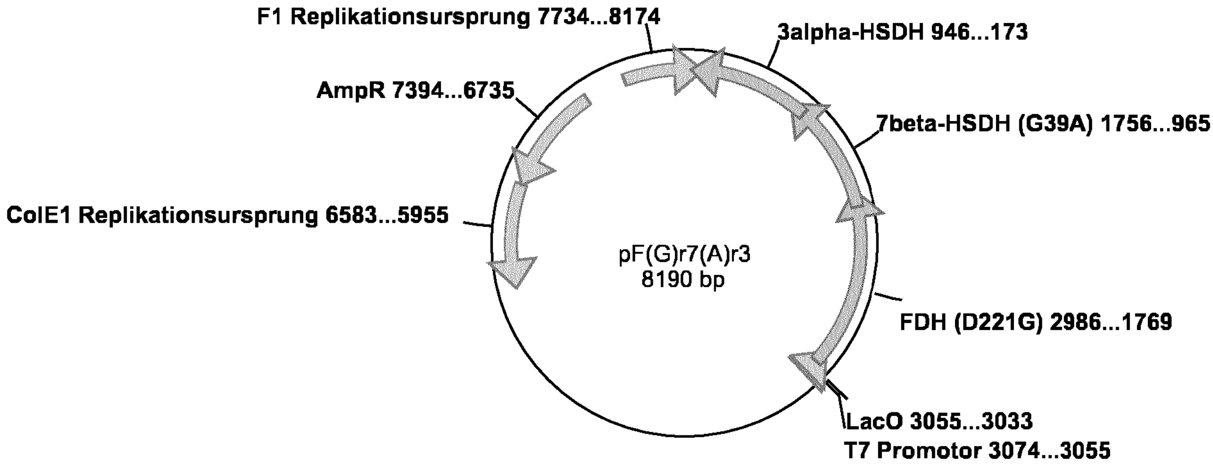


Fig. 17a

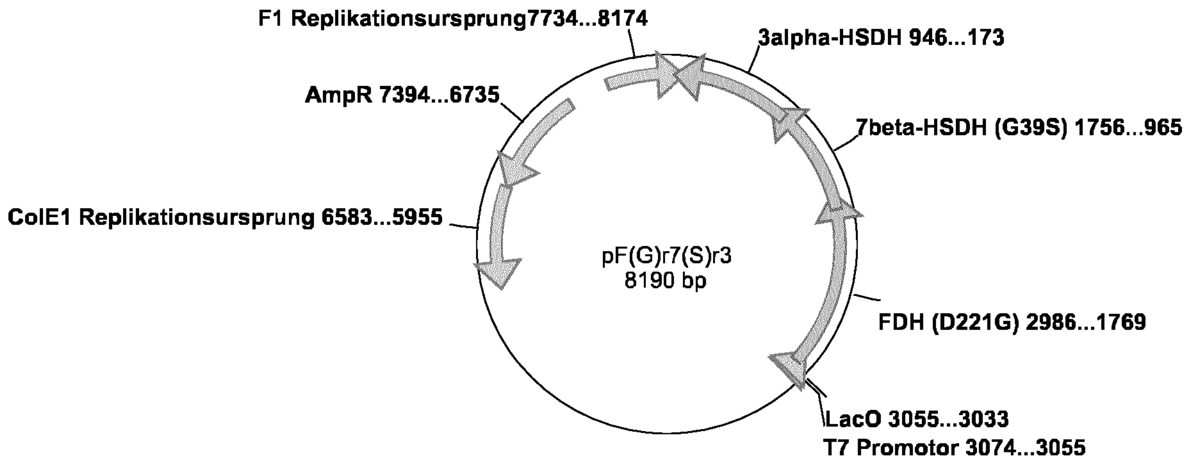


Fig. 17b

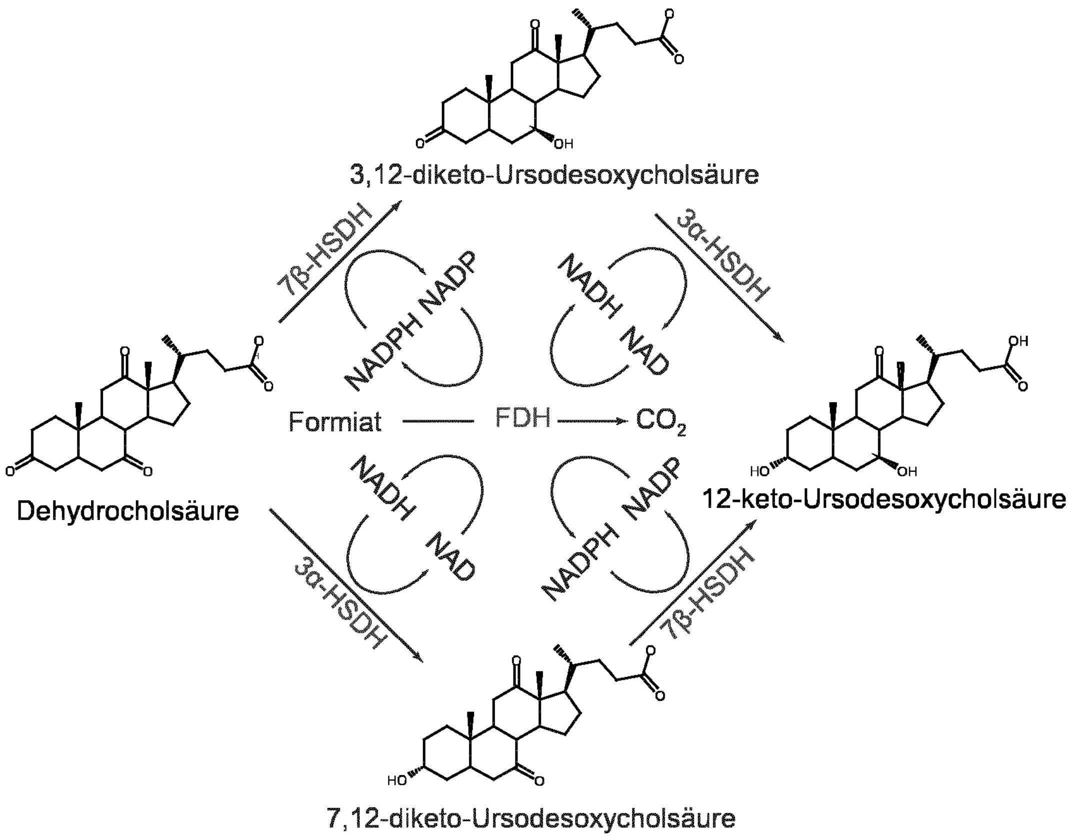


3,12-diketo-Ursodesoxycholsäure

Dehydrocholsäure

12-keto-Ursodesoxycholsäure

7,12-diketo-Ursodesoxycholsäure

Fig. 18

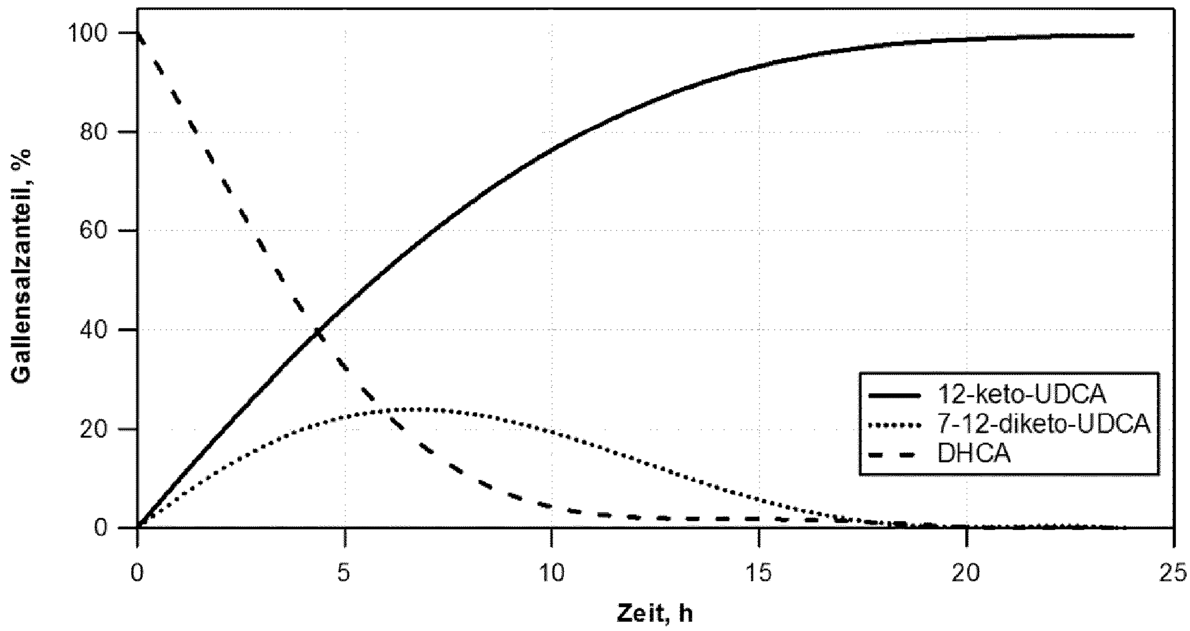


Fig. 19

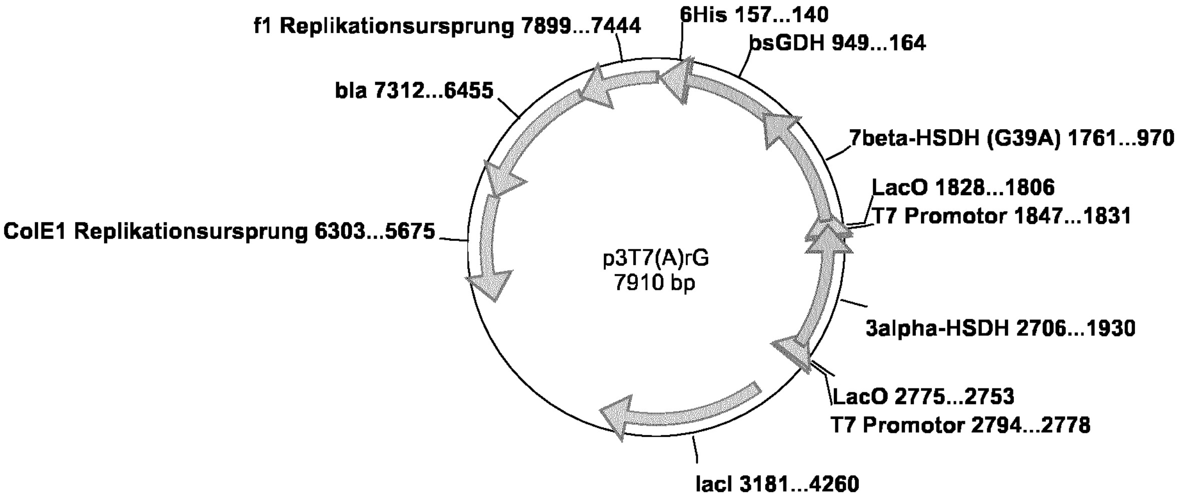
f1 Replikationsursprung 7899...7444
6His 157...140
bsGDH 949...164
bla 7312...6455
7beta-HSDH (G39A) 1761...970
LacO 1828...1806
T7 Promotor 1847...1831
ColE1 Replikationsursprung 6303...5675
p3T7(A)rG
7910 bp
3alpha-HSDH 2706...1930
LacO 2775...2753
T7 Promotor 2794...2778
lacI 3181...4260

Fig. 20

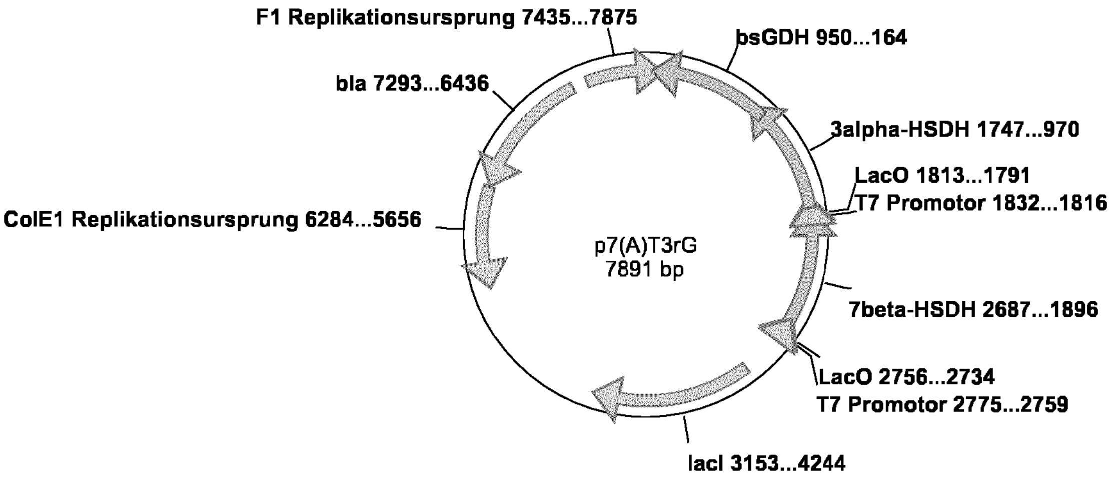
F1 Replikationsursprung 7435...7875
bsGDH 950...164
bla 7293...6436
3alpha-HSDH 1747...970
LacO 1813...1791
T7 Promotor 1832...1816
ColE1 Replikationsursprung 6284...5656
p7(A)T3rG
7891 bp
7beta-HSDH 2687...1896
LacO 2756...2734
T7 Promotor 2775...2759
lacI 3153...4244

Fig. 21

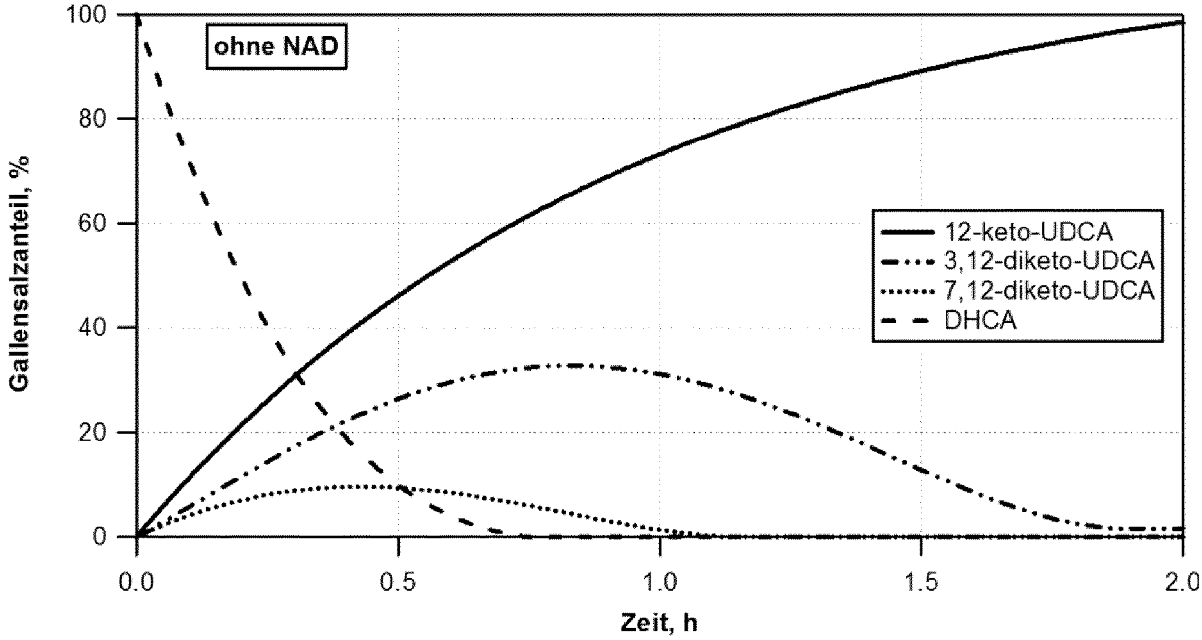
ohne NAD
Gallensalzanteil, %
100
80
60
40
20
0
0.0
0.5
1.0
1.5
2.0
Zeit, h
12-keto-UDCA
3,12-diketo-UDCA
7,12-diketo-UDCA
DHCA

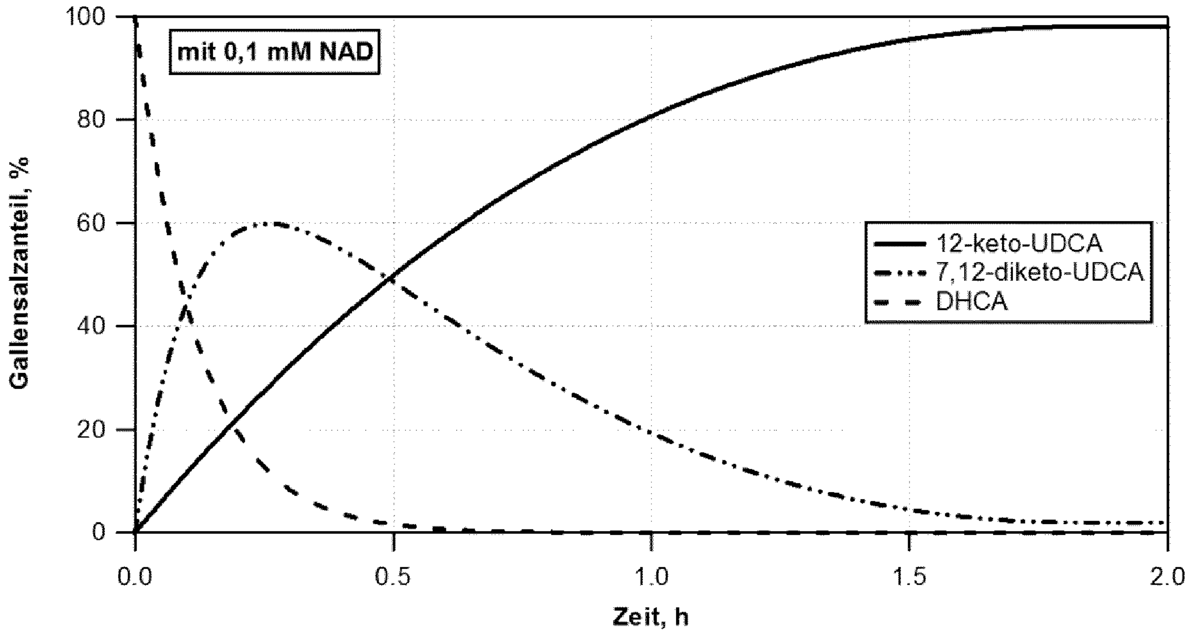
mit 0,1 mM NAD
Gallensalzanteil, %
100
80
60
40
20
0
0.0
0.5
1.0
1.5
2.0
Zeit, h
12-keto-UDCA
7,12-diketo-UDCA
DHCA

Fig. 22

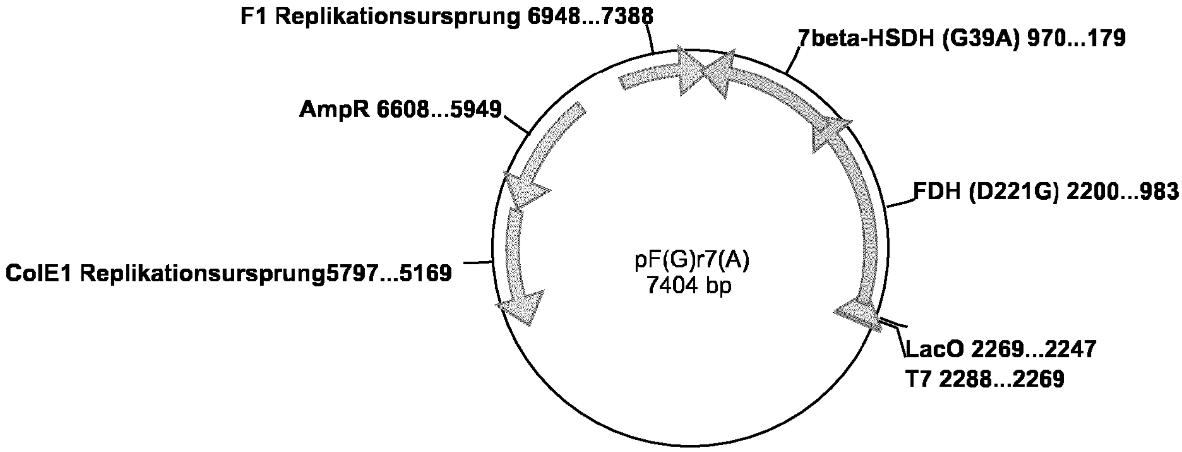


Fig. 23a

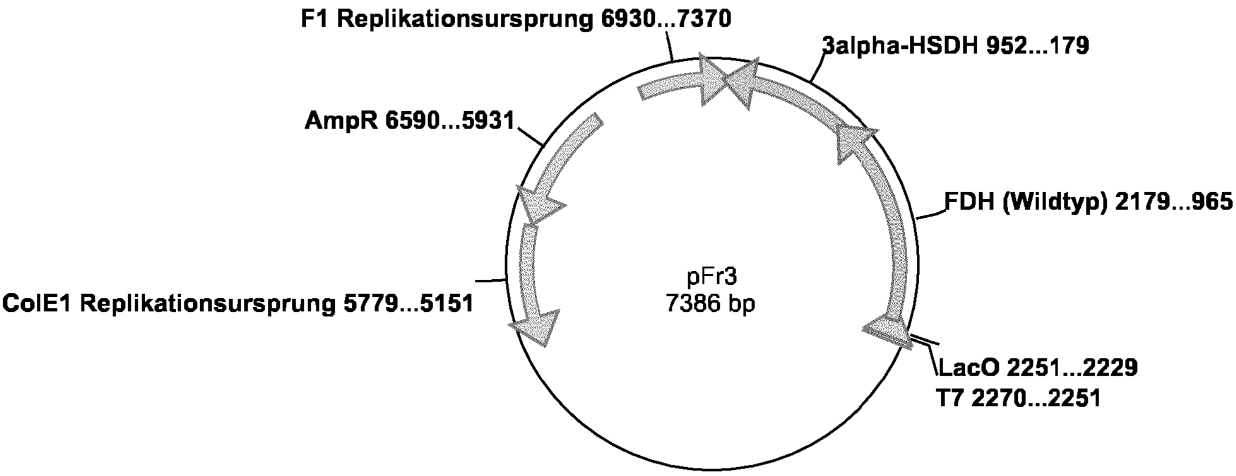


Fig. 23b

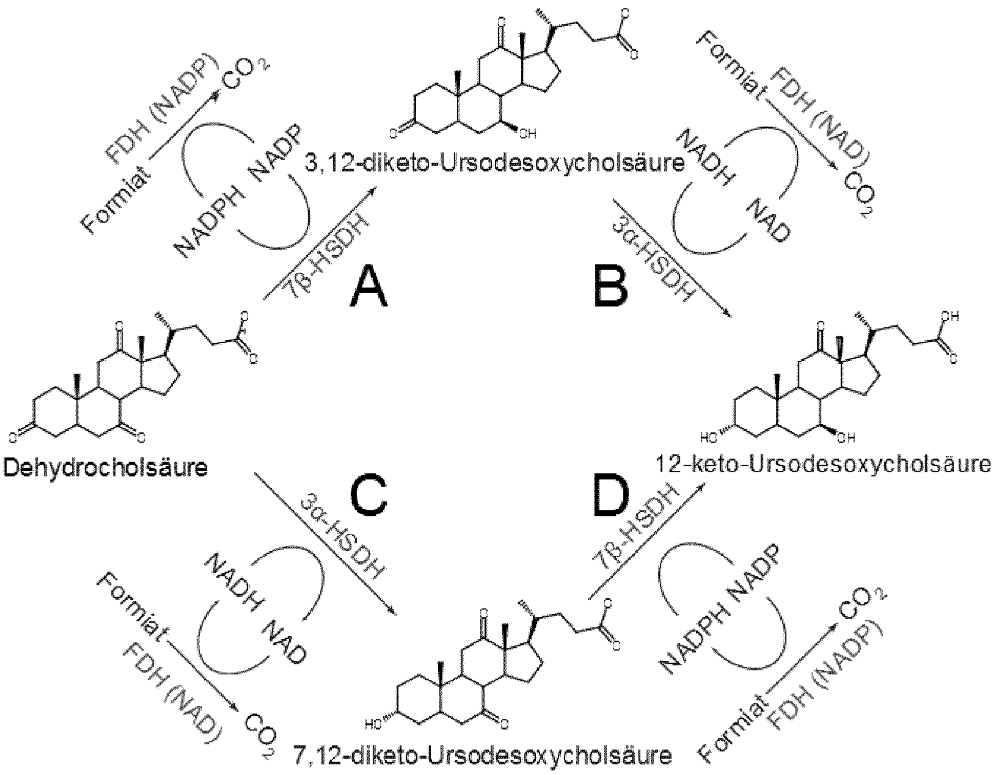
3,12-diketo-Ursodesoxycholsäure
Dehydrocholsäure
12-keto-Ursodesoxycholsäure
7,12-diketo-Ursodesoxycholsäure
A
B
C
D
7β-HSDH
3α-HSDH
3α-HSDH
7β-HSDH
NADPH NADP
NADH NAD
NADH NAD
NADPH NADP
Formiat
FDH (NADP)
$CO_2$
Formiat
FDH (NAD)
$CO_2$
Formiat
FDH (NAD)
$CO_2$
Formiat
FDH (NADP)
$CO_2$

Fig. 24

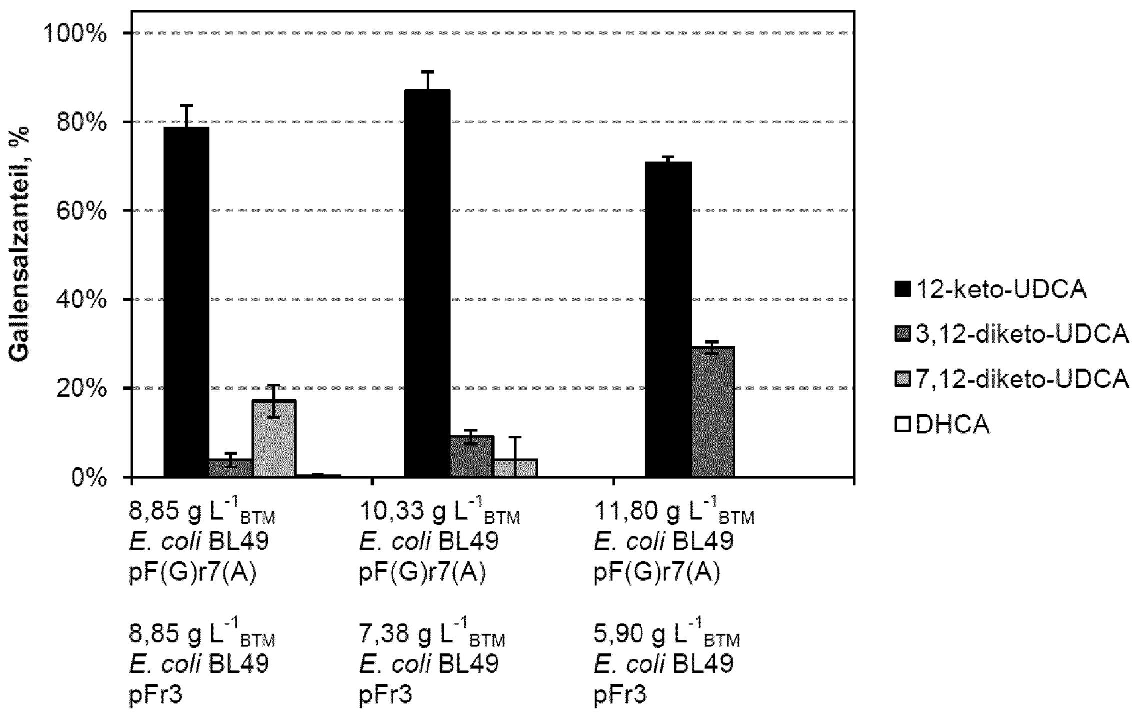
Gallensalzanteil, %
100%
80%
60%
40%
20%
0%
12-keto-UDCA
3,12-diketo-UDCA
7,12-diketo-UDCA
DHCA
8,85 g $L^{-1}_{BTM}$
*E. coli* BL49
pF(G)r7(A)
8,85 g $L^{-1}_{BTM}$
*E. coli* BL49
pFr3
10,33 g $L^{-1}_{BTM}$
*E. coli* BL49
pF(G)r7(A)
7,38 g $L^{-1}_{BTM}$
*E. coli* BL49
pFr3
11,80 g $L^{-1}_{BTM}$
*E. coli* BL49
pF(G)r7(A)
5,90 g $L^{-1}_{BTM}$
*E. coli* BL49
pFr3

Fig. 25

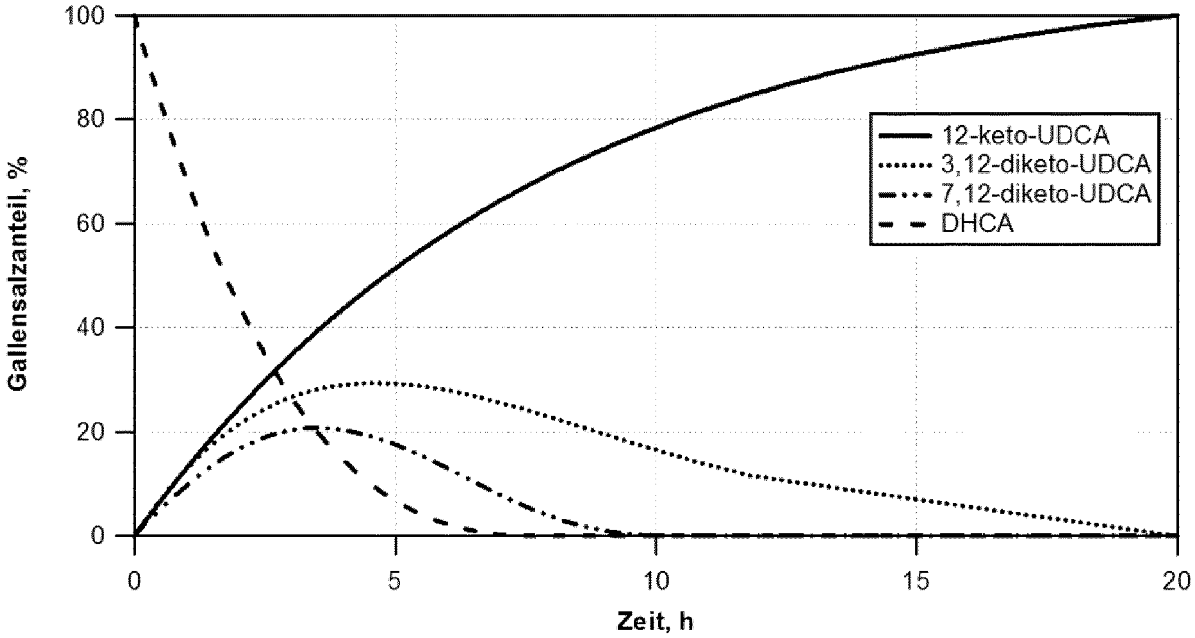
100
80
60
40
20
0
Gallensalzanteil, %
0
5
10
15
20
Zeit, h
12-keto-UDCA
3,12-diketo-UDCA
7,12-diketo-UDCA
DHCA

Fig. 26

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2009002190 W
- EP 2010068576 W
- EP 1149849 A
- EP 1069183 A
- DE 100193773 A
- EP 10164003


### In der Beschreibung aufgeführte Nicht-Patentliteratur


- **VON MONTI, D et al.** One-Pot Multienzymatic Synthesis of 12-Ketoursodeoxycholic Acid: Subtle Cofactor Specificities Rule the Reaction Equilibria of Five Biocatalysts Working in a Row. *Advanced Synthesis & Catalysis,* 2009
- **ZHU, D et al.** Enzymatic enantioselective reduction of -ketoesters by a thermostable 7 -hydroxysteroid dehydrogenase from Bacteroides fragilis. *Tetrahedron,* 2006, vol. 62 (18), 4535-4539
- **MACDONALD, I.A ; P.D. ROACH.** Bile induction of 7 alpha- and 7 beta-hydroxysteroid dehydrogenases in Clostridium absonum. *Biochim Biophys Acta,* 1981, vol. 665 (2), 262-9
- **HIRANO, S ; N. MASUDA.** Characterization of NADP-dependent 7 beta-hydroxysteroid dehydrogenases from Peptostreptococcus productus and Eubacterium aerofaciens. *Appl Environ Microbiol,* 1982, vol. 43 (5), 1057-63
- **TISHKOV et al.** *Biomolecular Engineering,* 2006, vol. 23, 89-110
- **J E PAWLOWSKI ; M HUIZINGA ; T M PENNING.** *The Journal of Biological Chemistry,* 15. Mai 1991, vol. 266, 8820-8825
- **R.K. SCOPES.** Protein Purification. Springer Verlag, 1982
- **PEARSON ; LIPMAN.** *Proc. Natl. Acad, Sci. (USA),* 1988, vol. 85 (8), 2444-2448
- **NARANG, S.A.** *Tetrahedron,* 1983, vol. 39, 3
- **ITAKURA et al.** *Annu. Rev. Biochem,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500
- Voet, Voet. Wiley Press, vol. 2, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory, Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **SAMBROOK, J. ; FRITSCH, E.F ; MANIATIS, T.** Molecular Cloning (A Laboratory Manual). Cold Spring Harbor Laboratory Press, 1989, vol. 2, 9.31-9.57
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCÄREL R ; STUNNENBERG HG.** *Nucleic Acids Res,* 1994, vol. 22, 541
- **BARIK S.** *Mol Biotechnol,* 1995, vol. 3 (1
- **ECKERT KA ; KUNKEL TA.** *Nucleic Acids Res,* 1990, vol. 18, 3739
- An efficient random mutagenesis technique u-sing an E.coli mutator strain. **GREENER A ; CALLAHAN M ; JERPSETH B.** In vitro mutagenesis protocols. Humana Press, 1996
- **STEMMER WPC.** *Nature,* 1994, vol. 370, 389

- **STEMMER WPC.** *Proc Natl Acad Sci USA,* 1994, vol. 91, 10747
- **REETZ MT ; JAEGER K-E.** *Topics Curr Chem,* 1999, vol. 200, 31
- Methods for optimizing industrial enzymes by directed evolution. **ZHAO H ; MOORE JC ; VOLKOV AA ; ARNOLD FH.** Manual of industrial microbiology and biotechnology. American Society for Microbiology. 1999
- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- Cloning Vectors. Elsevier, 1985
- Molecular Biology. Wiley Interscience, 1997
- **SAMBROOK et al.** Molecular Cloning: A Laboratory Manual. Cold Spring Harbor Laboratory, Cold Spring Harbor Laboratory Press, 1989
- **TERPE, K.** *Appl. Microbiol. Biotechnol,* 2006, vol. 72, 211-222
- Bioverfahrenstechnik. Gustav Fischer Verlag, 1991
- Manual of Methods für General Bacteriology. *American Society für Bacteriology,* 1981
- Applied Microbiol. Physiology, A Practical Approach. IRL Press, 1997, 53-73
- **HARLOW, E. ; LANE, D.** Antibodies: A Laboratory Manual. Cold Spring Harbor (N.Y.) Press, 1988
- **J. LALONDE ; A. MARGOLIN.** *Immobilization of Enzymes*
- **K. DRAUZ ; H. WALDMANN.** Enzyme Catalysis in Organic Synthesis. Wiley-VCH, 2002, vol. III, 991-1032
- **WILMS et al.** *BIOTECHNOLOGY AND BIOENGI-NEERING,* 2001, vol. 73 (2), 95-103
- **OPPERMANN et al.** *J Biochem,* 1996, vol. 241 (3), 744-749
- **SAMBROOK, J. ; FRITSCH, E.F ; MANIATIS, T.** Molecular Cloning: A Laboratory Manual. Cold Spring Harbor Laboratory, Cold Spring Harbor Laboratory Press, 1989
- Current Protocols in Molecular Biology. John Wiley &Sons, 1993
- **KRIEGLER.** Gene Transfer and Expression, A Laboratory Manual. Stockton Press, 1990
- **THOMPSON et al.** *Nucleic Acid Research,* 1997, vol. 25, 4876-82
- **CLAMP et al.** *Bioinformatics,* 2004, vol. 20, 426-7
- **JORNVALL, H. ; B. PERSSON ; M. KROOK ; S. ATRIAN ; R. GONZALEZ-DUARTE ; J. JEFFERY ; D. GHOSH.** Short-chain dehydrogenases/reductases (SDR). *Biochemistry,* 1995, vol. 34, 6003-13
- **PERSSON, B. ; M. KROOK ; H. JORNVALL.** Characteristics of short-chain alcohol dehydrogenases and related enzymes. *Eur J Biochem,* 1991, vol. 200, 537-43
- **MOBUS, E. ; E. MASER.** Molecular cloning, overexpression, and characterization of steroid-inducible 3alpha-hydroxysteroid dehydrogenaselcarbonyl reductase from Comamonas testosteroni. A novel member of the short-chain dehydrogenaselreductase superfamily. *J Biol Chem,* 1998, vol. 273 (47), 30888-96
- **SANCHIS J ; FERNÄNDEZ L ; CARBALLEIRA JD ; DRONE J ; GUMULYA Y ; HÖBENREICH H ; KAHAKEAW D ; KILLE S ; LOHMER R ; PEYRALANS JJ.** Improved PCR method for the creation of saturation mutagenesis libraries in directed evolution: application to difficult-to-amplify templates. *Appl Microbiol Biotechnol,* November 2008, vol. 81 (2), 387-97